(19) Europäisches Patentamt
European Patent Office
Office européen des brevets

(11) Publication number: **0 206 747 B1**

(12) **EUROPEAN PATENT SPECIFICATION**

(45) Date of publication of patent specification: **31.10.90**

(21) Application number: **86304679.3**

(22) Date of filing: **17.06.86**

(51) Int. Cl.[5]: **C 07 D 211/90,** C 07 D 401/04, C 07 D 401/12, C 07 D 405/04, C 07 D 215/54, A 61 K 31/44, A 61 K 31/47

# (54) 1,4-Dihydropyridine derivatives useful in the treatment of cardiovascular disorders, and a process for producing the derivatives.

(30) Priority: **17.06.85 US 745965**
**21.04.86 US 852731**

(43) Date of publication of application:
**30.12.86 Bulletin 86/52**

(45) Publication of the grant of the patent:
**31.10.90 Bulletin 90/44**

(84) Designated Contracting States:
**AT BE CH DE FR GB IT LI LU NL SE**

(56) References cited:
**EP-A-0 071 819**
**EP-A-0 125 803**
**EP-A-0 172 029**
**EP-A-0 200 524**
**DE-A-2 658 183**

(73) Proprietor: **WARNER-LAMBERT COMPANY**
**201 Tabor Road**
**Morris Plains New Jersey 07950 (US)**

(72) Inventor: **Sircar, Ila**
**3615 Charter Place**
**Ann Arbor Michigan 48105 (US)**

(74) Representative: **Jones, Michael Raymond et al**
**HASELTINE LAKE & CO. Hazlitt House 28**
**Southampton Buildings Chancery Lane**
**London WC2A 1AT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 206 747 B1

Courier Press, Leamington Spa, England.

**Description**

Dihydropyridine polyesters have been described in the literature as antihypertensive and antianginal agents. For example, British Patent Number 1,389,509 describes 1,4-dihydropyridine polyesters as coronary agents. Further, disclosure for cardiovascular activity used as antihypertensives, vasodilators, antifibrillatory agents, and also having marked smooth muscle effect is made for 1,2,3,4-tetrahydropyriding-3,5-dicarboxylic acid derivatives in West German Application Number 3,239,272A. Other dihydropyridines are shown to be calcium agonists or cardiotonics in Belgian Patent 893,984. Additional disclosure for various dihydropyridine polyesters having usefulness as cardiovascular agents is found in West German Application 2,248,150 and Belgian Patents 804,160, 803,474, 861,964, 843,576, and 817,540.

EP—A—0200524 discloses dihydropyridine calcium antagonists, but is relevant only under Article 54(3) EPC.

More specifically, 1,4-dihydropyridine derivatives substituted in the two position are said to be distinguished by a powerful vessel-influencing action in US Patent Number 4,188,395 by Bossert, et al. No mention is made of inotropic effect.

British Patent Number 2,112,782 discloses various dihydropyridines not teaching the compounds of the present invention but having increased myocardial contractility.

None of the above references teaches the combination of substituents on a dihydropyridine moiety of the present invention. More particularly, the 1,4-dihydropyridine derivatives of the present invention having Formula I or II are distinguished from Bossert, et al, by the differences in the sulfur containing substituent on the dihydropyridine moiety and, therefore, are not taught by Bossert, et al.

Thus, the present novel dihydropyridine derivatives of Formula I or Formula II each as defined hereinafter having valuable calcium antagonist and cardiotonic properties useful for the treatment of cardiovascular disorders are not within the teachings of the prior art.

Accordingly, the present invention provides a compound of Formula I and a pharmaceutically acceptable salt thereof; wherein R is (1) hydrogen; (2) lower alkyl of from one to four carbons, inclusive; which is optionally substituted with an amino group such as $NR_7R_8$ where $R_7$ and $R_8$ are independently hydrogen or lower alkyl, or taken together form a five- or six-membered ring; and (3) aralkyl wherein (a) ar is phenyl optionally substituted with one or more of halo, trifluoromethyl, nitro, amino, mono- of di-alkylamino wherein the alkyls when taken together are of from one to six carbons, inclusive, cyano, lower alkoxy of from one to four carbons, inclusive, lower thioalkyl of from one to four carbons, inclusive, and (b) alkyl is alkylene of from one to four carbons, inclusive.

$R_2$ is cyano;

$R_3$ is hydrogen, lower alkyl of from one to four carbons, inclusive, cyano, nitro, $CO_2R_4$ wherein $R_4$ is hydrogen or lower alkyl of from one to four carbons, inclusive, optionally substituted with amino, mono- or di- alkylamino wherein the alkyls when taken together are of from one to six carbons, inclusive.

$R_1$ is

(1) cycloalkyl of from four to seven carbon atoms;

(2) phenyl optionally substituted with one or more of (i) alkyl having from one to four carbon atoms, (ii) halo, (iii) trifluoromethyl, (iv) nitro, (v) amino, and (vi) mono- or di- alkylamino in which the or each alkyl is independently a lower alkyl of from one to six carbon atoms or, in the case of a dialkylamino, in which the two alkyls, when taken together, represent an alkylene of from one to six carbon atoms; or

(3) thienyl, furanyl, pyryl, pyridinyl, quinolyl, isoquinolyl, pyrimidyl, pyridazinyl, quinazolyl, quinoxalyl, benzothienyl, pyrazolyl, imidazolyl, oxazolyl, isoxazolyl, thiazolyl, triazolyl, pyrazinyl, oxazinyl, thiazinyl, indolizinyl, indolyl, benzofuranyl, indazolyl, benzothienyl, benzimidazolyl, benzoxazolyl, benzioxazolyl, benzthiazolyl, benztriazolyl, benzoxadiazolyl, cinnolinyl, phthalaxinyl, naphthyridinyl, or benzothiazinyl;

$R_5$ and $R_6$ are the same or different and are (1) lower alkyl of from one to four carbons, inclusive, which is optionally substituted with

a) an amino group, such as $NR_7R_8$ where $R_7$ and $R_8$ are independently hydrogen and lower alkyl which may also be taken together to form a five- or six-membered ring; or (b) $OR_9$ where $R_9$ is H or lower alkyl of from one to four carbons, inclusive, or

c) $CO_2R_4$, where $R_4$ has same definition as stated above; 2) cycloalkyl of from four to seven carbons; 3) phenyl optionally substituted with one or more of alkyl of from one to four carbons, inclusive, halo, trifluoromethyl, nitro, amino, mono- or di- alkylamino wherein the alkyls when taken together are of from one to six carbons, inclusive, cyano, lower alkoxy of from one to four carbons, inclusive, lower thioalkyl of from one to four carbons, inclusive; (4) aralkyl wherein ar is phenyl optionally substituted with one or more of alkyl of from one to four carbons, inclusive, halo, trifluoromethyl, nitro, amino, mono- or di- alkylamino wherein the alkyls when taken together have of from one to six carbons, inclusive, cyano, lower alkoxy of from one to four carbons, inclusive, lower thioalkyl of from one to four carbons, inclusive; or (5) heteroaryls as defined above under $R_1$.

Additionally, $R_2$ and $R_5$ may also be taken together to form a five- or six-membered ring having as a member of the ring a C(O)— or C(O)O— group.

n is one or two and

m is a number of from one to six, inclusive.

Additionally, the present invention is a compound of the Formula II and a pharmaceutically acceptable salt thereof where possible, e.g., when a basic nitrogen is present, wherein

R is

(1) hydrogen;

(2) lower alkyl of from one to four carbon atoms, optionally substituted with an amino group having the formula $NR_7R_8$ in which $R_7$ and $R_8$ are independently hydrogen or lower alkyl of from one to four carbons, or $R_7$ and $R_8$, when taken together with the nitrogen atom to which they are attached form a five- or six-membered ring having four or five $CH_2$ groups; or

(3) an arylalkylene radical in which the aryl part is phenyl optionally substituted with one or more of (i) halo, (ii) trifluoromethyl, (iii) nitro, (iv) amino, (v) mono- or di-alkylamino in which the or each alkyl is independently a lower alkyl of from one to six carbon atoms or, in the case of a dialkylamino, in which the two alkyls, when taken together represent an alkylene of from one to six carbon atoms, (vi) cyano, (vii) lower alkoxy of from one to four carbon atoms, (viii) lower alkyl of from one to four carbon atoms, (ix) lower thioalkyl of from one to four carbon atoms; and the alkylene part has from one to four carbon atoms;

$R_2$ and $R_3$, which may be the same or different, are each hydrogen, lower alkyl of from one to four carbon atoms, cyano, nitro, $—CO_2R_4$ in which $R_4$ is hydrogen or lower alkyl of from one to four carbon atoms, optionally substituted with amino, mono-, or dialkylamino in which the or each alkyl is independently a lower alkyl of from one to six carbon atoms or, in the case of a dialkylamino, in which the two alkyls, when taken together, represent an alkylene of from one to six carbon atoms;

$R_1$, $R_5$ and $R_6$, which may be the same or different, are each

(1) lower alkyl of from one to four carbon atoms, optionally substituted with (a) an amino group having the formula $NR_7R_8$ in which $R_7$ and $R_8$, when taken together with the nitrogen atom to which they are attached form a five- or six-membered ring having four or five carbon atoms, (b) $—OR_9$ is hydrogen or lower alkyl of from one to four carbon atoms, or (c) $—CO_2R_4$ in which $R_4$ is as defined above;

(2) cycloalkyl of from four to seven carbon atoms;

(3) phenyl optionally substituted with one or more of (i) alkyl having from one to four carbon atoms, (ii) halo, (iii) trifluoromethyl, (iv) nitro, (v) amino, (vi) mono- or di- alkylamino in which the or each alkyl is independently a lower alkyl of from one to six carbon atoms or, in the case of a dialkylamino, in which the two alkyls, when taken together, represent an alkylene of from one to six carbon atoms, (vii) cyano, (viii) lower alkoxy of from one to four carbon atoms, and (ix) lower thioalkyl of from one to four carbon atoms;

(4) arylalkylene in which the aryl part is phenyl optionally substituted with one or more of (i) alkyl of from one to four carbon atoms, (ii) halo, (iii) trifluoromethyl, (iv) nitro, (v) amino, (vi) mono- or di-alkylamino in which the or each alkyl is independently a lower alkyl of from one to six carbon atoms or, in the case of a dialkylamino, in which the two alkyls, when taken together, represent an alkylene of from one to six carbon atoms, (vii) cyano, (viii) lower alkoxy of from one to four carbon atoms, (ix) lower thioalkyl of from one to four carbon atoms; and the alkylene part has from one to four carbon atoms; or

(5) thienyl, furanyl, pyryl, pyridinyl, quinolyl, isoquinolyl, pyrimidyl, pyridazinyl, quinazolyl, quinoxalyl, benzothienyl, pyrazolyl, imidazolyl, oxazolyl, isoxazolyl, thiazolyl, triazolyl, pyrazinyl, oxazinyl, thiazinyl, indolizinyl, indolyl, benzofuranyl, indazolyl, benzothienyl, benzimidazolyl, benzoxazolyl, benzioxazolyl, benzthiazolyl, benztriazolyl, benzoxadiazolyl, cinnolinyl, phthalaxinyl, naphthyridinyl, or benzothiazinyl;

and wherein $R_2$ and $R_5$ may also be taken together to form a five- or six-membered ring having, as a member of the ring, a

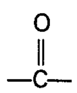 or 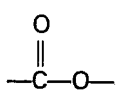 group;

n is zero or an integer of one or two.

Preferred compounds of the present invention are those of the formula:

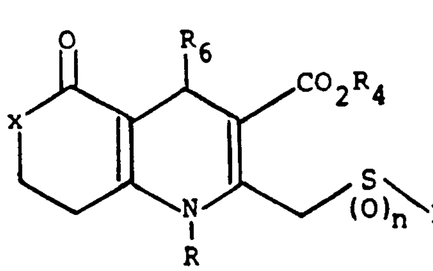

or

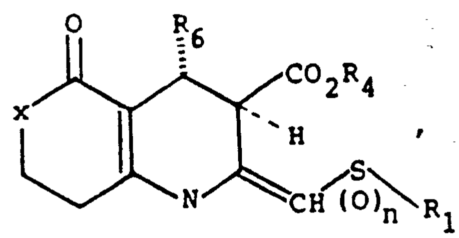

,

wherein X is $>CH_2$ or an oxygen atom.

Other preferred compounds of Formulae I and II are those in which R is hydrogen, $R_2$ is cyano, $R_3$ is $—CO_2R_4$, $R_5$ is methyl and m is one.

Other preferred compounds of Formulae II are those in which R is hydrogen, $R_2$ and $R_3$ are independently $—CO_2R_4$, $R_5$ is methyl and m is one.

The present invention also relates to a pharmaceutical composition comprising a therapeutically

effective amount of a compound of Formula I or II as each is defined above with a pharmaceutically acceptable carrier. A therapeutically effective amount comprises a therapeutic cardiovascular effective amount.

The compounds of this invention are useful in a method of treating cardiovascular disorders in a mammal, including man, suffering therefrom, which method comprises administering to such mammals a therapeutically effective amount of a compound of Formula I or a compound of Formula II.

The preferred compounds of the present invention are (1) the compounds of Formula II wherein $R_2$ is CN and n is one or two; (2) the compounds of Formula I and II wherein $R_2$ and $R_5$ may also be taken together to form a five- or six-membered ring having as a member of the ring a —C(O)— or —C(O)O group and n is one or two.

The preferred compounds provide an increased myocardial contractility giving the compounds greater value as cardiotonic agents for treatment of cardiovascular disorders. These compounds demonstrate only positive inotropic activity in in vitro tests which activity is associated with increased myocardial contractility (PCSI). That is, these compounds do not demonstrate undesirable negative inotropic activity usually associated with these types of compounds.

The most preferred compounds of the present invention are the compounds of Formula I wherein $R_5$ is lower alkyl, $R_3$ is $CO_2R_4$, m is one, n is one or two, $R_4$ is the same as defined above and $R_1$ is phenyl or heteroaryl as defined herein.

Also included in the invention are methods using novel compounds of Formula II of the present invention wherein n is zero having the disclosed utility recited herein or compounds analogous to those of Formula I in which n is zero as intermediates for the preparation of the 1,4-dihydropyridine derivatives of the invention therefrom having sulfinyl or sulfonyl moieties.

The salts of the compounds of Formula I or Formula II; possible when a basic nitrogen is present, are prepared in a manner which is in itself known by reacting the compounds obtained according to the processes of the invention with suitable acids. For example, the appropriate compounds of Formula I or II are dissolved in ethanol, then suitable acids are added to the solution.

Examples of inorganic and organic acids which form physiologically acceptable acid addition salts with the dihydropyridines of the present invention are hydrogen halide acids, such as hydrochloric acid and hydrobromic acid, particularly hydrochloric, phosphoric, sulfuric, nitric, monofunctional and bifunctional carboxylic acids and hydroxycarboxylic acids, such as acetic, maleic, succinic, fumaric, tartaric, citric, salicyclic, lactic, and 1,5-naphthalenedicarboxylic and naphthalenedicarboxylic acid and naphthalenedisulphonic acids.

Lower alkyl or alkyl of from one to four carbons, inclusive, is meant to include a straight or branched chain alkyl group such as methyl, ethyl, propyl, butyl, and isomers thereof.

Halogen or halo means fluorine, chlorine, bromine, or trifluoromethyl.

Lower alkoxy and lower thioalkyl of from one to four carbons, inclusive is also meant to include methyl, ethyl, propyl, butyl, and isomers thereof.

Mono- or di- alkylamino is meant to include methylamino, dimethylamino, ethylamino, N-methyl-N-ethylamino, propylamino, diisopropylamino, butylamino, N-ethyl-N-ethylamino, N-butyl-N-ethylamino and the like.

A heteroaryl means thienyl, furanyl, pyryl, pyridinyl, quinolyl, isoquinolyl, pyrimidyl, pyridazinyl, quinazolyl, quinoxalyl, benzothienyl, pyrazolyl, imidazolyl, oxazolyl, isoxazolyl, thiazolyl, triazolyl, pyrazinyl, oxazinyl, thiazinyl, indolizinyl, indolyl, benzofuranyl, indazolyl, benzothienyl, benzimidazolyl, benzoxazolyl, benzioxazolyl, benzthiazolyl, benztriazolyl, benzoxadiazolyl, cinnolinyl, phthalaxinyl, naphthyridinyl, or benzothiazinyl.

Alkylene of from one to four carbons, inclusive, are methylene, ethylene, propylene, butylene, or isomers thereof.

The compounds of Formula I are useful as salts derived from physiologically acceptable acids. The acids may be mineral acids such as hydrochloric acid, sulfuric acid, and the like, or organic acids, such as ethanesulfonic acid, benzenesulfonic acid, p-toluenesulfonic acid, and the like; giving the hydrochloride, sulfamate, ethanesulfonate, benzenesulfonate, p-toluenesulfonate, and the like, respectively.

The compound of Formula I and II of the present invention wherein at least $R_2$ and $R_3$ are not just the same as each other, includes stereoisomers due to the presence of at least one asymmetric carbon atom at the four position of the 1,4-dihydropyridine nucleus and can exist as each optical isomer or a racemic mixture. Further, some of the compounds of Formula I and II which have not less than two asymmetric carbon atoms in its molecule may exist as each diastereomer(s) or the mixture thereof. The mixture of the diastereomers can be resolved to each racemic compound by conventional resolution methods such as chromatography or fractional recrystallization and the like and the racemic compound can be resolved into each optical isomer by a conventional method for racemic resolution by fractional recrystallization of a salt of the racemic compound with an optically active acid, e.g., tartaric acid or camphor sulfonic acid.

The terms "stereoisomers" and "stereoisomerism" as used throughout this specification and the appended claims are to be given the meaning usually ascribed to them by practitioners of the organic chemical arts, and specifically as defined by Eliel in "Stereochemistry of Carbon Compounds," pp 1—6, McGraw-Hill, New York, 1962.

Generally, the compounds of Formula I and Formula II wherein n is one or two may be conveniently

synthesized by oxidation of the compounds of Formula II wherein n is 0 or compounds analogous to those of Formula I in which n is zero. Such oxidation is accomplished in a manner analogous to those known in the art. See, for example, "Oxidation of Sulfides to Sulfoxides and Sulfones" in Advanced Organic Chemistry, Ed. Jerry, March, p. 1089, 3rd Edition, John Wiley & Sons (1985). See Scheme I.

The compounds of Formula II wherein n is zero and the compounds analogous to those of Formula I in which n is zero are generally prepared by the following procedures.

A compound having the Formula III wherein $R_2$ and $R_5$ are as defined above a compound of the Formula IV wherein $R_6$ is as defined above and a compound of Formula V wherein $R_1$ and $R_3$ are as defined above is contacted in the presence of an inert organic solvent such as ethanol, 1-butanol, or dioxane under reflux conditions. The products of the reaction are a mixture of the compounds of Formula $I_o$ and $II_o$. Such products are recovered and/or separated by conventional means such as extraction, distillation, chromatography, and the like. See Scheme II.

The compounds of Formula $I_o$ and $II_o$ may optionally be reacted with a compound of Formula RX wherein R is as defined above and X is halogen by conventional methods to further obtain the compounds having a definition of R other than hydrogen. See Scheme III.

The desired compound of Formula I or Formula II is isolated and purified by conventional methods. Such isolation and purification do not necessitate any methods not within the skills of the ordinary artisan.

Representative novel compounds of Formula I and of Formula II are found to possess inotropic and vasodilatory activity in vitro and as such, these are useful for the treatment of congestive heart failure, coronary heart disease, myocardial ischemia, angina and hypertension. These are, thus, the cardiovascular disorders noted above to be within the meaning of the present invention.

Representative compounds of Formula I and II inhibit niterendipine (a reference calcium antagonist) binding with an $IC_{50}$ of $1 \times 10^{-6}$M to $1 \times 10^{-9}$M range. These data predict calcium modulator activity beneficial for the utility of the present invention.

It is now additionally found that some of the above named preferred compounds advantageously demonstrate positive inotropic effect in vitro.

For the preferred compounds of the present invention as defined above, the compounds also produce effects that include increases in contractility and decreases in vascular resistance, with only moderate increases in heart rate in anesthetized dogs. These effects are further complemented by a direct stimulatory effect on the myocardium (i.e., a positive inotropic effect). The complementary effects are observed in isolated atrial tissues and the effects are not subject to negative inotropic effect. Thus, the compounds having the combined activity may provide support to a failing heart without off-setting negative effects usually associated with previously known calcium blocking agents.

Additionally, the compounds of the present invention generally exhibit an advantageous vasodilatory effect resulting in the after load reduction associated with calcium antagonists.

Test for In Vitro Myocardial Inotropic Activity
in Isolated Guinea Pig Atria

Adult male guinea pigs weighing 300—400 g are reserpenized (2000 units i.p.) and, after ten minutes, are killed by cervical dislocation. The hearts are quickly removed and placed in a physiological salt solution (PSS) containing: 118 mM NaCl, 4.8 mM KCl, 2.4 mM CaCl, 1.2 mM $MgSO_4$, 1.0 mM $NaH_2PO_4$, 11.1 m dextrose, and 27.2 mM $NaHCO_3$, maintained at pH 7.4 and 30°C. After removing all superficial blood, the hearts are perfused via the aorta to remove any blood from the coronary arteries. After five to ten minutes of perfusion, the left atria are dissected and mounted vertically in a 50 ml, water-jacketed bather containing PSS at 30°C and continuously oxygenated with 95% $O_2$/5% $CO_2$. The left atria are electrically stimulated at 1.5 Hz. The tissues are equilibrated for 30 minutes and washed with fresh PSS. The stimulation voltage is adjusted to 20% above the threshold and tension adjusted to Lmax (the muscle length at which tension development is maximal). The tissues are then equilibrated for an additional 30 minutes, after which the compounds to be examined are added to the tissue bather. The compounds are initially dissolved in DMSO and dilutions made with normal saline, such that the final concentration of DMSO does not exceed 0.5%. The compounds are added in full or half-log concentration increments, usually over a concentration range of $1 \times 10^{-8}$ to $1 \times 10^{-4}$M. The muscles are generally allowed to assume a new steady-state contractile response before each subsequent addition of drug.

Using this procedure the inotropic activity of preferred compounds of the present invention is shown as found in the following Table 1. The reference calcium antagonist has also been tested for comparison.

TABLE 1

% Change in Myocardial Contractility (PSCI)

| Example | @ $10^{-5}$M |
|---|---|
| 2 Isomer A | +33 |
| 2 Isomer B | +53 |
| 2B | +30 |
| 2D | +26 |
| 3A | +26 |
| 3C | +33 |
| 3D | +33 |

The compounds of the present invention are useful in a method for treating mammals, including humans, suffering from the diseases noted above by administering to such mammals a corresponding pharmaceutical composition containing a compound of the Formula I as defined above in appropriate unit dosage form. A physician or veterinarian of ordinary skill readily determines a subject exhibiting symptoms of the diseases. The routes of administration and the dosage forms therefor are from among those conventional to the pharmaceutical art. Regardless of the route of administration selected the invention provides a compound of Formula I, in unit dosage form comprising the compound either alone or in admixture with a pharmaceutically acceptable carrier appropriately selected from those known.

An effective but nontoxic quantity of the compound I is employed in treatment. The dosage regimen is selected in accordance with a variety of factors including the type, age, weight, sex, and medical condition of the mammal, the severity of symptoms of the disease being treated, the route of administration and particular compound of Formula I employed. An ordinarily skilled physician or veterinarian will readily determine and prescribe the effective amount of the compound I to prevent or arrest the progress of the disease condition. In so proceeding, the physician or veterinarian could employ relatively low dosages at first, subsequently increasing the dose until a maximum response is obtained. For convenience, the total daily dosage may be divided and administered in portions during the day if desired.

It is understood that the compositions and methods of treatment of the present invention as described above also include the pharmacologically acceptable acid addition salts of the compounds of Formula I and Formula II.

The following Examples (other than the Reference and Preparative Examples) further merely illustrate the invention and, therefore, are not meant to be limiting.

## REFERENCE EXAMPLES

### Example 1A

Ethyl 5-cyano-1,4-dihydro-6-methyl-2-[(phenylthio]methyl]-4-[2-(trifluoromethylphenyl]-3-pyridinecarboxylate

A solution of 2-trifluoromethyl benzaldehyde (3.48 g, 0.02 mole), 3-aminocrotononitrile (1.6 g, 0.02 mole) and ethyl 3-oxo-4-(phenylthio)butanoate (4.76 g, 0.02 mole) in ethanol (50 ml) is refluxed for 24 hours. The solution is concentrated to a brown oil from which the above product is isolated by chromatography on silica gel with isopropyl ether as eluent. The crude product was crystallized from ethyl acetate to give analytically pure material; mp 122—123°C.

| | | | |
|---|---|---|---|
| Anal. cald. for $C_{24}H_{21}F_3O_2N_2S$: | C, 62.88; | H, 4.58; | N, 6.11 |
| Found: | C, 62.85; | H, 4.73; | N, 6.01 |

### Example 1B

The chromatographic separation also provides the isomeric compound 3-pyridinecarboxylic acid, 5-cyano-1,2,3,4-tetrahydro-2-[(phenylthio]methylene]-6-methyl-4-[(2-trifluoromethyl)phenyl]-, ethyl ester; mp 146—148°C.

Anal. cald. for $C_{24}H_{21}F_3O_2N_2S$: C, 62.87; H, 4.61; N, 6.1; S, 6.99
Found: C, 63.40; H, 4.83; N, 6.31; S, 6.91

Similarly, by substituting 1-amino cyclohexen-3-one for the 3-aminocrotononitrile in Example 1A one obtains:

Example 1C

3-Quinolinecarboxylic acid, 1,4,5,6,7,8-hexahydro-5-oxo-2-[(phenylthio)methyl]-4-[2-trifluoromethyl)phenyl-, ethyl ester; mp 176—177°C.

Chromatographic separation also provides the isomeric compound as follows:

Example 1D

3-Quinolinecarboxylic acid, 1,2,3,4,5,6,7,8-octahydro-5-oxo-2-[(phenylthio)methylene]-4-[2-trifluoromethyl)phenyl-, ethyl ester; mp 204—205°C.

Example 1E

Ethyl 5-cyano-1,4-dihydro-6-methyl-2-[[(2-methylphenyl)thio]methyl]-4-[2-trifluoromethyl)phenyl-pyridinecarboxylate; mp 146—148°.

Example 1F

Ethyl 5-cyano-1,4-dihydro-6-methyl-2-[(phenylthio)methyl]-4-(3-nitrophenyl)-3-pyridinecarboxylate; mp 122—123°C.

Example 1G

Ethyl 4-(2-chlorophenyl)-5-cyano-1,4-dihydro-6-methyl-2-[(phenylthio)methyl]-3-pyridinecarboxylate; mp 161—162°C.

Example 1H

Ethyl 5-cyano-1,4-dihydro-6-methyl-2-[(4-pyridinylthio)methyl]-4-[2-(trifluoromethyl)phenyl-3-pyridinecarboxylate; mp 229—230°C.

Also, similarly, by using appropriate corresponding starting materials in the procdure described in Example 1A, the following compounds are obtained.

Example 1I

3-Pyridinecarboxylic acid, 5-cyano-2-[[[4-(1,1-dimethylethyl)-2-methylphenyl]thio]methyl]-1,4-dihydro-6-methyl-4-[2-(trifluoromethyl)phenyl]-, ethyl ester; mp 126—128°C.

Example 1J

Ethyl 3-cyano-1,4-dihydro-6-methyl-2-[(phenylthio)methyl]-4-[2-(trifluoromethyl)phenyl]-5-pyridinecarboxylate; mp 144—145°C.

Example 1K

3-Pyridinecarboxylic acid, 5-cyano-1,4-dihydro-2-[[(4-methoxyphenyl)thio]methyl]-6-methyl-4-[2-(trifluoromethyl)phenyl]-, ethyl ester; mp 108—109°C.

Example 1L

3-Pyridinecarboxylic acid, 2-[[(2-chlorophenylthio]methyl]-5-cyano-1,4-dihydro-6-methyl-4-[2-(trifluoromethyl)phenyl]-, ethyl ester; mp 172—173°C.

Example 1M

3-Pyridinecarboxylic acid, 5-cyano-2-[[(2-fluorophenyl)thio]methyl]-1,4-dihydro-6-methyl-4-[2-(trifluoromethyl)phenyl]-, ethyl ester; mp 121—122°C.

Example 1N

3-Pyridinecarboxylic acid, 2-[[[4-(acetylaminophenyl)thio]methyl]-5-cyano-1,4-dihydro-6-methyl-4-[2-(trifluoromethyl)phenyl]-, ethyl ester; mp 187—189°C.

Example 1O

3-Pyridinecarboxylic acid, 2-[[(4-chlorophenyl)thio]methyl]-5-cyano-1,4-dihydro-6-methyl-4-[2-(trifluoromethyl)phenyl]-, ethyl ester; mp 107—109°C.

Example 1P

3-Pyridinecarboxylic acid, 5-cyano-1,4-dihydro-6-methyl-4-phenyl-2-[(phenylthio)methyl]-, ethyl ester; (Isomer A); mp 168—170°C.

Example 1Q

3-Pyridinecarboxylic acid, 5-cyano-4-(2-furanyl-1,4-dihydro-6-methyl-2-[(phenylthio)methyl]-, ethyl ester; mp 109—110°C.

Example 1R

3-Pyridinecarboxylic acid, 5-cyano-1,4-dihydro-6-methyl-4-(2-phenyl-1*H*-imidazol-4-yl)-2-[(phenylthio)methyl]-, ethyl ester; mp 198—199°C.

Example 1S

3-Pyridinecarboxylic acid, 5-cyano-1,4-dihydro-6-methyl-2-[(phenylthio)methyl]-(2-pyridinyl)-, ethyl ester; mp 164—167°C.

Example 1T

3-Pyridinecarboxylic acid, 5-cyano-1,4-dihydro-6-methyl-2-[(2-pyridinylthio)methyl]-4-[2-(trifluoromethyl)phenyl-, ethyl methyl; mp 179—180°C.

Example 1U

3-Pyridinecarboxylic acid, 5-cyano-1,4-dihydro-6-methyl-2-[(phenylthio)methyl]-4-[2-(trifluoromethyl)phenyl-, 2-cyanoethyl ester; mp 158—160°C.

Example 1V

3-Pyridinecarboxylic acid, 5-cyano-1,4-dihydro-6-methyl-2-[(phenylthio)methyl]-4-[2-(trifluoromethyl)phenyl]-; mp 204—206°C.

Example 1W

3-Pyridinecarboxylic acid, 5-cyano-1,4-dihydro-6-methyl-2-[(phenylthio)methyl]-4-[2-(trifluoromethyl)phenyl]-, 1-methylethyl ester; mp 123—125°C.

Example 1X

3-Pyridinecarboxylic acid, 5-cyano-1,4-dihydro-6-methyl-2-[(phenylthio)methyl]-4-[2-(trifluoromethyl)phenyl]-, methyl ester; mp 117—118°C.

Example 1Y

3-Pyridinecarboxylic acid, 5-cyano-1,4-dihydro-6-methyl-2-[(phenylthio)methyl]-4-[2-(trifluoromethyl)phenyl]-, 2-propenyl ester; mp 113—114°C.

Example 1Z

Following the procedure of Example 1A and by substituting ethyl 3-aminocrotonate in place of 3-aminocrotonitrile, one obtains 3,5-pyridinedicarboxylic acid, 1,4-dihydro-2-methyl-6-[(phenylthio)methyl]-4-[2-(trifluoromethyl)phenyl]-, diethyl ester; mp 129—130°C.

| | | | | |
|---|---|---|---|---|
| Anal. calcd. for $C_{26}H_{26}F_3O_4NS$: | C, 61.72; | H, 5.14; | N, 2.76; | S, 6.33 |
| Found: | C, 61.98; | H, 5.31; | N, 2.74; | S, 6.37 |

Example 1AA

3,5-Pyridinedicarboxylic acid, 1,4-dihydro-6-methyl-2-[[(2-methylphenyl)thio]methyl]-4-[2-(trifluoromethyl)phenyl]-, diethyl ester; mp 117—119°C.

Similarly by using appropriate starting materials in the procedure described in Example 1Z, the following additional analogs are obtained.

Example 1BB

3,5-Pyridinedicarboxylic acid, 1,4-dihydro-2-methyl-6-[(2-pyridinylthio)methyl]-4-[2-(trifluoromethyl)phenyl]-, diethyl ester; mp 118—119°C.

Example 1CC

3,5-Pyridinedicarboxylic acid, 1,4-dihydro-2-methyl-6-[(4-pyridinylthio)methyl]-4-[2-(trifluoromethyl)phenyl]-, diethyl ester; mp 146—148°C.

Example 1DD

3,5-Pyridinedicarboxylic acid, 1,4-dihydro-2-methyl-4-(2-phenyl-1*H*-imidazolyl-4-yl)-6-[(phenylthio)methyl]-, diethyl ester; mp 179—180°C.

Example 1EE

[3,4′-Bipyridine]-3′,5′-dicarboxylic acid, 1′,4′-dihydro-2′-methyl-6′-[(phenylthio)methyl]-, diethyl ester; mp 116—118°C.

Example 1FF

3,5-Pyridinedicarboxylic acid, 2-[[(2-chlorophenyl)thio]methyl-1,4-dihydro-6-methyl-4-[2-(trifluoromethyl)phenyl]-, 3-ethyl 5-methyl ester; mp 123—125°C.

EXAMPLES OF THE INVENTION
(unless otherwise indicated)

Example 2

Ethyl 5-cyano-1,4-dihydro-6-methyl-2-[(phenylsulfinyl)methyl]-4-[2-(trifluoromethyl)phenyl]-3-pyridinecarboxylate

m-Chloroperbenzoic acid (0.43 g, 0.002 mole) is added to a solution of ethyl-5-cyano-1,4-dihydro-6-methyl-2-[(phenylthio)methyl]-4-[2-(trifluoromethyl)phenyl]-3-pyridinecarboxylate as prepared above in Example 1 (0.96 g, 0.002 mole) in 20 ml of dichloromethane with stirring at 0°C.

The solution is stirred for two hours at 0°C to complete the reaction. The solution is washed with 10% aqueous potassium carbonate solution followed by water, dried, and stripped to give a semisolid product. This is purified by chromatography to give two isomers of ethyl-5-cyano-1,4-dihydro-6-methyl-2-[(phenylsulfinyl)methyl]-4-[2-(trifluoromethyl)phenyl]-3-pyridinecarboxylate.

Isomer A, mp 120—122°C.

| | | | |
|---|---|---|---|
| Anal calcd. for $C_{24}H_{21}F_3N_2O_3S$: | C, 60.75; | H, 4.40; | N, 5.90 |
| Found: | C, 60.35; | H, 4.38; | N, 5.89 |

Isomer B, mp 183—185°C.

| | | | |
|---|---|---|---|
| Anal calcd. for $C_{24}H_{21}F_3N_2O_3S$: | C, 60.75; | H, 4.40; | N, 5.90 |
| Found: | C, 60.72; | H, 4.59; | N, 5.83 |

By following the procedure described in the Example 2 and using substrates selected from among Reference Examples 1 the following additional sulfinyl derivatives are obtained.

Example 2A

Ethyl 5-cyano-1,4-dihydro-6-methyl-2-[[(2-methylphenyl)sulfinyl]methyl]-4-[2-(trifluoromethyl)phenyl]-3-pyridinecarboxylate; mp 176—180°C.

Example 2B

Ethyl 5-cyano-1,4-dihydro-6-methyl-4-(3-nitrophenyl)-2-[(phenyl)sulfinyl]methyl]-3-pyridinecarboxylate; mp 194—195°C [Isomer A]; mp 168—170°C [Isomer B].

Example 2C

Ethyl 5-cyano-1,4-dihydro-6-methyl-2-[[(4-methoxyphenyl)sulfinyl]methyl]-4-[2-(trifluoromethyl)phenyl]-3-pyridinecarboxylate; mp 151—154°C [diastereoisomers].

Example 2D

Ethyl 4-(2-chlorophenyl)-5-cyano-1,4-dihydro-6-methyl-2-[(phenyl)sulfinyl]methyl]-3-pyridinecarboxylate; mp 198—202°C [diastereoisomers].

Example 2E

Ethyl 5-cyano-1,4-dihydro-6-methyl-2-[(4-pyridinylsulfinyl)methyl]-4-[2-(trifluoromethyl)phenyl]-3-pyridinecarboxylate; mp 202—203°C (dec) [Isomer A].

Example 2F
(Reference)

3,5-Pyridinecarboxylic acid, 1,4-dihydro-2-methyl-6-[(4-pyridinylsulfinyl)methyl]-4-[2-(trifluoromethyl)phenyl]-, diethyl ester; mp 202—203°C.

Example 2G

Ethyl 2-[[(2-chlorophenyl)sulfinyl)methyl]-5-cyano-1,4-dihydro-6-methyl-4-[(2-trifluoromethyl)phenyl]-3-pyridinecarboxylate; mp 174—184°C.

Example 2H

5-Cyano-2-[[[2-(dimethylamino)ethyl]sulfinyl]methyl]-1,4-dihydro-6-methyl-4-[(2-trifluoromethyl)phenyl]-3-pyridinecarboxylic acid, ethyl ester.

Example 2I

Ethyl 4-(2-chlorophenyl)-5-cyano-1,4-dihydro-6-methyl-2-[(4-pyridinylsulfinyl)methyl-3-pyridinecarboxylate; mp 236—237°C.

Example 2J

Ethyl 5-cyano-4-(2,3-dichlorophenyl)-1,4-dihydro-6-methyl-2-[4-pyridinylsulfinyl)methyl-3-pyridinecarboxylate; mp.

Example 2K

5-Cyano-1,4-dihydro-6-methyl-2-[(2-pyrimidinsulfinyl)methyl]-4-[2-(trifluoromethyl)phenyl]-3-pyridinecarboxylic acid, ethyl ester; mp 151—154°C (dec).

Example 2L

5-Cyano-1,4-dihydro-6-methyl-2-[[(1-methyl-1*H*-imidazol-2-yl)sulfinyl]methyl]-4-[2-(trifluoromethyl)phenyl]-3-pyridinecarboxylic acid, ethyl ester.

Example 2M

Ethyl 5-Cyano-1,4-dihydro-6-methyl-2-[(phenylsulfinyl)methyl]-4-phenyl-3-pyridinecarboxylate.

Example 2N

3,5-Pyridinedicarboxylic acid, 2-[[(2-chlorophenyl)sulfinyl]methylene-6-methyl-1,2,3,4-tetrahydro-4-[(2-(trifluoromethyl)phenyl]-3-ethyl-5-methyl ester; mp 153—154°C.

Example 2O

3-Pyridinecarboxylic acid, 5-cyano-4-cyclohexyl-1,4-dihydro-6-methyl-2-[(phenylsulfinyl)methyl-, ethyl ester; mp 144—146°C.

Example 2P

3-Pyridinecarboxylic acid, 5-cyano-4-[2-(difluoromethoxy)phenyl]-1,4-dihydro-6-methyl-2-[(phenylsulfinyl)methyl-, ethyl ester; mp 122—124°C.

Example 2Q

3-Pyridinecarboxylic acid, 5-cyano-1,4-dihydro-6-methyl-2-[(phenylsulfinyl)methyl-4-[2-(trifluoromethyl)phenyl]-, methyl ester; mp 207—210°C.

Example 2R

3-Pyridinecarboxylic acid, 5-cyano-1,4-dihydro-6-methyl-2-[(phenylsulfinyl)methyl]-4-[2-(trifluoromethyl)phenyl]-, 1-methylethyl ester; Isomer A; mp 189—191°C.

Example 2S

3-Pyridinecarboxylic acid, 5-cyano-1,4-dihydro-6-methyl-2-[(phenylsulfinyl)methyl]-4-[2-(trifluoromethyl)phenyl]-, 2-hydroxyethyl ester; mp 172—173°C.

Example 2T

3-Pyridinecarboxylic acid, 5-cyano-1,4-dihydro-6-methyl-4-(2-phenyl-1*H*-imidazol-4-yl)-2-[(phenylsulfinyl)methyl]-, ethyl ester, (diastereoisomers); mp 211—212°C.

Example 2U

3-Pyridinecarboxylic acid, 5-cyano-1,4-dihydro-6-methyl-2-[(2-pyridinylsulfinyl)methyl]-4-[2-(trifluoromethyl)phenyl]-, ethyl ester; mp 155—174°C (dec).

Example 2V

3-Pyridinecarboxylic acid, 5-cyano-2-[[(2-fluorophenyl)sulfinyl]methyl]-1,4-dihydro-6-methyl-4-[2-(trifluoromethyl)phenyl]-, ethyl ester, (diastereoisomers); mp 186—187°C.

Example 2W

3-Pyridinecarboxylic acid, 2-[[[4-(acetylamino)phenyl]sulfinyl]methyl]-5-cyano-1,4-dihydro-6-methyl-4-[2-(trifluoromethyl)phenyl]-, ethyl ester, (mixture of isomers); mp 228—230°C.

Example 2X

3-Pyridinecarboxylic acid, 2-[[(4-chlorophenyl)sulfinyl]methyl]-5-cyano-1,4-dihydro-6-methyl-4-[2-(trifluoromethyl)phenyl]-, ethyl ester, mp 207—210°C.

Example 2Y

3-Pyridinecarboxylic acid, 5-cyano-2-[[(3,4-dichlorophenyl)sulfinyl]methyl]-1,4-dihydro-6-methyl-4-[2-(trifluoromethyl)phenyl]-, ethyl ester, (diastereoisomers); mp 173—176°C.

Example 2Z

3-Pyridinecarboxylic acid, 5-cyano-1,4-dihydro-6-methyl-2-[(phenylsulfinyl]methyl]-4-[2-((trifluoromethyl)phenyl]-; mp 209—211°C.

Example 2AA

3-Quinolinecarboxylic acid, 1,4,5,6,7,8-hexahydro-1-methyl-5-oxo-2-[(phenylsulfinyl)methyl]-4-[2-(trifluoromethyl)phenyl]-, ethyl ester; mp 177—178°C.

Example 2BB

3-Quinolinecarboxylic acid, 1,4,5,6,7,8-hexahydro-5-oxo-2-[(phenylsulfinyl)methyl]-4-[2-(trifluoromethyl)phenyl]-, ethyl ester, (±)-; mp 223—224°C.

Example 2CC
(Reference)

Ethyl 3-cyano-1,4-dihydro-6-methyl-2-[(phenylsulfinyl)methyl]-4-[2-(trifluoromethyl)phenyl]-5-pyridinecarboxylate.

Example 2DD

3-Pyridinecarboxylic acid, 5-cyano-1-[2-(diethylamino)ethyl]-1,4-dihydro-6-methyl-2-[(phenylsulfinyl)methyl]-4-[2-(trifluoromethyl)phenyl]-, ethyl ester; (mixture of isomers); mp 128—131°C.

Example 2EE

3-Pyridinecarboxylic acid, 5-cyano-1,4-dihydro-2-[[(3-methoxy-3-oxopropyl]sulfinyl]methyl]-6-methyl-4-[2-(trifluoromethyl)phenyl]-, ethyl ester; mp 135—137°C.

Example 2FF

3-Pyridinecarboxylic acid, 5-cyano-2-[[(2-ethoxy-2-oxoethyl)sulfinyl]methyl]-1,4-dihydro-6-methyl-4-[2-(trifluoromethyl)phenyl]-, ethyl ester; mp 127—130°C.

Example 2GG

[2,4'-Bipyridine]-3'-carboxylic acid, 5'-cyano-1',4'-dihydro-6'-methyl-2'-[(phenylsulfinyl]methyl]-, ethyl ester; mp 167—170°C.

By following the procedure described in the Example 2 and using substrates from Example 1Z, the following additional sulfinyl derivatives are obtained.

Example 2HH
(Reference)

3,5-Pyridinedicarboxylic acid, 1,4-dihydro-6-methyl-2-[[(2-methylphenyl)sulfinyl]methyl]-4-[2-(trifluoromethyl)phenyl], diethyl ester; mp 143—147°C [Isomer A], mp 140—144°C [Isomer B].

Example 2II
(Reference)

3,5-Pyridinedicarboxylic acid, 1,4-dihydro-2-methyl-6-[(phenylsulfinyl)methyl]-4-[2-(trifluoromethyl)-phenyl]-, diethyl ester; mp 142—143°C.

Example 2JJ
(Reference)

3,5-Pyridinedicarboxylic acid, 1,4-dihydro-2-methyl-6-[(2-pyridinylsulfinyl)methyl]-4-[2-(trifluoromethyl)-phenyl]-, diethyl ester; mp 182—183°C.

Example 2KK
(Reference)

3,5-Pyridinedicarboxylic acid, 1,4-dihydro-2-[[(4-methoxyphenyl)sulfinyl)methyl]-6-methyl-4-[2-(trifluoromethyl)phenyl]-, diethyl ester; Isomer A, mp 190—191°C; Isomer B, mp 159—160°C.

Example 3
(Reference)

3,5-Pyridinedicarboxylic acid, 1,4-dihydro-2-methyl-6-[(phenylsulfonyl)methyl-4-[2-(trifluoromethyl)phenyl]diethyl ester

m-Chloropenbenzoic acid (0.4 g, 0.002 mole) is added with stirring to a solution of 0.5 g (0.001 mole) of 3,5-pyridinedicarboxylic acid, 1,4-dihydro-6-methyl-2-[(2-phenylthio)methyl]-4-[2-(trifluoromethyl)phenyl]-diethyl ester in 20 ml of dichloromethane at room temperature. The reaction mixture is stirred for four hours at room temperature to complete the reaction. The solution is washed successively with dilute sodium bicarbonate solution followed by water, dried, and stripped to give a solid. This is purified by chromatography to give pure 3,5-pyridinecarboxylic acid, 1,4-dihydro-2-methyl-6-[(phenylsulfonyl)methyl-4-[2-(trifluoromethyl)phenyl]diethyl ester; mp 194—195°C.

| | | | |
|---|---|---|---|
| Anal. calcd. for $C_{26}H_{26}NF_3O_6S$: | C, 58.04; | H, 4.83; | N, 2.60 |
| Found: | C, 58.36; | H, 5.14; | N, 2.58 |

Following the procedure of Reference Example 3 and using substrates selected from among Reference

Examples 1, additional sulfonyl derivatives were obtained.

Example 3A

Ethyl 5-cyano-1,4-dihydro-6-methyl-2-[(phenylsulfonyl)methyl]-4-[2-(trifluoromethyl)phenyl]-3-pyridinecarboxylate; mp 209—211°C.

Example 3B

Ethyl 5-cyano-1,4-dihydro-6-methyl-2-[[(4-methoxyphenyl)sulfonyl]methyl]-4-[2-(trifluorophenyl)-3-pyridinecarboxylate; mp 205—208°C.

Example 3C

Ethyl 5-cyano-1,4-dihydro-6-methyl-2-[[(2-methylphenyl)sulfonyl]methyl]-4-[2-(trifluorophenyl)-3-pyridinecarboxylate; mp 223—225°C (dec).

Example 3D

Ethyl 2-[[(2-chlorophenyl)sulfonyl]methyl]-5-cyano-1,4-dihydro-6-methyl-2-[(2-trifluoromethyl)phenyl]-3-pyridinecarboxylate.

Example 3E
(Reference)

3,5-Pyridinecarboxylic acid, 2-[[(2-chlorophenyl)sulfonyl]methyl]-1,4-dihydro-6-methyl-4-[2-(trifluoromethyl)phenyl]-3-ethyl, 5-methyl ester; mp 155—157°C.

Example 3F

Ethyl 5-cyano-1,4-dihydro-6-methyl-2-[(4-pyridinylsulfonyl)methyl]-4-[2-(trifluoromethyl)phenyl]-3-pyridinecarboxylate; mp 219—220°C (dec).

Example 3G

5-Cyano-1,4-dihydro-6-methyl-2-[(2-pyridinylsulfonyl)methyl]-4-[2-(trifluoromethyl)phenyl]-3-pyridinecarboxylic acid, ethyl ester, N-oxide; mp 233—235°C.

Example 3H

Ethyl 5-cyano-1,4-dihydro-6-methyl-2-[(phenylsulfonyl)methyl]-4-phenyl-3-pyridinecarboxylate.

Example 3I

[2,4'-Bipyridine]-3'-carboxylic acid, 5'-cyano-1',4'-dihydro-6'-methyl-2'-[(phenylsulfonyl)methyl]-, ethyl ester, 1-oxide.

Example 3J

[3,4'-Bipyridine]-3'-carboxylic acid, 5'-cyano-1',4'-dihydro-6'-methyl-2'-[(phenylsulfonyl)methyl]-, ethyl ester, (mixture of isomers); mp 201—202°C.

Example 3K

Ethyl 3-cyano-1,4-dihydro-6-methyl-2-[(phenylsulfonyl)methyl]-4-[2-(trifluoromethyl)phenyl-5-carboxylate.

Example 3L

3-Pyridinecarboxylic acid, 2-[[(4-aminophenyl)sulfonyl]methyl]-5-cyano-1,4-dihydro-6-methyl-4-[2-(trifluoromethyl)phenyl]-, ethyl ester; mp 237—240°C.

Example 3M

[2,4'-Bipyridine]-3'-carboxylic acid, 5'-cyano-1',4'-dihydro-6'-methyl-2'-[(phenylsulfonyl)methyl]-, ethyl ester, mp 196—198°C.

Example 3N

3-Pyridinecarboxylic acid, 5-cyano-4-cyclohexyl-6-methyl-1,4-dihydro-2-[(phenylsulfonyl)methyl]-, ethyl ester, mp 150—152°C.

Example 3O

3-Pyridinecarboxylic acid, 5-cyano-1,4-dihydro-6-methyl-2-[(phenylsulfonyl)methyl]-4-[2-(trifluoromethyl)phenyl]-, 1-methylethyl ester; mp 205—207°C.

Example 3P

3-Pyridinecarboxylic acid, 5-cyano-1,4-dihydro-6-methyl-2-[(phenylsulfonyl)methyl]-4-[2-(trifluoromethyl)phenyl]-, 2-propenyl ester; mp 187—189°C.

Example 3Q

3-Pyridinecarboxylic acid, 5-cyano-2-[[[4-(1,1-dimethylethyl)-2-methylphenyl]sulfonyl]methyl]-1,4-dihydro-6-methyl-4-[2-(trifluoromethyl)phenyl]-, ethyl ester; mp 155—159°C.

Example 3R

3-Pyridinecarboxylic acid, 5-cyano-2-[[(2-fluorophenyl)sulfonyl]methyl]-1,4-dihydro-6-methyl-4-[2-(trifluoromethyl)phenyl]-, ethyl ester; mp 212—213°C.

Example 3S

3-Pyridinecarboxylic acid, 2-[[(4-(chlorophenyl)sulfonyl]methyl]-5-cyano-1,4-dihydro-6-methyl-4-[2-(trifluoromethyl)phenyl]-, ethyl ester; mp 230—233°C.

Example 3T

3-Pyridinecarboxylic acid, 5-cyano-1,4-dihydro-6-methyl-4-(3-nitrophenyl)-2-[(phenylsulfonyl)methyl]-, ethyl ester; mp 175—176°C.

Example 3U

3-Pyridinecarboxylic acid, 5-cyano-1,4-dihydro-6-methyl-2-[(phenylsulfonyl)methyl]-4-[2-(trifluoromethyl)phenyl]-, 2,3-dihydroxypropyl ester; mp 102—103°C.

Example 3V

3-Pyridinecarboxylic acid, 5-cyano-1,4-dihydro-2-[[(3-methoxy-3-oxopropyl)sulfonyl]methyl]-6-methyl-4-[2(trifluoromethyl)phenyl]-, ethyl ester; mp 119—120°C.

Example 3W

3-Quinolinecarboxylic acid, 1,4,5,6,7,8-hexahydro-1-methyl-5-oxo-2-[(phenylsulfonyl)methyl]-4-[2-(trifluoromethyl)phenyl]-, ethyl ester; mp 214—215°C.

Example 3X

3-Quinolinecarboxylic acid, 1,4,5,6,7,8-hexahydro-5-oxo-2-[(phenylsulfonyl)methyl]-4-[2-(trifluoromethyl)phenyl]-, ethyl ester; mp 235—236°C.

Example 4
(Reference)

3-Quinolinecarboxylic acid, 1,4,5,6,7,8-hexahydro-1-methyl-5-oxo-2-[(phenylthio)methyl)-4-[2-(trifluoromethyl)phenyl]-, ethyl ester

To a stirred solution of sodium hydride (40 mg, 60% oil dispersion) in 5 ml of tetrahydrofuran is added dropwise a solution of 480 mg of 3-quinolinecarboxylic acid, 1,4,5,6,7,8-hexahydro-5-oxo-2-[(phenylsulfonyl)methyl]-4-[2-(trifluoromethyl)phenyl]- ethyl ester, in 15 ml of tetrahydrofuran. Hydrogen is evolved and a bright orange color develops. Methyl iodide (0.9 g) is added and the reaction mixture is stirred at room temperature for 2 hours. The solvent is distilled, the residue is treated with water and the organic material is extracted with methylene chloride. The organic extract is separated, dried over anhydrous magnesium sulfate and evaporated to dryness. The residue is crystallized from ether to give 250 mg of the title compound; mp 129—130°C.

| | | | |
|---|---|---|---|
| Anal calcd. for $C_{27}H_{26}F_3NO_3S$: | C, 64.67; | H, 5.19; | N, 2.79 |
| Found: | C, 64.75; | H, 5.38; | N, 2.74 |

Starting materials for use in preparation of compounds of the present invention are known or may be prepared according to methods analogous to those described below.

Preparative Examples

Ethyl 3-oxo-4-(4-pyridylthio)butanoate (see compound of the Formula V in Scheme II, wherein $R_3$ is $CO_2Et$ and $R_1$ is 4-pyridyl

Ethyl 4-chloroacetoacetate (12.4 g, 0.075 mole) is added dropwise with stirring to a suspension of 4-mercaptopyridine, (8.3 g, 0.075 mole) in a mixture of tetrahydrofuran (80 ml) and *N,N*-dimethylformamide (40 ml). Extherm is observed and precipitate begins to appear. The reaction mixture is stirred at ambient temperature for three hours and filtered. The filtrate is stripped to remove solvent and used as is without further purification.

Ethyl 3-oxo-4-(phenylthio)butanoate (see compound of the Formula V in Scheme II, wherein $R_3$ is $CO_2Et$ and $R_1$ is phenyl

Ethyl 4-chloroacetoacetate (15 g, 0.09 mole) is added dropwise with stirring to a solution of benzenethiol (10 g, 0.09 mole) in pyridine (15 ml). The reaction mixture is stirred for an additional two hours at ambient temperature. The reaction mixture is poured into water and the oil is extracted with ether. The ether extract is washed successively with 1 N HCl, water, dried over anhydrous $MgSO_4$ and evaporated.

This material is used as is for the next step without further purification.

Anal. calcd. for $C_{12}H_{14}O_3S$: C, 60.48; H, 5.92; S, 13.45
Found: C, 60.18; H, 5.59; S, 14.02

Similarly, by substituting 4-bromo-3-oxo-butane-nitrile (Chem. Ber. 115(1), 355—80) in place of ethyl 4-chloroacetoacetate one obtains 3-oxo-4-(phenylthio)butanenitrile. (Formula V in Scheme II, wherein $R_3$ is CN and $R_1$ is phenyl.)

FORMULA

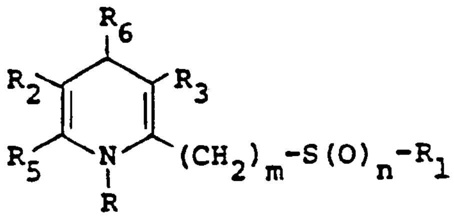

I

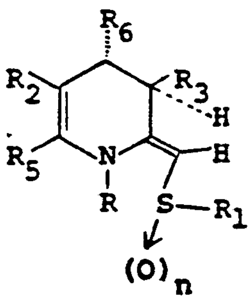

II

SCHEME I

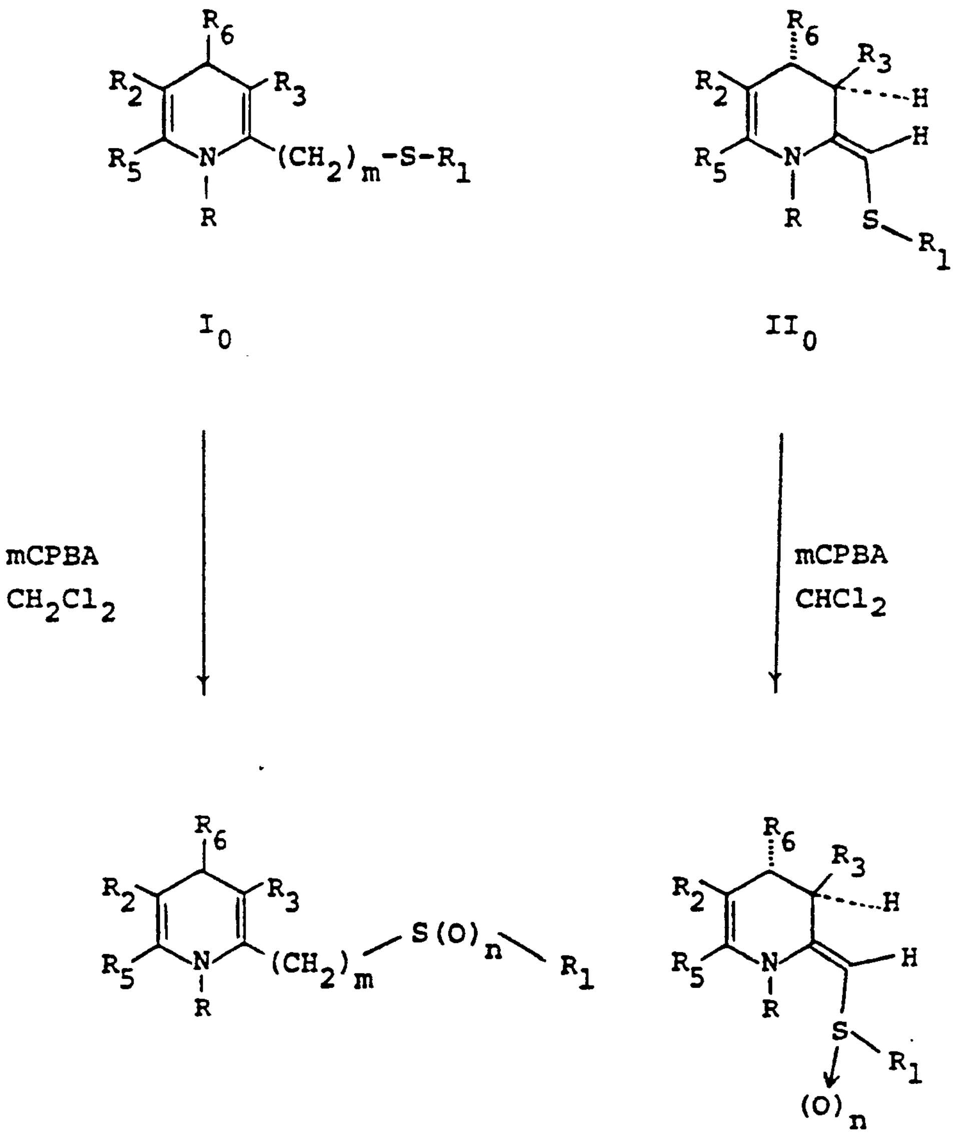


$I_0$ $II_0$

$I_{1-2}$ $II_{1-2}$

where in n is 1 or 2 where in n is 1 or 2

SCHEME II

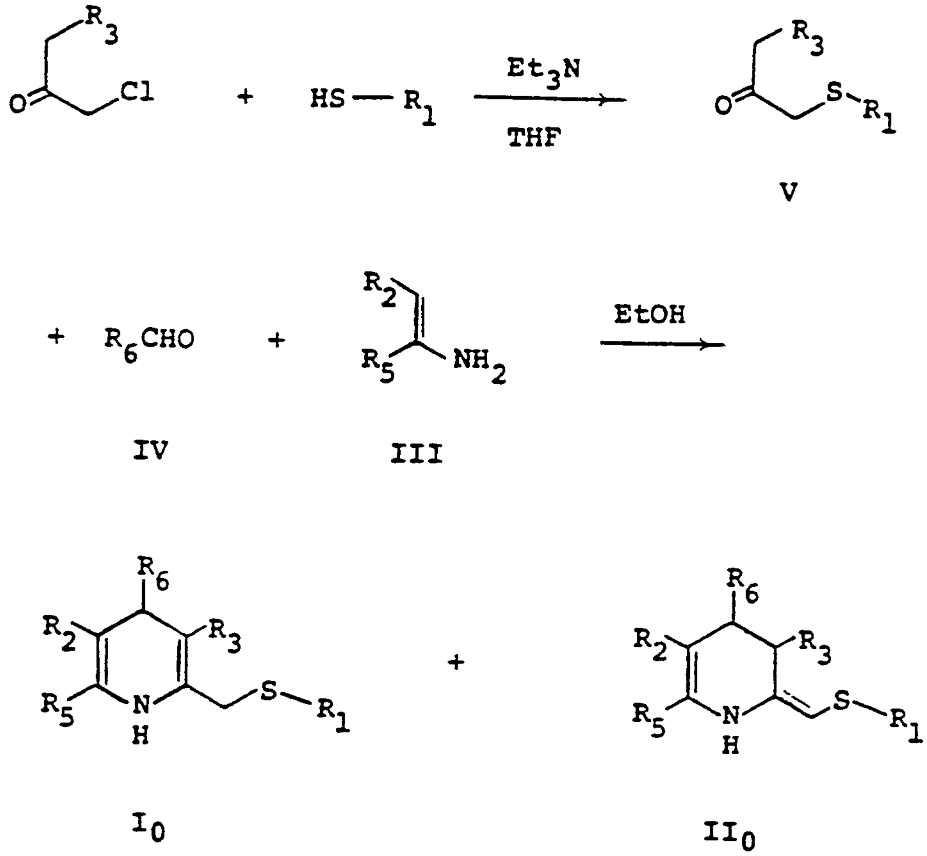


V

IV

III

$I_0$

$II_0$

SCHEME III

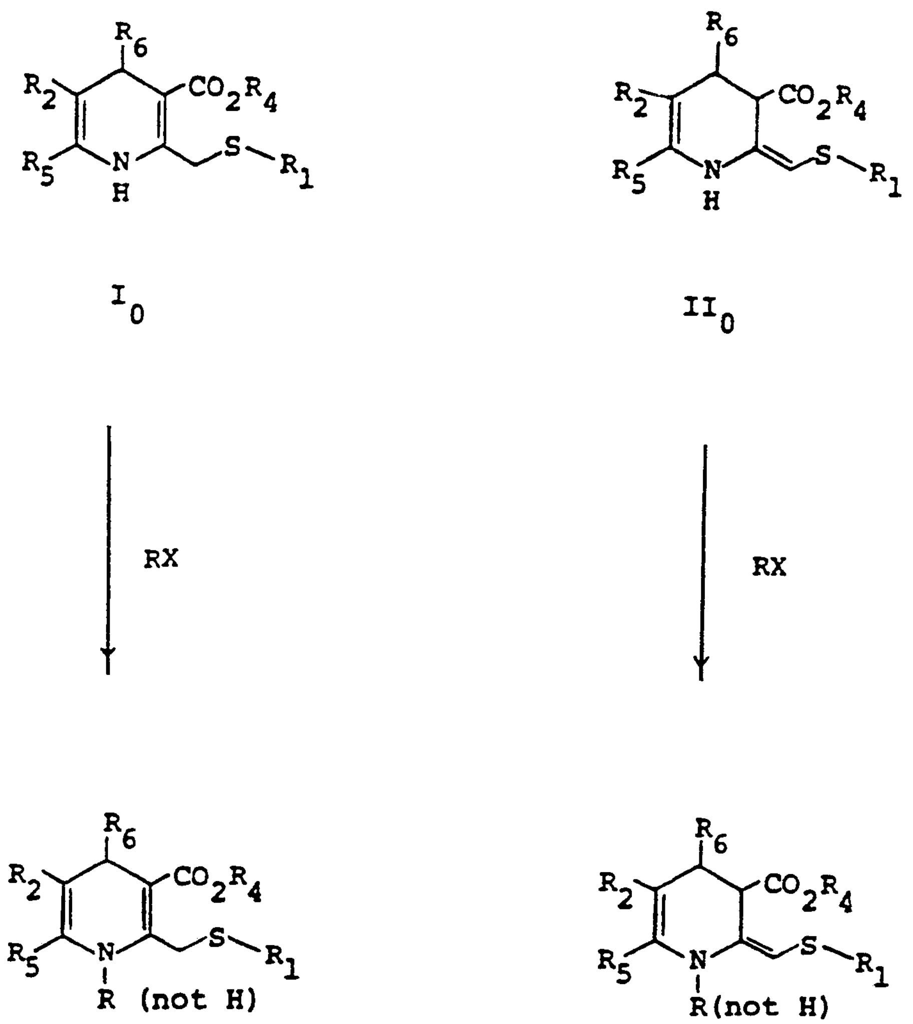


$I_0$ $II_0$

**Claims for the Contracting States: BE CH DE FR GB IT LI LU NL SE**

1. A compound having the following general formula (I)

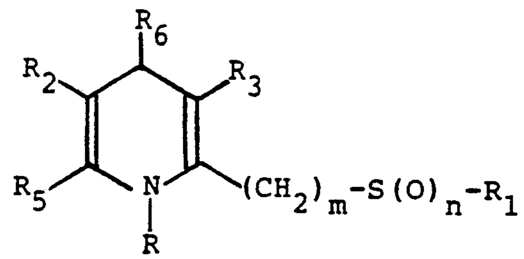

(I)

and a pharmaceutically acceptable salt thereof; wherein

R is

(1) hydrogen;

(2) lower alkyl of from one to four carbon atoms, optionally substituted with an amino group having the formula $NR_7R_8$ in which $R_7$ and $R_8$ are independently hydrogen or lower alkyl of from one to four carbon atoms, or $R_7$ and $R_8$, when taken together with the nitrogen atom to which they are attached form a five- or six-membered ring having four or five $CH_2$ groups; or

(3) an arylalkylene radical in which the aryl part is phenyl optionally substituted with one or more of (i) halo, (ii) trifluoromethyl, (iii) nitro, (iv) amino (v) mono- or di-alkylamino in which the or each alkyl is independently a lower alkyl of from one to six carbon atoms or, in the case of a dialkylamino, in which the two alkyls, when taken together represent an alkylene of from one to six carbon atoms, (vi) cyano, (vii) lower alkoxy of from one to four carbon atoms, (viii) lower thioalkyl of from one to four carbon atoms; and in which the alkylene part has from one to four carbon atoms;

$R_2$ is cyano;

$R_3$ is hydrogen, lower alkyl of from one to four carbon atoms, cyano, nitro, $—CO_2R_4$ in which $R_4$ is hydrogen or lower alkyl of from one to four carbon atoms, optionally substituted with amino, mono-, or dialkylamino in which the or each alkyl is independently a lower alkyl of from one to six carbon atoms or, in the case of a dialkylamino, in which the two alkyls, when taken together, represent an alkylene of from one to six carbon atoms;

$R_1$ is

(1) cycloalkyl of from four to seven carbon atoms;

(2) phenyl optionally substituted with one or more of (i) alkyl having from one to four carbon atoms, (ii) halo, (iii) trifluoromethyl, (iv) nitro, (v) amino, and (vi) mono- or di- alkylamino in which the or each alkyl is independently a lower alkyl of from one to six carbon atoms or, in the case of a dialkylamino, in which the two alkyls, when taken together, represent an alkylene of from one to six carbon atoms; or

(3) thienyl, furanyl, pyryl, pyridinyl, quinolyl, isoquinolyl, pyrimidyl, pyridazinyl, quinazolyl, quinoxalyl, benzothienyl, pyrazolyl, imidazolyl, oxazolyl, isoxazolyl, thiazolyl, triazolyl, pyrazinyl, oxazinyl, thiazinyl, indolizinyl, indolyl, benzofuranyl, indazolyl, benzothienyl, benzimidazolyl, benzoxazolyl, benzioxazolyl, benzthiazolyl, benztriazolyl, benzoxadiazolyl, cinnolinyl, phthalaxinyl, naphthyridinyl, or benzothiazinyl;

$R_5$ and $R_6$, which may be the same or different, are each

(1) lower alkyl of from one to four carbon atoms, optionally substituted with (a) an amino group having the formula $NR_7R_8$ in which $R_7$ and $R_8$ are independently hydrogen or lower alkyl, or $R_7$ and $R_8$, when taken together with the nitrogen atom to which they are attached form a five- or six-membered ring having four or five carbon atoms, (b) $—OR_9$ in which $R_9$ is hydrogen or lower alkyl of from one to four carbon atoms, or (c) $—CO_2R_4$ in which $R_4$ is as defined above;

(2) cycloalkyl of from four to seven carbon atoms;

(3) phenyl optionally substituted with one or more of (i) alkyl having from one to four carbon atoms, (ii) halo, (iii) trifluoromethyl, (iv) nitro, (v) amino, (vi) mono- or di- alkylamino in which the or each alkyl is independently a lower alkyl of from one to six carbon atoms or, in the case of a dialkylamino, in which the two alkyls, when taken together, represent an alkylene of from one to six carbon atoms; (vii) cyano, (viii) lower alkoxy of from one to four carbon atoms, and (ix) lower thioalkyl of from one to four carbon atoms;

(4) arylalkylene in which the aryl part is phenyl optionally substituted with one or more of (i) alkyl of from one to four carbon atoms, (ii) halo, (iii) trifluoromethyl, (iv) nitro, (v) amino, (vi) mono- or di-alkylamino in which the or each alkyl is independently a lower alkyl of from one to six carbon atoms or, in the case of a dialkylamino, in which the two alkyls, when taken together, represent an alkylene of from one to six carbon atoms; (vii) cyano, (viii) lower alkoxy of from one to four carbon atoms, and (ix) lower thioalkyl of from one to four carbon atoms; and the alkylene part has from one to four carbon atoms; or

(5) thienyl, furanyl, pyryl, pyridinyl, quinolyl, isoquinolyl, pyrimidyl, pyridazinyl, quinazolyl, quinoxalyl, benzothienyl, pyrazolyl, imidazolyl, oxazolyl, isoxazolyl, thiazolyl, triazolyl, pyrazinyl, oxazinyl, thiazinyl, indolizinyl, indolyl, benzofuranyl, indazolyl, benzothienyl, benzimidazolyl, benzoxazolyl, benzioxazolyl, benzthiazolyl, benztriazolyl, benzoxadiazolyl, cinnolinyl, phthalaxinyl, naphthyridinyl, or benzothiazinyl;

and wherein $R_2$ and $R_5$ may also be taken together to form a five- or six-membered ring having, as a member of the ring, a

O O<br>
|| ||<br>
—C— or — C—O— group;

n is one or two; m is an integer of from one to six.

2. A compound of the formula (II)

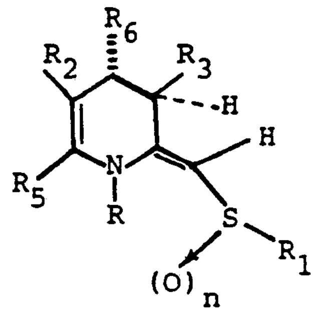

(II)

and a pharmaceutically acceptable salt thereof; wherein

R is

(1) hydrogen;

(2) lower alkyl of from one to four carbon atoms, optionally substituted with an amino group having the formula $NR_7R_8$ in which $R_7$ and $R_8$ are independently hydrogen or lower alkyl of from one to four carbons, or $R_7$ and $R_8$, when taken together with the nitrogen atom to which they are attached form a five- or six-membered ring having four or five $CH_2$ groups; or

(3) an arylalkylene radical in which the aryl part is phenyl optionally substituted with one or more of (i) halo, (ii) trifluoromethyl, (iii) nitro, (iv) amino, (iv) mono- or di-alkylamino in which the or each alkyl is independently a lower alkyl of from one to six carbon atoms or, in the case of a dialkylamino, in which the two alkyls, when taken together represent an alkylene of from one to six carbon atoms, (vi) cyano, (vii) lower alkoxy of from one to four carbon atoms, (viii) lower alkyl of from one to four carbon atoms, (ix) lower thioalkyl of from one to four carbon atoms; and the alkylene part has from one to four carbon atoms;

$R_2$ and $R_3$, which may be the same or different, are each hydrogen, lower alkyl of from one to four carbon atoms, cyano, nitro, $—CO_2R_4$ in which $R_4$ is hydrogen or lower alkyl of from one to four carbon atoms, optionally substituted with amino, mono-, or di- alkylamino in which the or each alkyl is independently a lower alkyl of from one to six carbon atoms or, in the case of a dialkylamino, in which the two alkyls, when taken together, represent an alkylene of from one to six carbon atoms;

$R_1$, $R_5$, and $R_6$, which may be the same or different, are each

(1) lower alkyl of from one to four carbon atoms, optionally substituted with (a) an amino group having the formula $NR_7R_8$ in which $R_7$ and $R_8$ are independently hydrogen or lower alkyl, or $R_7$ and $R_8$, when taken together with the nitrogen atom to which they are attached form a five- or six-membered ring having four or five carbon atoms, (b) $—OR_9$ in which $R_9$ is hydrogen or lower alkyl of from one to four carbon atoms, or (c) $—CO_2R_4$ in which $R_4$ is as defined above;

(2) cycloalkyl of from four to seven carbon atoms;

(3) phenyl optionally substituted with one or more of (i) alkyl having from one to four carbon atoms, (ii) halo, (iii) trifluoromethyl, (iv) nitro, (v) amino, (vi) mono- or di- alkylamino in which the or each alkyl is independently a lower alkyl of from one to six carbon atoms or, in the case of a dialkylamino, in which the two alkyls, when taken together, represent an alkylene of from one to six carbon atoms; (vii) cyano, (viii) lower alkoxy of from one to four carbon atoms, and (ix) lower thioalkyl of from one to four carbon atoms;

(4) arylalkylene in which the aryl part is phenyl optionally substituted with one or more of (i) alkyl of from one to four carbon atoms, (ii) halo, (iii) trifluoromethyl, (iv) nitro, (v) amino, (vi) mono- or di-alkylamino in which the or each alkyl is independently a lower alkyl of from one to six carbon atoms or, in the case of a dialkylamino, in which the two alkyls, when taken together, represent an alkylene of from one to six carbon atoms; (vii) cyano, (viii) lower alkoxy of from one to four carbon atoms, (xi) lower thioalkyl of from one to four carbon atoms; and the alkylene part has from one to four carbon atoms; or

(5) thienyl, furanyl, pyryl, pyridinyl, quinolyl, isoquinolyl, pyrimidyl, pyridazinyl, quinazolyl, quinoxalyl, benzothienyl, pyrazolyl, imidazolyl, oxazolyl, isoxazolyl, thiazolyl, triazolyl, pyrazinyl, oxazinyl, thiazinyl, indolizinyl, indolyl, benzofuranyl, indazolyl, benzothienyl, benzimidazolyl, benzoxazolyl, benzioxazolyl, benzthiazolyl, benztriazolyl, benzoxadiazolyl, cinnolinyl, phthalaxinyl, naphthyridinyl, or benzothiazinyl;

and wherein $R_2$ and $R_5$ may also be taken together to form a five- or six-membered ring having, as a member of the ring, a

O O<br>
|| ||<br>
—C— or — C—O— group;

n is zero or an integer of one or two.

3. A compound according to Claim 2, wherein $R_2$ is cyano and n is one or two.

4. A compound according to Claim 1 or 2, and being of the formula

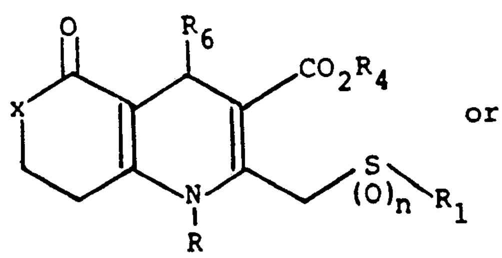

or

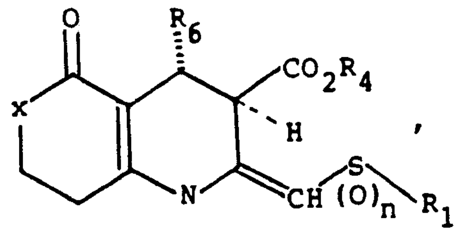

,

wherein X is $>CH_2$ or an oxygen atom.

5. A compound according to Claim 1 or 2, wherein R is hydrogen, $R_2$ is cyano, $R_3$ is $—CO_2R_4$, $R_5$ is methyl and m is one.

6. A compound according to Claim 2, wherein R is hydrogen, $R_2$ and $R_3$ are independently $—CO_2R_4$, $R_5$ is methyl and m is one.

7. A compound according to Claim 4, 5 or 6, when appendent to Claim 2 wherein n is zero.

8. A compound according to Claim 1 or 2 having one of the following names:

3-pyridinecarboxylic acid, 5-cyano-1,2,3,4-tetrahydro-2-[(phenylthio)methylene]-6-methyl-4-[(2-trifluoromethyl)phenyl]-, ethyl ester;

3-quinolinecarboxylic acid, 1,2,3,4,5,6,7,8-octahydro-5-oxo-2-[(phenylthio)methylene]-4-[2-(trifluoromethyl)phenyl]-, ethyl ester;

ethyl 5-cyano-1,4-dihydro-6-methyl-2-[(phenylsulfinyl)methyl]-4-[2-(trifluoromethyl)phenyl]-3-pyridinecarboxylate;

ethyl 5-cyano-1,4-dihydro-6-methyl-2-[[(2-methylphenyl)sulfinyl]methyl]-4-[2-(trifluoromethyl)phenyl]-3-pyridinecarboxylate;

ethyl 5-cyano-1,4-dihydro-6-methyl-4-(3-nitrophenyl)-2-[(phenylsulfinyl)methyl]-3-pyridinecarboxylate;

ethyl 5-cyano-1,4-dihydro-6-methyl-2-[[(4-methoxyphenyl)sulfinyl]methyl]-4-[2-(trifluoromethyl)-phenyl]-3-pyridinecarboxylate;

ethyl 5-cyano-1,4-dihydro-6-methyl-2-[(phenylsulfinyl)methyl]-4-[2-(chlorophenyl)-3-pyridinecarboxylate;

ethyl 5-cyano-1,4-dihydro-6-methyl-2-[(4-pyridinylsulfinyl)methyl]-4-[2-(trifluoromethyl)phenyl]-3-pyridinecarboxylate;

is ethyl 2-[[(2-chlorophenyl)sulfinyl]methyl]-5-cyano-1,4-dihydro-6-methyl-4-[(2-trifluoromethyl)phenyl]-3-pyridinecarboxylate;

5-cyano-2-[[[2-(dimethylamino)ethyl]sulfinyl]methyl]-1,4-dihydro-6-methyl-4-[2-(trifluoromethyl)-phenyl]-3-pyridinecarboxylic acid, ethyl ester;

ethyl 4-(2-chlorophenyl)-5-cyano-1,4-dihydro-6-methyl-2-[(4-pyridinylsulfinyl)methyl]-3-pyridinecarboxylate;

ethyl 5-cyano-4-(2,3-dichlorophenyl)-1,4-dihydro-6-methyl-2-[4-pyridinylsulfinyl)methyl-3-pyridinecarboxylate;

5-cyano-1,4-dihydro-6-methyl-2-[(2-pyrimidinylsulfinyl)methyl]-4-[2-(trifluoromethyl)phenyl]-3-pyridinecarboxylic acid, ethyl ester;

5-cyano-1,4-dihydro-6-methyl-2-[[(1-methyl-1*H*-imidazol-2-yl)sulfinyl]methyl]-4-[2-(trifluoromethyl)-phenyl]-3-pyridinecarboxylic acid, ethyl ester;

ethyl 5-cyano-1,4-dihydro-6-methyl-2-[(phenylsulfinyl)methyl]-4-phenyl-3-pyridinecarboxylate;

3,5-pyridinedicarboxylic acid, 2-[[(2-chlorophenyl)sulfinyl]methylene]-6-methyl-1,2,3,4-tetrahydro-4-[(2-trifluoromethyl)phenyl]-3-ethyl-5-methyl ester;

3-pyridinecarboxylic acid, 5-cyano-4-cyclohexyl-1,4-dihydro-6-methyl-2-[(phenylsulfinyl)methyl]ethyl ester (±)-;

3-pyridinecarboxylic acid, 5-cyano-4-[2-(difluoromethoxy)phenyl]-1,4-dihydro-6-methyl-2-[(phenylsulfinyl)methyl]-, ethyl ester;

3-pyridinecarboxylic acid, 5-cyano-1,4-dihydro-6-methyl-2-[(phenylsulfinyl)methyl]-4-[2-(trifluoromethyl)phenyl]-, methyl ester; (53.47 mixture of isomers);

3-pyridinecarboxylic acid, 5-cyano-1,4-dihydro-6-methyl-2-[(phenylsulfinyl)methyl]-4-[2-(trifluoromethyl)phenyl]-, 1-methylethyl ester;

3-pyridinecarboxylic acid, 5-cyano-1,4-dihydro-6-methyl-2-[(phenylsulfinyl)methyl]-4-[2-(trifluoromethyl)phenyl]-, 2-hydroxyethyl ester;

3-pyridinecarboxylic acid, 5-cyano-1,4-dihydro-6-methyl-4-(2-phenyl-1*H*-imidazol-4-yl)-2-[(phenylsulfinyl)methyl]-, ethyl ester;

3-pyridinecarboxylic acid, 5-cyano-1,4-dihydro-6-methyl-2-[(2-pyridinylsulfinyl)methyl]-4-[2-(trifluoromethyl)phenyl]-, ethyl ester, (±)-;

3-pyridinecarboxylic acid, 5-cyano-2-[[(2-fluorophenyl)sulfinyl]methyl]-1,4-dihydro-6-methyl-4-[2-(trifluoromethyl)phenyl]-, ethyl ester;

3-pyridinecarboxylic acid, 2-[[[4-(acetylamino)phenyl]sulfinyl]methyl]-5-cyano-1,4-dihydro-6-methyl-4-[2-(trifluoromethyl)phenyl]-, ethyl ester;

3-pyridinecarboxylic acid, 2-[[(4-chlorophenyl)sulfinyl]methyl]-5-cyano-1,4-dihydro-6-methyl-4-[2-(trifluoromethyl)phenyl]-, ethyl ester, (±)-;

3-pyridinecarboxylic acid, 5-cyano-2-[[(3,4-dichlorophenyl)sulfinyl]methyl]-1,4-dihydro-6-methyl-4-[2-(trifluoromethyl)phenyl]-, ethyl ester;

3-pyridinecarboxylic acid, 5-cyano-1,4-dihydro-6-methyl-2-[(lphenylsulfinyl)methyl]-4-[2-(trifluoromethyl)phenyl]-;

3-quinolinecarboxylic acid, 1,4,5,6,7,8-hexahydro-1-methyl-5-oxo-2-[(phenylsulfinyl)methyl]-4-[2-(trifluoromethyl)phenyl]-, ethyl ester;

3-quinolinecarboxylic acid, 1,4,5,6,7,8-hexahydro-5-oxo-2-[(phenylsulfinyl)methyl]-4-[2-(trifluoromethyl)phenyl]-, ethyl ester, (±)-;

ethyl 3-cyano-1,4-dihydro-6-methyl-2-[(phenylsulfinyl)methyl]-4-[2-(trifluoromethyl)phenyl]-5-pyridinecarboxylate;

3-pyridinecarboxylic acid, 5-cyano-1-[2-(diethylamino)ethyl]-1,4-dihydro-6-methyl-2-[(phenylsulfinyl)methyl]-4-[2-(trifluoromethyl)phenyl]-, ethyl ester;

3-pyridinecarboxylic acid, 5-cyano-1,4-dihydro-2-[[(3-methoxy-3-oxopropyl]sulfinyl]methyl]-6-methyl-4-[2-(trifluoromethyl)phenyl]-, ethyl ester;

3-pyridinecarboxylic acid, 5-cyano-2-[[(2-ethoxy-2-oxoethyl)sulfinyl]methyl]-1,4-dihydro-6-methyl-4-[2-(trifluoromethyl)phenyl]-, ethyl ester;

[2,4'-bipyridine]-3'-carboxylic acid, 5'-cyano-1',4'-dihydro-6'-methyl-2'-[(phenylsulfinyl)methyl]-, ethyl ester;

ethyl 5-cyano-1,4-dihydro-6-methyl-2-[(phenylsulfonyl)methyl]-4-[2-(trifluoromethyl)phenyl]-3-pyridinecarboxylate;

ethyl 5-cyano-1,4-dihydro-6-methyl-2-[[(4-methoxyphenyl)sulfonyl]methyl]-4-[2-(trifluoromethyl)phenyl]-3-pyridinecarboxylate;

ethyl 5-cyano-1,4-dihydro-6-methyl-2-[[(2-methylphenyl)sulfonyl]methyl]-4-[2-(trifluoromethyl)phenyl]-3-pyridinecarboxylate;

ethyl 2-[[(2-chlorophenyl)sulfonyl]methyl]-5-cyano-1,4-dihydro-6-methyl-4-[(2-trifluoromethyl)phenyl]-3-pyridinecarboxylate;

ethyl 5-cyano-1,4-dihydro-6-methyl-2-[(4-pyridinylsulfonyl)methyl]-4-[2-(trifluoromethyl)phenyl]-3-pyridinecarboxylate;

5-cyano-1,4-dihydro-6-methyl-2-[(2-pyridinylsulfonyl)methyl]-4-[2-(trifluoromethyl)phenyl]-3-pyridinecarboxylic acid, ethyl ester, N-oxide;

ethyl 5-cyano-1,4-dihydro-6-methyl-2-[(phenylsulfonyl)methyl]-4-phenyl-3-pyridinecarboxylate;

[2,4'-bipyridine]-3'-carboxylic acid, 5'-cyano-1',4'-dihydro-6'-methyl-2'-[(phenylsulfonyl)methyl]-, ethyl ester, 1-oxide;

[3,4'-bipyridine]-3'-carboxylic acid, 5'-cyano-1',4'-dihydro-6'-methyl-2'-[(phenylsulfonyl)methyl]-, ethyl ester;

3-pyridinecarboxylic acid, 2-[[(4-aminophenyl)sulfonyl]methyl]-5-cyano-1,4-dihydro-6-methyl-4-[2-(trifluoromethyl)phenyl]-, ethyl ester;

[2,4'-bipyridine]-3'-carboxylic acid, 5'-cyano-1',4'-dihydro-6'-methyl-2'-[(phenylsulfonyl)methyl]-, ethyl ester;

3-pyridinecarboxylic acid, 5-cyano-4-cyclohexyl-6-methyl-1,4-dihydro-2-[(phenylsulfonyl)methyl]-, ethyl ester;

3-pyridinecarboxylic acid, 5-cyano-1,4-dihydro-6-methyl-2-[(phenylsulfonyl)methyl]-4-[2-(trifluoromethyl)phenyl]-, 1-methylethyl ester;

3-pyridinecarboxylic acid, 5-cyano-1,4-dihydro-6-methyl-2-[(phenylsulfonyl)methyl]-4-[2-(trifluoromethyl)phenyl]-, 2-propenyl ester;

3-pyridinecarboxylic acid, 5-cyano-2-[[[4-(1,1-dimethylethyl)-2-methylphenyl]sulfonyl]methyl]-1,4-dihydro-6-methyl-4-[2-(trifluoromethyl)phenyl]-, ethyl ester;

3-pyridinecarboxylic acid, 5-cyano-2-[[(2-fluorophenyl)sulfonyl]methyl]-1,4-dihydro-6-methyl-4-[2-(trifluoromethyl)phenyl]-, ethyl ester;

3-pyridinecarboxylic acid, 2-[[(4-chlorophenyl)sulfonyl]methyl]-5-cyano-1,4-dihydro-6-methyl-4-[2-(trifluoromethyl)phenyl]-, ethyl ester;

3-pyridinecarboxylic acid, 5-cyano-1,4-dihydro-6-methyl-4-(3-nitrophenyl)-2-[(phenylsulfonyl)methyl]-, ethyl ester;

3-pyridinecarboxylic acid, 5-cyano-1,4-dihydro-6-methyl-2-[(phenylsulfonyl)methyl]-4-[2-(trifluoromethyl)phenyl]-, 2,3-dihydroxypropyl ester;

3-pyridinecarboxylic acid, 5-cyano-1,4-dihydro-2-[[(3-methoxy-3-oxopropyl)sulfonyl]methyl]-6-methyl-4-[2(trifluoromethyl)phenyl]-, ethyl ester;

3-quinolinecarboxylic acid, 1,4,5,6,7,8-hexahydro-1-methyl-5-oxo-2-[(phenylsulfonyl)methyl]-4-[2-(trifluoromethyl)phenyl]-, ethyl ester;

3-quinolinecarboxylic acid, 1,4,5,6,7,8-hexahydro-5-oxo-2-[(phenylsulfonyl)methyl]-4-[2-(trifluoromethyl)phenyl]-, ethyl ester; and

3-quinolinecarboxylic acid, 1,4,5,6,7,8-hexahydro-1-methyl-5-oxo-2-[(phenylthio)methyl]-4-[2-(trifluoromethyl)phenyl]-, ethyl ester.

9. A pharmaceutical composition comprising an effective amount of a compound as claimed in any preceding claim in admixture with a pharmaceutically acceptable carrier or diluent.

10. For use in a method of treating a mammal suffering from a cardiovascular disorder, a compound as claimed in any one of Claims 1 to 8 or a pharmaceutical composition as claimed in Claim 9.

11. A process for preparing a compound of the formula (I) or (II) as defined in Claims 1 and 2 respectively, which process comprises:

contacting the compounds

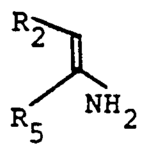

(III),

$R_6CHO$ (IV) and

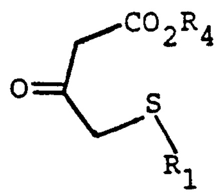

(V),

wherein $R_1$, $R_2$, $R_4$, $R_5$ and $R_6$ are as defined in Claims 1 or 2 in an organic solvent to form a compound of formula ($I_o$) or ($II_o$)

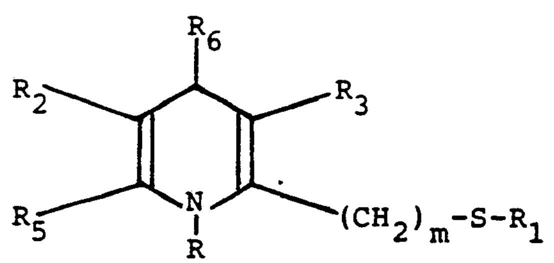

($I_o$)

or

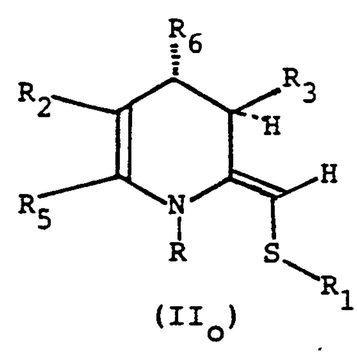

($II_o$)

and, if desired, further reacting the product in one or more of the following steps:

(1) oxygenating the sulphur atom

(2) alkylating the nitrogen atom; and

(3) reacting the $—CO_2R_4$ group to obtain an $R_3$ group other than $—CO_2R_4$;

and optionally forming a pharmaceutically acceptable salt thereof.

**Claims for the Contracting State: AT**

1. A process for preparing a compound having the following general formula (I)

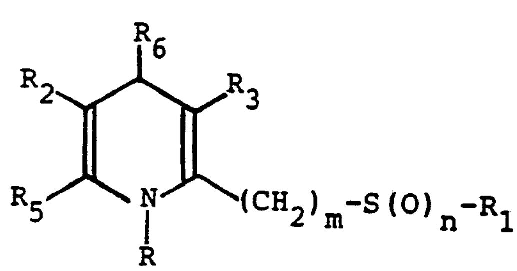

(I)

and a pharmaceutically acceptable salt thereof; wherein

R is

(1) hydrogen;

(2) lower alkyl of from one to four carbon atoms, optionally substituted with an amino group having the formula $NR_7R_8$ in which $R_7$ and $R_8$ are independently hydrogen or lower alkyl of from one to four carbon atoms, or $R_7$ and $R_8$, when taken together with the nitrogen atom to which they are attached form a five- or six-membered ring having four or five $CH_2$ groups; or

(3) an arylalkylene radical in which the aryl part is phenyl optionally substituted with one or more of (i) halo, (ii) trifluoromethyl, (iii) nitro, (iv) amino; (v) mono- or di-alkylamino in which the or each alkyl is independently a lower alkyl of from one to six carbon atoms or, in the case of a dialkylamino, in which the two alkyls, when taken together represent an alkylene of from one to six carbon atoms, (vi) cyano, (vii) lower alkoxy of from one to four carbon atoms, (viii) lower thioalkyl of from one to four carbon atoms; and in which the alkylene part has from one to four carbon atoms;

$R_2$ is cyano;

$R_3$ is hydrogen, lower alkyl of from one to four carbon atoms, cyano, nitro, $—CO_2R_4$ in which $R_4$ is hydrogen or lower alkyl of from one to four carbon atoms, optionally substituted with amino, mono-, or dialkylamino in which the or each alkyl is independently a lower alkyl of from one to six carbon atoms or, in the case of a dialkylamino, in which the two alkyls, when taken together, represent an alkylene of from one to six carbon atoms;

$R_1$ is

(1) cycloalkyl of from four to seven carbon atoms;

(2) phenyl optionally substituted with one or more of (i) alkyl having from one to four carbon atoms, (ii) halo, (iii) trifluoromethyl, (iv) nitro, (v) amino, and (vi) mono- or di- alkylamino in which the or each alkyl is independently a lower alkyl of from one to six carbon atoms or, in the case of a dialkylamino, in which the two alkyls, when taken together, represent an alkylene of from one to six carbon atoms; or

(3) thienyl, furanyl, pyryl, pyridinyl, quinolyl, isoquinolyl, pyrimidyl, pyridazinyl, quinazolyl, quinoxalyl, benzothienyl, pyrazolyl, imidazolyl, oxazolyl, isoxazolyl, thiazolyl, triazolyl, pyrazinyl, oxazinyl, thiazinyl, indolizinyl, indolyl, benzofuranyl, indazolyl, benzothienyl, benzimidazolyl, benzoxazolyl, benzioxazolyl, benzthiazolyl, benztriazolyl, benzoxadiazolyl, cinnolinyl, phthalaxinyl, naphthyridinyl, or benzothiazinyl;

$R_5$, and $R_6$, which may be the same or different, are each

(1) lower alkyl of from one to four carbon atoms, optionally substituted with (a) an amino group having the formula $NR_7R_8$ in which $R_7$ and $R_8$ are independently hydrogen or lower alkyl, or $R_7$ and $R_8$, when taken together with the nitrogen atom to which they are attached form a five- or six-membered ring having four or five carbon atoms, (b) $—OR_9$ in which $R_9$ is hydrogen or lower alkyl of from one to four carbon atoms, or (c) $—CO_2R_4$ in which $R_4$ is as defined above;

(2) cycloalkyl of from four to seven carbon atoms;

(3) phenyl optionally substituted with one or more of (i) alkyl having from one to four carbon atoms, (ii) halo, (iii) trifluoromethyl, (iv) nitro, (v) amino, (vi) mono- or di- alkylamino in which the or each alkyl is independently a lower alkyl of from one to six carbon atoms or, in the case of a dialkylamino, in which the two alkyls, when taken together, represent an alkylene of from one to six carbon atoms; (vii) cyano, (viii) lower alkoxy of from one to four carbon atoms, and (ix) lower thioalkyl of from one to four carbon atoms;

(4) arylalkylene in which the aryl part is phenyl optionally substituted with one or more of (i) alkyl of from one to four carbon atoms, (ii) halo, (iii) trifluoromethyl, (iv) nitro, (v) amino, (vi) mono- or di-alkylamino in which the or each alkyl is independently a lower alkyl of from one to six carbon atoms or, in the case of a dialkylamino, in which the two alkyls, when taken together, represent an alkylene of from one to six carbon atoms; (vii) cyano, (viii) lower alkoxy of from one to four carbon atoms, and (ix) lower thioalkyl of from one to four carbon atoms; and the alkylene part has from one to four carbon atoms; or

(5) thienyl, furanyl, pyryl, pyridinyl, quinolyl, isoquinolyl, pyrimidyl, pyridazinyl, quinazolyl, quinoxalyl, benzothienyl, pyrazolyl, imidazolyl, oxazolyl, isoxazolyl, thiazolyl, triazolyl, pyrazinyl, oxazinyl, thiazinyl, indolizinyl, indolyl, benzofuranyl, indazolyl, benzothienyl, benzimidazolyl, benzoxazolyl, benzioxazolyl, benzthiazolyl, benztriazolyl, benzoxadiazolyl, cinnolinyl, phthalaxinyl, naphthyridinyl, or benzothiazinyl;

and wherein $R_2$ and $R_5$ may also be taken together to form a five- or six-membered ring having, as a member of the ring, a

$$-\overset{\overset{O}{\|}}{C}- \text{ or } -\overset{\overset{O}{\|}}{C}-O- \text{ group;}$$

n is one or two; m is an integer of from one to six;
which process comprises:
contacting the compounds

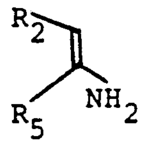

(III),

$R_6CHO$ (IV) and

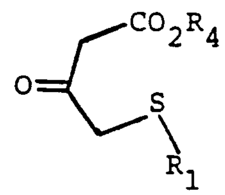

(V),

wherein $R_1$, $R_2$, $R_4$, $R_5$ and $R_6$ are as defined above in an organic solvent to form a compound of formula ($I_o$)

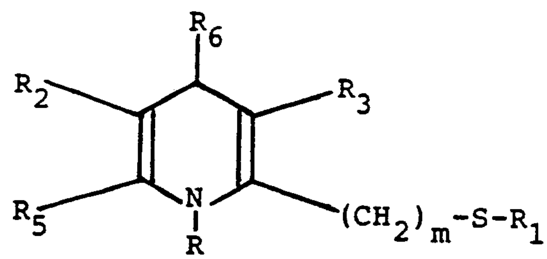

($I_o$)

and, if desired, further reacting the product in one or more of the following steps:
(1) oxygenating the sulphur atom
(2) alkylating the nitrogen atom; and
(3) reacting the $—CO_2R_4$ group to obtain an $R_3$ group other than $—CO_2R_4$;
and optionally forming a pharmaceutically acceptable salt thereof.

2. A process for preparing a compound of the formula (II)

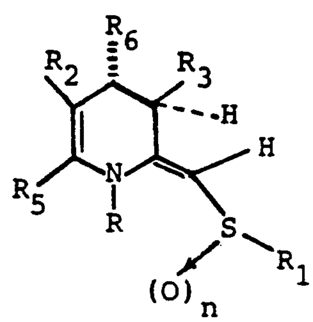

(II)

and a pharmaceutically acceptable salt thereof; wherein

R is

(1) hydrogen;

(2) lower alkyl of from one to four carbon atoms, optionally substituted with an amino group having the formula $NR_7R_8$ in which $R_7$ and $R_8$ are independently hydrogen or lower alkyl of from one to four carbons, or $R_7$ and $R_8$, when taken together with the nitrogen atom to which they are attached form a five- or six-membered ring having four or five $CH_2$ groups; or

(3) an arylalkylene radical in which the aryl part is phenyl optionally substituted with one or more of (i) halo, (ii) trifluoromethyl, (iii) nitro, (iv) amino, (iv) mono- or di-alkylamino in which the or each alkyl is independently a lower alkyl of from one to six carbon atoms or, in the case of a dialkylamino, in which the two alkyls, when taken together represent an alkylene of from one to six carbon atoms, (vi) cyano, (vii) lower alkoxy of from one to four carbon atoms, (viii) lower alkyl of from one to four carbon atoms, (ix) lower

thioalkyl of from one to four carbon atoms; and the alkylene part has from one to four carbon atoms;

$R_2$ and $R_3$, which may be the same or different, are each hydrogen, lower alkyl of from one to four carbon atoms, cyano, nitro, $—CO_2R_4$ in which $R_4$ is hydrogen or lower alkyl of from one to four carbon atoms, optionally substituted with amino, mono-, or di- alkylamino in which the or each alkyl is independently a lower alkyl of from one to six carbon atoms or, in the case of a dialkylamino, in which the two alkyls, when taken together, represent an alkylene of from one to six carbon atoms;

$R_1$, $R_5$, and $R_6$, which may be the same or different, are each

(1) lower alkyl of from one to four carbon atoms, optionally substituted with (a) an amino group having the formula $NR_7R_8$ in which $R_7$ and $R_8$ are independently hydrogen or lower alkyl, or $R_7$ and $R_8$, when taken together with the nitrogen atom to which they are attached form a five- or six-membered ring having four or five carbon atoms, (b) $—OR_9$ in which $R_9$ is hydrogen or lower alkyl of from one to four carbon atoms, or (c) $—CO_2R_4$ in which $R_4$ is as defined above;

(2) cycloalkyl of from four to seven carbon atoms;

(3) phenyl optionally substituted with one or more of (i) alkyl having from one to four carbon atoms, (ii) halo, (iii) trifluoromethyl, (iv) nitro, (v) amino, (vi) mono- or di- alkylamino in which the or each alkyl is independently a lower alkyl of from one to six carbon atoms or, in the case of a dialkylamino, in which the two alkyls, when taken together, represent an alkylene of from one to six carbon atoms; (vii) cyano, (viii) lower alkoxy of from one to four carbon atoms, and (ix) lower thioalkyl of from one to four carbon atoms;

(4) arylalkylene in which the aryl part is phenyl optionally substituted with one or more of (i) alkyl of from one to four carbon atoms, (ii) halo, (iii) trifluoromethyl, (iv) nitro, (v) amino, (vi) mono- or di- alkylamino in which the or each alkyl is independently a lower alkyl of from one to six carbon atoms or, in the case of a dialkylamino, in which the two alkyls, when taken together, represent an alkylene of from one to six carbon atoms; (vii) cyano, (viii) lower alkoxy of from one to four carbon atoms, (xi) lower thioalkyl of from one to four carbon atoms; and the alkylene part has from one to four carbon atoms; or

(5) thienyl, furanyl, pyryl, pyridinyl, quinolyl, isoquinolyl, pyrimidyl, pyridazinyl, quinazolyl, quinoxalyl, benzothienyl, pyrazolyl, imidazolyl, oxazolyl, isoxazolyl, thiazolyl, triazolyl, pyrazinyl, oxazinyl, thiazinyl, indolizinyl, indolyl, benzofuranyl, indazolyl, benzothienyl, benzimidazolyl, benzoxazolyl, benzioxazolyl, benzthiazolyl, benztriazolyl, benzoxadiazolyl, cinnolinyl, phthalaxinyl, naphthyridinyl, or benzothiazinyl;

and wherein $R_2$ and $R_5$ may also be taken together to form a five- or six-membered ring having, as a member of the ring, a

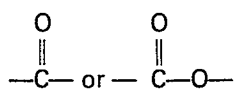 group;

n is zero or an integer of one or two;

which process comprises:

contacting the compounds

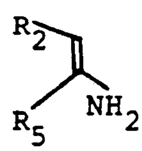 (III),

$R_6CHO$ (IV) and

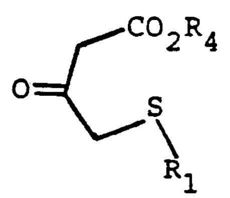 (V),

wherein $R_1$, $R_2$, $R_4$, $R_5$ and $R_6$ are as defined above in an organic solvent to form a compound of formula ($I_o$)

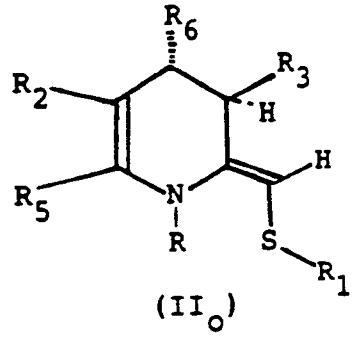

($II_o$)

and, if desired, further reacting the product in one or more of the following steps:
(1) oxygenating the sulphur atom
(2) alkylating the nitrogen atom; and
(3) reacting the $—CO_2R_4$ group to obtain an $R_3$ group other than $—CO_2R_4$;
and optionally forming a pharmaceutically acceptable salt thereof.

3. A process according to Claim 2, wherein $R_2$ is cyano and n is one or two.

4. A process according to Claim 1 or 2, for preparing a compound of the formula:

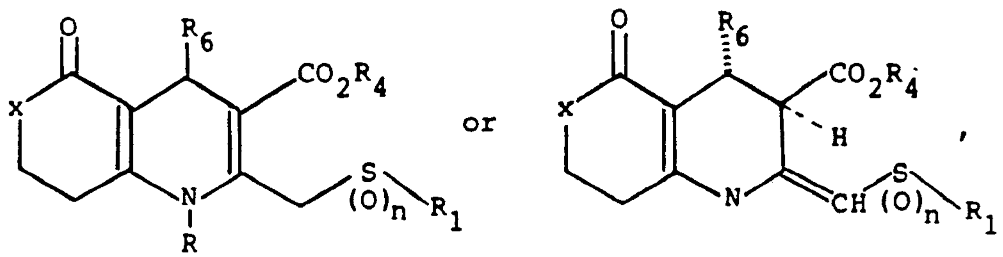

wherein X is >$CH_2$ or an oxygen atom.

5. A process according to Claim 1 or 2, wherein R is hydrogen, $R_2$ is cyano, $R_3$ is $—CO_2R_4$, $R_5$ is methyl and m is one.

6. A process according to Claim 2, wherein R is hydrogen, $R_2$ and $R_3$ are independently $—CO_2R_4$, $R_5$ is methyl and m is one.

7. A process according to Claim 4, 5 or 6, when appendant to Claim 2 wherein n is zero.

8. A process according to Claim 1 or 2 for preparing a compound having one of the following names:

3-pyridinecarboxylic acid, 5-cyano-1,2,3,4-tetrahydro-2-[(phenylthio)methylene]-6-methyl-4-[(2-trifluoromethyl)phenyl]-, ethyl ester;

3-quinolinecarboxylic acid, 1,2,3,4,5,6,7,8-octahydro-5-oxo-2-[(phenylthio)methylene]-4-[2-(trifluoromethyl)phenyl]-, ethyl ester;

ethyl 5-cyano-1,4-dihydro-6-methyl-2-[(phenylsulfinyl)methyl]-4-[2-(trifluoromethyl)phenyl]-3-pyridinecarboxylate;

ethyl 5-cyano-1,4-dihydro-6-methyl-2-[[(2-methylphenyl)sulfinyl]methyl]-4-[2-(trifluoromethyl)phenyl]-3-pyridinecarboxylate;

ethyl 5-cyano-1,4-dihydro-6-methyl-4-(3-nitrophenyl)-2-[(phenylsulfinyl)methyl]-3-pyridinecarboxylate;

ethyl 5-cyano-1,4-dihydro-6-methyl-2-[[(4-methoxyphenyl)sulfinyl]methyl]-4-[2-(trifluoromethyl)-phenyl]-3-pyridinecarboxylate;

ethyl 5-cyano-1,4-dihydro-6-methyl-2-[(phenylsulfinyl)methyl]-4-[2-(chlorophenyl)-3-pyridinecarboxylate;

ethyl 5-cyano-1,4-dihydro-6-methyl-2-[(4-pyridinylsulfinyl)methyl]-4-[2-(trifluoromethyl)phenyl]-3-pyridinecarboxylate;

is ethyl 2-[[(2-chlorophenyl)sulfinyl]methyl]-5-cyano-1,4-dihydro-6-methyl-4-[(2-trifluoromethyl)phenyl]-3-pyridinecarboxylate;

5-cyano-2-[[[2-(dimethylamino)ethyl]sulfinyl]methyl]-1,4-dihydro-6-methyl-4-[2-(trifluoromethyl)-phenyl]-3-pyridinecarboxylic acid, ethyl ester;

ethyl 4-(2-chlorophenyl)-5-cyano-1,4-dihydro-6-methyl-2-[(4-pyridinylsulfinyl)methyl]-3-pyridinecarboxylate;

ethyl 5-cyano-4-(2,3-dichlorophenyl)-1,4-dihydro-6-methyl-2-[4-pyridinylsulfinyl)methyl-3-pyridinecarboxylate;

5-cyano-1,4-dihydro-6-methyl-2-[(2-pyrimidinylsulfinyl)methyl]-4-[2-(trifluoromethyl)phenyl]-3-pyridinecarboxylic acid, ethyl ester;

5-cyano-1,4-dihydro-6-methyl-2-[[(1-methyl-1*H*-imidazol-2-yl)sulfinyl]methyl]-4-[2-(trifluoromethyl)-phenyl]-3-pyridinecarboxylic acid, ethyl ester;

ethyl 5-cyano-1,4-dihydro-6-methyl-2-[(phenylsulfinyl)methyl]-4-phenyl-3-pyridinecarboxylate;

3,5-pyridinedicarboxylic acid, 2-[[(2-chlorophenyl)sulfinyl]methylene]-6-methyl-1,2,3,4-tetrahydro-4-[(2-trifluoromethyl)phenyl]-3-ethyl-5-methyl ester;

3-pyridinecarboxylic acid, 5-cyano-4-cyclohexyl-1,4-dihydro-6-methyl-2-[(phenylsulfinyl)methyl]ethyl ester (±)-;

3-pyridinecarboxylic acid, 5-cyano-4-[2-(difluoromethoxy)phenyl]-1,4-dihydro-6-methyl-2-[(phenylsulfinyl)methyl]-, ethyl ester;

3-pyridinecarboxylic acid, 5-cyano-1,4-dihydro-6-methyl-2-[(phenylsulfinyl)methyl]-4-[2-(trifluoromethyl)phenyl]-, methyl ester; (53.47 mixture of isomers);

3-pyridinecarboxylic acid, 5-cyano-1,4-dihydro-6-methyl-2-[(phenylsulfinyl)methyl]-4-[2-(trifluoromethyl)phenyl]-, 1-methylethyl ester;

3-pyridinecarboxylic acid, 5-cyano-1,4-dihydro-6-methyl-2-[(phenylsulfinyl)methyl]-4-[2-(trifluoromethyl)phenyl]-, 2-hydroxyethyl ester;

3-pyridinecarboxylic acid, 5-cyano-1,4-dihydro-6-methyl-4-(2-phenyl-1*H*-imidazol-4-yl)-2-[(phenylsulfinyl)methyl]-, ethyl ester;

3-pyridinecarboxylic acid, 5-cyano-1,4-dihydro-6-methyl-2-[(2-pyridinylsulfinyl)methyl]-4-[2-(trifluoromethyl)phenyl]-, ethyl ester, (±)-;

3-pyridinecarboxylic acid, 5-cyano-2-[[(2-fluorophenyl)sulfinyl]methyl]-1,4-dihydro-6-methyl-4-[2-(trifluoromethyl)phenyl]-, ethyl ester;

3-pyridinecarboxylic acid, 2-[[[4-(acetylamino)phenyl]sulfinyl]methyl]-5-cyano-1,4-dihydro-6-methyl-4-[2-(trifluoromethyl)phenyl]-, ethyl ester;

3-pyridinecarboxylic acid, 2-[[(4-chlorophenyl)sulfinyl]methyl]-5-cyano-1,4-dihydro-6-methyl-4-[2-(trifluoromethyl)phenyl]-, ethyl ester, (±)-;

3-pyridinecarboxylic acid, 5-cyano-2-[[(3,4-dichlorophenyl)sulfinyl]methyl]-1,4-dihydro-6-methyl-4-[2-(trifluoromethyl)phenyl]-, ethyl ester;

3-pyridinecarboxylic acid, 5-cyano-1,4-dihydro-6-methyl-2-[(phenylsulfinyl)methyl]-4-[2-(trifluoromethyl)phenyl]-;

3-quinolinecarboxylic acid, 1,4,5,6,7,8-hexahydro-1-methyl-5-oxo-2-[(phenylsulfinyl)methyl]-4-[2-(trifluoromethyl)phenyl]-, ethyl ester;

3-quinolinecarboxylic acid, 1,4,5,6,7,8-hexahydro-5-oxo-2-[(phenylsulfinyl)methyl]-4-[2-(trifluoromethyl)phenyl]-, ethyl ester, (±)-;

ethyl 3-cyano-1,4-dihydro-6-methyl-2-[(phenylsulfinyl)methyl]-4-[2-(trifluoromethyl)phenyl]-5-pyridinecarboxylate;

3-pyridinecarboxylic acid, 5-cyano-1-[2-(diethylamino)ethyl]-1,4-dihydro-6-methyl-2-[(phenylsulfinyl)methyl]-4-[2-(trifluoromethyl)phenyl]-, ethyl ester;

3-pyridinecarboxylic acid, 5-cyano-1,4-dihydro-2-[[(3-methoxy-3-oxopropyl]sulfinyl]methyl]-6-methyl-4-[2-(trifluoromethyl)phenyl]-, ethyl ester;

3-pyridinecarboxylic acid, 5-cyano-2-[[(2-ethoxy-2-oxoethyl)sulfinyl]methyl]-1,4-dihydro-6-methyl-4-[2-(trifluoromethyl)phenyl]-, ethyl ester;

[2,4′-bipyridine]-3′-carboxylic acid, 5′-cyano-1′,4′-dihydro-6′-methyl-2′-[(phenylsulfinyl)methyl]-, ethyl ester;

ethyl 5-cyano-1,4-dihydro-6-methyl-2-[(phenylsulfonyl)methyl]-4-[2-(trifluoromethyl)phenyl]-3-pyridinecarboxylate;

ethyl 5-cyano-1,4-dihydro-6-methyl-2-[[(4-methoxyphenyl)sulfonyl]methyl]-4-[2-(trifluoromethyl)phenyl]-3-pyridinecarboxylate;

ethyl 5-cyano-1,4-dihydro-6-methyl-2-[[(2-methylphenyl)sulfonyl]methyl]-4-[2-(trifluoromethyl)phenyl]-3-pyridinecarboxylate;

ethyl 2-[[(2-chlorophenyl)sulfonyl]methyl]-5-cyano-1,4-dihydro-6-methyl-4-[(2-trifluoromethyl)phenyl]-3-pyridinecarboxylate;

ethyl 5-cyano-1,4-dihydro-6-methyl-2-[(4-pyridinylsulfonyl)methyl]-4-[2-(trifluoromethyl)phenyl]-3-pyridinecarboxylate;

5-cyano-1,4-dihydro-6-methyl-2-[(2-pyridinylsulfonyl)methyl]-4-[2-(trifluoromethyl)phenyl]-3-pyridinecarboxylic acid, ethyl ester, N-oxide;

ethyl 5-cyano-1,4-dihydro-6-methyl-2-[(phenylsulfonyl)methyl]-4-phenyl-3-pyridinecarboxylate;

[2,4′-bipyridine]-3′-carboxylic acid, 5′-cyano-1′,4′-dihydro-6′-methyl-2′-[(phenylsulfonyl)methyl]-, ethyl ester, 1-oxide;

[3,4′-bipyridine]-3′-carboxylic acid, 5′-cyano-1′,4′-dihydro-6′-methyl-2′-[(phenylsulfonyl)methyl]-, ethyl ester;

3-pyridinecarboxylic acid, 2-[[(4-aminophenyl)sulfonyl]methyl]-5-cyano-1,4-dihydro-6-methyl-4-[2-(trifluoromethyl)phenyl]-, ethyl ester;

[2,4′-bipyridine]-3′-carboxylic acid, 5′-cyano-1′,4′-dihydro-6′-methyl-2′-[(phenylsulfonyl)methyl]-, ethyl ester;

3-pyridinecarboxylic acid, 5-cyano-4-cyclohexyl-6-methyl-1,4-dihydro-2-[(phenylsulfonyl)methyl]-, ethyl ester;

3-pyridinecarboxylic acid, 5-cyano-1,4-dihydro-6-methyl-2-[(phenylsulfonyl)methyl]-4-[2-(trifluoromethyl)phenyl]-, 1-methylethyl ester;

3-pyridinecarboxylic acid, 5-cyano-1,4-dihydro-6-methyl-2-[(phenylsulfonyl)methyl]-4-[2-(trifluoromethyl)phenyl]-, 2-propenyl ester;

3-pyridinecarboxylic acid, 5-cyano-2-[[[4-(1,1-dimethylethyl)-2-methylphenyl]sulfonyl]methyl]-1,4-dihydro-6-methyl-4-[2-(trifluoromethyl)phenyl]-, ethyl ester;

3-pyridinecarboxylic acid, 5-cyano-2-[[(2-fluorophenyl)sulfonyl]methyl]-1,4-dihydro-6-methyl-4-[2-(trifluoromethyl)phenyl]-, ethyl ester;

3-pyridinecarboxylic acid, 2-[[(4-chlorophenyl)sulfonyl]methyl]-5-cyano-1,4-dihydro-6-methyl-4-[2-(trifluoromethyl)phenyl]-, ethyl ester;

3-pyridinecarboxylic acid, 5-cyano-1,4-dihydro-6-methyl-4-(3-nitrophenyl)-2-[(phenylsulfonyl)methyl]-, ethyl ester;

3-pyridinecarboxylic acid, 5-cyano-1,4-dihydro-6-methyl-2-[(phenylsulfonyl)methyl]-4-[2-(trifluoromethyl)phenyl]-, 2,3-dihydroxypropyl ester;

3-pyridinecarboxylic acid, 5-cyano-1,4-dihydro-2-[[(3-methoxy-3-oxopropyl)sulfonyl]methyl]-6-methyl-4-[2(trifluoromethyl)phenyl]-, ethyl ester;

3-quinolinecarboxylic acid, 1,4,5,6,7,8-hexahydro-1-methyl-5-oxo-2-[(phenylsulfonyl)methyl]-4-[2-(trifluoromethyl)phenyl]-, ethyl ester;

3-quinolinecarboxylic acid, 1,4,5,6,7,8-hexahydro-5-oxo-2-[(phenylsulfonyl)methyl]-4-[2-(trifluoromethyl)phenyl]-, ethyl ester; and

3-quinolinecarboxylic acid, 1,4,5,6,7,8-hexahydro-1-methyl-5-oxo-2-[(phenylthio)methyl]-4-[2-(trifluoromethyl)phenyl]-, ethyl ester.

9. A process for preparing a pharmaceutical composition which process comprises combining an effective amount of a compound prepared by a process as claimed in any preceding claim together with a pharmaceutically acceptable carrier or diluent.

10. For use in a method of treating a mammal suffering from a cardiovascular disorder, a compound prepared by a process as claimed in any one of Claims 1 to 8 or a pharmaceutical composition prepared by a process as claimed in Claim 9.

**Patentansprüche für die Vertragsstaaten: BE CH DE FR GB IT LI LU NL SE**

1. Verbindung der folgenden allgemeinen Formel (I)

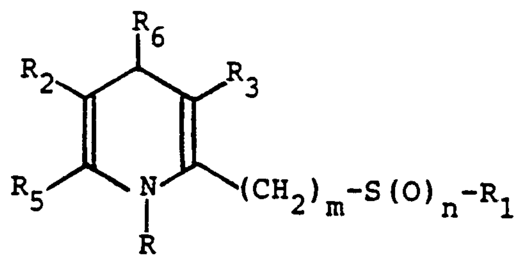

(I)

und ein pharmazeutisch akzeptables Salz davon; worin

R ist:

(1) Wasserstoff;

(2) Niedrigalkyl mit einem bis vier Kohlenstoffatomen, gegebenenfalls substituiert mit einer Aminogruppe der Formel $NR_7R_8$, worin $R_7$ und $R_8$ unabhängig voneinander Wasserstoff oder Niedrigalkyl mit einem bis vier Kohlenstoffatomen sind, oder $R_7$ und $R_8$, wenn sie mit dem Stickstoffatom, an welches sie gebunden sind, zusammengenommen werden, einen fünf- oder sechsgliedrigen Ring mit vier oder fünf $CH_2$-Gruppen bilden; oder

(3) eine Arylalkylenradikal, worin der Arylteil Phenyl ist, gegebenenfalls substituiert mit einem oder mehreren von (i) Halogen, (ii) Trifluormethyl, (iii) Nitro, (iv) Amino, (v) Mono- oder Dialkylamino, worin das oder jedes Alkyl für sich ein Niedrigalkyl mit einem bis sechs Kohlenstoffatomen ist, oder im Falle von Dialkylamino, worin die zwei Alkyl, wenn sie zusammengenommen werden, ein Alkylen mit einem bis sechs Kohlenstoffatomen repräsentieren, (vi) Cyano, (vii) Niedrigalkoxy mit einem bis vier Kohlenstoffatomen, (viii) Niedrigthioalkyl mit einem bis vier Kohlenstoffatomen, und worin der Alkylenteil ein bis sechs Kohlenstoffatome hat;

$R_2$ für Cyano steht;

$R_3$ ist: Wasserstoff, Niedrigalkyl mit einem bis vier Kohlenstoffatomen, Cyano, Nitro, $—CO_2R_4$, worin $R_4$ ist: Wasserstoff oder Niedrigalkyl mit einem bis vier Kohlenstoffatomen, gegebenenfalls substituiert mit Amino, Mono- oder Dialkylamino, worin das oder jedes Alkyl für sich ein Niedrigalkyl mit einem bis sechs Kohlenstoffatomen ist, oder im Fall von Dialkylamino, worin die zwei Alkyl, wenn sie zusammengenommen werden, ein Alkylen mit einem bis sechs Kohlenstoffatomen repräsentieren;

$R_1$ ist:

(1) Cycloalkyl mit vier bis sieben Kohlenstoffatomen;

(2) Phenyl gegebenenfalls substituiert mit einem oder mehreren von (i) Alkyl mit einem bis vier Kohlenstoffatomen, (ii) Halogen, (iii) Trifluormethyl, (iv) Nitro, (v) Amino und (vi) Mono- oder Di-Alkylamino, worin das oder jedes Alkyl für sich ein Niedrigalkyl mit einem bis sechs Kohlenstoffatomen oder im Fall von Dialkylamino, worin die zwei Alkyl, wenn sie zusammengenommen werden, ein Alkylen mit einem bis sechs Kohlenstoffatomen repräsentieren; oder

(3) Thienyl, Furanyl, Pyryl, Pyridinyl, Chinolyl, Isochinolyl, Pyrimidyl, Pyridazinyl, Chinazolyl, Chinoxalyl, Benzothienyl, Pyrazolyl, Imidazolyl, Oxazolyl, Isoxazolyl, Thiazolyl, Triazolyl, Pyrazinyl, Oxazinyl, Thiazinyl, Indolizinyl, Indolyl, Benzofuranyl, Indazolyl, Benzothienyl, Benzimidazolyl, Benzoxazolyl, Benzioxazolyl, Benzthiazolyl, Benztriazolyl, Benzoxadiazolyl, Cinnolinyl, Phthalaxinyl, Naphthyridinyl oder Benzothiazinyl;

$R_5$ und $R_6$, welche gleich oder verschieden sein können, jeweils sind:

(1) Niedrigalkyl mit einem bis vier Kohlenstoffatomen, gegebenenfalls substituiert mit (a) einer

Aminogruppe der Formel $NR_7R_8$, worin $R_7$ und $R_8$ jedes für sich Wasserstoff oder Niedrigalkyl sind, oder $R_7$ und $R_8$, wenn sie mit dem Stickstoffatom, an welches sie gebunden sind, zusammengenommen werden, einen fünf- oder sechsgliedrigen Ring mit vier oder fünf Kohlenstoffatomen bilden, (b) $—OR_9$, worin $R_9$ Wasserstoff oder Niedrigalkyl mit einem bis vier Kohlenstoffatomen ist, oder (c) $—CO_2R_4$, worin $R_4$ wie oben definiert ist; (2) Cycloalkyl mit vier bis sieben Kohlenstoffatomen; (3) Phenyl gegebenenfalls substituiert mit einem oder mehreren von (i) Alkyl mit einem bis vier Kohlenstoffatomen, (ii) Halogen, (iii) Trifluormethyl, (iv) Nitro, (v) Amino, (vi) Mono- oder Dialkylamino, worin das oder jedes Alkyl für sich ein Niedrigalkyl mit einem bis sechs Kohlenstoffatomen ist, oder im Fall von Dialkylamino, worin die zwei Alkyl, wenn sie zusammengenommen werden, ein Alkylen mit einem bis sechs Kohlenstoffatomen repräsentieren, (vii) Cyano, (viii) Niedrigalkoxy mit einem bis vier Kohlenstoffatomen und (ix) Niedrigthioalkyl mit einem bis vier Kohlenstoffatomen;

(4) Arylalkylen, worin der Arylteil Phenyl ist, welches gegebenenfalls substituiert ist mit einem oder mehreren von (i) Alkyl mit einem bis vier Kohlenstoffatomen, (ii) Halogen, (iii) Trifluormethyl, (iv) Nitro, (v) Amino, (vi) Mono- oder Dialkylamino, worin das oder jedes Alkyl für sich ein Niedrigalkyl mit einem bis sechs Kohlenstoffatomen ist, oder im Fall eines Dialkylamino, worin die zwei Alkyl, wenn sie zusammengenommen werden, ein Alkylen mit einem bis sechs Kohlenstoffatomen repräsentieren, (vii) Cyano, (viii) Niedrigalkoxy mit einem bis vier Kohlenstoffatomen und (ix) Niedrigthioalkyl mit einem bis vier Kohlenstoffatomen; und worin der Alkylenteil ein bis vier Kohlenstoffatome aufweist; oder

(5) Thienyl, Furanyl, Pyryl, Pyridinyl, Chinolyl, Isochinolyl, Pyrimidyl, Pyridazinyl, Chinazolyl, Chinoxalyl, Benzothienyl, Pyrazolyl, Imidazolyl, Oxazolyl, Isoxazolyl, Thiazolyl, Triazolyl, Pyrazinyl, Oxazinyl, Thiazinyl, Indolizinyl, Indolyl, Benzofuranyl, Indazolyl, Benzothienyl, Benzimidazolyl, Benzoxazolyl, Benzioxazolyl, Benzthiazolyl, Benztriazolyl, Benzoxadiazolyl, Cinnolinyl, Phthalaxinyl, Naphthyridinyl oder Benzothiazinyl;

und worin $R_2$ und $R_5$ auch zusammengenommen werden können, um einen fünf- oder sechsgliedrigen Ring zu bilden, welcher als ein Ringglied eine

$$—\overset{\overset{O}{\|}}{C}— \text{ oder } —\overset{\overset{O}{\|}}{C}—\text{O-Gruppe}$$

aufweist;

n für Eins oder Zwei steht; m eine ganze Zahl von Eins bis Sechs darstellt.

2. Verbindung der Formel (II)

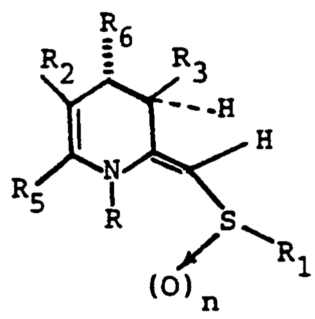

(II)

und ein pharmazeutisch akzeptables Salz davon; worin R ist:

(1) Wasserstoff;

(2) Niedrigalkyl mit einem bis vier Kohlenstoffatomen, gegebenenfalls substituiert mit einer Aminogruppe der Formel $NR_7R_8$, worin $R_7$ und $R_8$ unabhängig voneinander Wasserstoff oder Niedrigalkyl mit einem bis vier Kohlenstoffatomen sind, oder $R_7$ und $R_8$, wenn sie mit dem Stickstoffatom, an welches sie gebunden sind, zusammengenommen werden, einen fünf- oder sechsgliedrigen Ring bilden, welcher vier oder fünf $CH_2$-Gruppen aufweist; oder

(3) eine Arylalkylenradikal, worin der Arylteil Phenyl ist, welches gegebenenfalls substituiert ist mit einem oder mehreren von (i) Halogen, (ii) Trifluormethyl, (iii) Nitro, (iv) Amino, (v) Mono- oder Dialkylamino, worin das oder jedes Alkyl für sich ein Niedrigalkyl mit einem bis sechs Kohlenstoffatomen ist oder im Fall von Dialkylamino, worin die zwei Alkyl, wenn sie zusammengenommen werden, ein Alkylen mit einem bis sechs Kohlenstoffatomen repräsentieren, (vi) Cyano, (vii) Niedrigalkoxy mit einem bis vier Kohlenstoffatomen, (viii) Niedrigalkyl mit einem bis vier Kohlenstoffatomen, (ix) Niedrigthioalkyl mit einem bis vier Kohlenstoffatomen; und worin der Alkylenteil ein bis vier Kohlenstoffatome aufweist;

$R_2$ und $R_3$, welche gleich oder verschieden sein können, jeweils sind: Wasserstoff, Niedrigalkyl mit einem bis vier Kohlenstoffatomen, Cyano, Nitro, $—CO_2R_4$, worin $R_4$ Wasserstoff oder Niedrigalkyl mit einem bis vier Kohlenstoffatomen ist, gegebenenfalls substituiert mit Amino, Mono- oder Dialkylamino, worin das oder jedes Alkyl für sich ein Niedrigalkyl mit einem bis sechs Kohlenstoffatomen ist, oder im Fall eines Dialkylamino, worin die zwei Alkyl, wenn sie zusammengenommen werden, ein Alkylen mit einem bis sechs Kohlenstoffatomen repräsentieren;

$R_1$, $R_5$ und $R_6$, welche gleich oder verschieden sein können, jeweils sind:

(1) Niedrigalkyl mit 1 bis 4 Kohlenstoffatomen, gegebenenfalls substituiert mit (a) einer Aminogruppe der Formel $NR_7R_8$, worin $R_7$ und $R_8$ unabhängig voneinander Wasserstoff oder Niedrigalkyl sind, $R_7$ und $R_8$, wenn sie mit dem Stickstoffatom, an welches sie gebunden sind, zusammengenommen werden, einen fünf- oder sechsgliedrigen ring mit vier bis fünf Kohlenstoffatomen bilden, (b) —$OR_9$, worin $R_9$ Wasserstoff oder Niedrigalkyl mit einem bis vier Kohlenstoffatomen ist, oder (c) —$CO_2R_4$, worin $R_4$ wie oben definiert ist;

(2) Cycloalkyl mit vier bis sieben Kohlenstoffatomen;

(3) Phenyl gegebenenfalls substituiert mit einem oder mehreren von (i) Alkyl mit einem bis vier Kohlenstoffatomen, (ii) Halogen, (iii) Trifluormethyl, (iv) Nitro, (v) Amino, (vi) Mono- oder Di-alkylamino, worin das oder jedes Alkyl für sich ein Niedrigalkyl mit einem bis sechs Kohlenstoffatomen ist oder im Fall von Dialkylamino, worin die zwei Alkyl, wenn sie zusammengenommen werden, ein Alkylen mit einem bis sechs Kohlenstoffatomen repräsentieren; (vii) Cyano, (viii) Niedrigalkoxy mit einem bis vier Kohlenstoffatomen und (ix) Niedrigthioalkyl mit einem bis vier Kohlenstoffatomen;

(4) Arylalkylen, worin der Arylteil Phenyl ist, gegebenenfalls substituiert mit einem oder mehreren von (i) Alkyl mit einem bis vier Kohlenstoffatomen, (ii) Halogen, (iii) Trifluormethyl, (iv) Nitro, (v) Amino, (vi) Mono- oder Dialkylamino, worin das oder jedes Alkyl für sich ein Niedrigalkyl mit einem bis sechs Kohlenstoffatomen ist, oder im Fall von Dialkylamino, worin die zwei Alkyl, wenn sie zusammengenommen werden, ein Alkylen mit einem bis sechs Kohlenstoffatomen repräsentieren, (vii) Cyano, (viii) Niedrigalkoxy mit einem bis vier Kohlenstoffatomen, (ix) Niedrigthioalkyl mit einem bis vier Kohlenstoffatomen; und worin der Alkylenteil ein bis vier Kohlenstoffatome, hat; oder

(5) Thienyl, Furanyl, Pyryl, Pyridinyl, Chinolyl, Isochinolyl, Pyrimidyl, Pyridazinyl, Chinazolyl, Chinoxalyl, Benzothienyl, Pyrazolyl, Imidazolyl, Oxazolyl, Isoxazolyl, Thiazolyl, Triazolyl, Pyrazinyl, Oxazinyl, Thiazinyl, Indolizinyl, Indolyl, Benzofuranyl, Indazolyl, Benzothienyl, Benzimidazolyl, Benzoxazolyl, Benzioxazolyl, Benzthiazolyl, Benztriazolyl, Benzoxadiazolyl, Cinnolinyl, Phthalaxinyl, Naphthyridinyl oder Benzothiazinyl;

und worin $R_2$ und $R_5$ ebenso zusammengenommen werden können, um einen fünf- oder sechgliedrigen Ring zu bilden, welcher als ein Ringglied eine

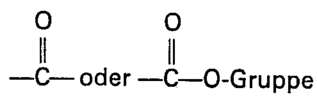
oder -Gruppe

aufweist;

n für Null oder eine ganze Zahl von Eins oder Zwei steht.

3. Verbindung nach Anspruch 2, worin $R_2$ für Cyano steht, und n Eins oder Zwei ist.

4. Verbindung nach Anspruch 1 oder 2 mit der Formel

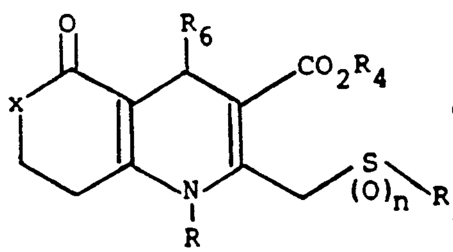

oder

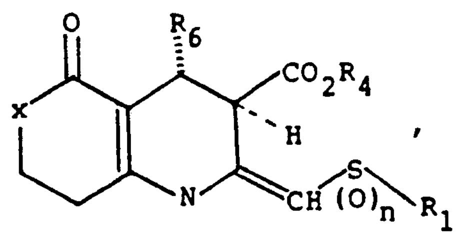
,

worin X für $>CH_2$ oder ein Sauerstoffatom steht.

5. Verbindung nach Anspruch 1 oder 2, worin R für Wasserstoff steht, $R_2$ Cyano ist, $R_3$ für —$CO_2R_4$ steht, $R_5$ Methyl ist, und m Eins ist.

6. Verbindung nach Anspruch 2, worin R Wasserstoff ist, $R_2$ und $R_3$ unabhängig voneinander —$CO_2R_4$ sind, $R_5$ Methyl ist, und m für Eins steht.

7. Verbindung nach Anspruch 4, 5 oder 6, wenn auf Anspruch 2 rückbezogen, worin n für Null steht.

8. Verbindung nach Anspruch 1 oder 2 mit einem der folgenden Namen:

3-Pyridincarbonsäure-5-cyano-1,2,3,4-tetrahydro-2-[(phenylthio)methylen]-6-methyl-4-[(2-trifluormethyl)phenyl]-ethylester;

3-Chinolincarbonsäure-1,2,3,4,5,6,7,8-octahydro-5-oxo-2-[(phenylthio)methylen]-4-[2-(trifluormethyl)-phenyl]-ethylester;

Ethyl-5-cyano-1,4-dihydro-6-methyl-2-[(phenylsulfinyl)-methyl)-4-[2-(trifluormethyl)-phényl]-3-pyrindincarboxylat;

Ethyl-5-cyano-1,4-dihydro-6-methyl-2-[[(2-methylphenyl)sulfinyl]-methyl]-4-[2-(trifluormethyl)-phenyl]-3-pyridincarboxylat;

Ethyl-5-cyano-1,4-dihydro-6-methyl-4-(3-nitrophenyl)-2-[(phenylsulfinyl)-methyl]-3-pyridincarboxylat;

Ethyl-5-cyano-1,4-dihydro-6-methyl-2-[[(4-methoxyphenyl)sulfinyl]-methyl]-4-[2-(trifluormethyl)-phenyl]-3-pyridincarboxylat;

Ethyl-5-cyano-1,4-dihydro-6-methyl-2-[(phenylsulfinyl)methyl]-4-[2-(chlorphenyl)-3-pyridincarboxylat;

Ethyl-5-cyano-1,4-dihydro-6-methyl-2-[(4-pyridinylsulfinyl)-methyl]-4-[2-(trifluormethyl)-phenyl]-3-pyridincarboxylat;

Isethyl-2-[[(2-chlorphenyl)-sulfinyl]-methyl]-5-cyano-1,4-dihydro-6-methyl-4-[(2-trifluormethyl)-phenyl]-3-pyridincarboxylat;

5-cyano-2-[[[2-(dimethylamino)-ethyl]-sulfinyl]-methyl]-1,4-dihydro-6-methyl-4-[2-(trifluormethyl)-phenyl]-3-pyridincarbonsäure-ethylester;

Ethyl-4-(2-chlorphenyl)-5-cyano-1,4-dihydro-6-methyl-2-[(4-pyridinylsulfinyl)-methyl]-3-pyridincarboxylat;

Ethyl-5-cyano-4-(2,3-dichlorphenyl)-1,4-dihydro-6-methyl-2-[(4-pyridinylsulfinyl)-methyl-3-pyridincarboxylat;

5-Cyano-1,4-dihydro-6-methyl-2-[(2-pyrimidinylsulfinyl)-methyl]-4-[2-(trifluormethyl)-phenyl]-3-pyridincarbonsäure-ethylester;

5-Cyano-1,4-dihydro-6-methyl-2-[[(1-methyl-1*H*-imidazol-2-yl)sulfinyl]-methyl]-4-[2-(trifluormethyl)-phenyl]-3-pyridincarbonsäure-ethylester;

Ethyl-5-cyano-1,4-dihydro-6-methyl-2-[(phenylsulfinyl)-methyl]-4-phenyl-3-pyridincarboxylat;

3,5-Pyridin-dicarbonsäure-2-[[(2-chlorphenyl)-sulfinyl]methylen]-6-methyl-1,2,3,4-tetrahydro-4-[(2-trifluormethyl)phenyl]-3-ethyl-5-methylester;

3-Pyridincarbonsäure-5-cyano-4-cyclohexyl-1,4-dihydro-6-methyl-2-[(phenylsulfinyl)methyl]-ethylester (±)-;

3-Pyridincarbonsäure-5-cyano-4-[2-(difluormethoxy)-phenyl]-1,4-dihydro-6-methyl-2-[(phenylsulfinyl)methyl]-ethylester;

3-Pyridincarbonsäure-5-cyano-1,4-dihydro-6-methyl-2-[(phenylsulfinyl)-methyl]-4-[2-(trifluormethyl)-phenyl]-methylester; (53,47 Mischung von Isomeren);

3-Pyridin-carbonsäure-5-cyano-1,4-dihydro-6-methyl-2-[(phenylsulfinyl)-methyl]-4-[2-(trifluormethyl)-phenyl]-1-methylethylester;

3-Pyridin-carbonsäure-5-cyano-1,4-dihydro-6-methyl-2-[(phenylsulfinyl)-methyl]-4-[2-(trifluormethyl)-phenyl]-2-hydroxyethylester;

3-Pyridincarbonsäure-5-cyano-1,4-dihydro-6-methyl-4-(2-phenyl-1*H*-imidazol-4-yl)-2-[(phenylsulfinyl)-methyl]-ethylester;

3-Pyridincarbonsäure-5-cyano-1,4-dihydro-6-methyl-2-[(2-pyridinylsulfinyl)-methyl]-4-[2-(trifluormethyl)-phenyl]-ethylester (±)-;

3-Pyridincarbonsäure-5-cyano-2-[[(2-fluorphenyl)-sulfinyl]-methyl]-1,4-dihydro-6-methyl-4-[2-(trifluormethyl)-phenyl]-ethylester;

3-Pyridincarbonsäure-2-[[[4-(acetylamino)-phenyl]-sulfinyl]-methyl]-5-cyano-1,4-dihydro-6-methyl-4-[2-(trifluormethyl)-phenyl]-ethylester;

3-Pyridincarbonsäure-2-[[(4-chlorphenyl)-sulfinyl]-methyl]-5-cyano-1,4-dihydro-6-methyl-4-[2-(trifluormethyl)-phényl]-ethylester (±)-;

3-Pyridincarbonsäure-5-cyano-2-[[(3,4-dichlorphenyl)-sulfinyl]-methyl]-1,4-dihydro-6-methyl-4-[2-(trifluormethyl)-phenyl]-ethylester;

3-Pyridincarbonsäure-5-cyano-1,4-dihydro-6-methyl-2-[(phenylsulfinyl)-methyl]-4-[2-(trifluormethyl)-phenyl]-;

3-Chinolincarbonsäure-1,4,5,6,7,8-hexahydro-1-methyl-5-oxo-2-[(phenylsulfinyl)-methyl]-4-[2-(trifluormethyl)-phenyl]-ethylester;

3-Chinolincarbonsäure-1,4,5,6,7,8-hexahydro-5-oxo-2-[(phenylsulfinyl)-methyl]-4-[2-(trifluormethyl)-phenyl]-ethylester (±)-;

Ethyl-3-cyano-1,4-dihydro-6-methyl-2-[(phenylsulfinyl)-methyl]-4-[2-(trifluormethyl)-phenyl]-5-pyridincarboxylat;

3-Pyridincarbonsäure-5-cyano-1-[2-(diethylamino)-ethyl]-1,4-dihydro-6-methyl-2-[(phenylsulfinyl)-methyl]-4-[2-(trifluormethyl)-phenyl]-ethylester;

3-Pyridincarbonsäure-5-cyano-1,4-dihydro-[[(3-methoxy-3-oxopropyl]-sulfinyl]-methyl]-6-methyl-4-[2-(trifluormethyl)-phenyl]-ethylester;

3-Pyridincarbonsäure-5-cyano-2-[[(2-ethoxy-2-oxo-ethyl)-sulfinyl]-methyl]-1,4-dihydro-6-methyl-4-[2-(trifluorméthyl)-phenyl]-ethylester;

[2,4'-Bipyridin]-3'-carbonsäure-5'-cyano-1',4'-dihydro-6'-methyl-2'-[(phenylsulfinyl)-methyl]-ethylester;

Ethyl-5-cyano-1,4-dihydro-6-methyl-2-[(phenylsulfonyl)-methyl]-4-[2-(trifluormethyl)-phenyl]-3-pyridincarboxylat;

Ethyl-5-cyano-1,4-dihydro-6-methyl-2-[[(4-methoxyphenyl)-sulfonyl]-methyl]-4-[2-(trifluormethyl)-phenyl]-3-pyridincarboxylat;

Ethyl-5-cyano-1,4-dihydro-6-methyl-2-[[(2-methylphenyl)-sulfonyl]-methyl]-4-[2-(trifluormethyl)-phenyl]-3-pyridincarboxylat;

Ethyl-2-[[(2-chlorphenyl)-sulfonyl]-methyl]-5-cyano-1,4-dihydro-6-methyl-4-[2-(trifluormethyl)-phenyl]-3-pyridincarboxylat;

Ethyl-5-cyano-1,4-dihydro-6-methyl-2-[(4-pyridinylsulfonyl)-methyl]-4-[2-(trifluormethyl)-phényl]-3-pyridincarboxylat;

5-Cyano-1,4-dihydro-6-methyl-2-[(2-pyridinylsulfonyl)-methyl]-4-[2-(trifluormethyl)-phenyl]-3-pyridincarbonsäure-ethylester-N-oxid;

Ethyl-5-cyano-1,4-dihydro-6-methyl-2-[(phenylsulfonyl)-methyl]-4-phényl-3-pyridincarboxylat;

[2,4'-Bipyridin]-3'-carbonsäure-5'-cyano-1',4'-dihydro-6'-methyl-2'-[(phenylsulfonyl)-methyl]-ethylester-1-oxid;

[3,4'-Bipyridin]-3'-carbonsäure-5'-cyano-1',4'-dihydro-6'-methyl-2'-[(phenylsulfonyl)-methyl]-ethylester;

3-Pyridin-carbonsäure-2-[[(4-aminophenyl)-sulfonyl]-methyl]-5-cyano-1,4'-dihydro-6-methyl-4-[2-(trifluormethyl)-phenyl]-ethylester;

[2,4'-Bipyridin]-3'-carbonsäure-5'-cyano-1',4'-dihydro-6'-methyl-2'-[(phenylsulfonyl)-methyl]-ethylester;

3-Pyridincarbonsäure-5-cyano-4-cyclohexyl-6-methyl-1,4-dihydro-2-[(phenylsulfonyl)-methyl]-ethylester;

3-Pyridincarbonsäure-5-cyano-1,4-dihydro-6-methyl-2-[(phenylsulfonyl)-methyl]-4-[2-(trifluormethyl)-phenyl]-1-methylethylester;

3-Pyridin-carbonsäure-5-cyano-1,4-dihydro-6-methyl-2-[(phenylsulfonyl)-methyl]-4-[2-(trifluormethyl)-phenyl]-2-propenylester;

3-Pyridincarbonsäure-5-cyano-2[[[4-(1,1-dimethylethyl)-2-methylphenyl]-sulfonyl]-methyl]-1,4-dihydro-6-methyl-4-[2-(trifluormethyl)-phenyl]-ethylester;

3-Pyridincarbonsäure-5-cyano-2[[(2-fluorphenyl)-sulfonyl]-methyl]-1,4-dihydro-6-methyl-4-[2-(trifluormethyl)-phenyl]-ethylester;

3-Pyridincarbonsäure-2-[[(4-chlorphenyl)-sulfonyl]-methyl]-5-cyano-1,4-dihydro-6-methyl-4-[2-(trifluormethyl)-phenyl]-ethylester;

3-Pyridincarbonsäure-5-cyano-1,4-dihydro-6-methyl-4-(3-nitrophenyl)-2-[(phenylsulfonyl)-methyl]-ethylester;

3-Pyridincarbonsäure-5-cyano-1,4-dihydro-6-methyl-2-[(phenylsulfonyl)-methyl]-4-[2-(trifluormethyl)-phenyl]-2,3-dihydroxypropylester;

3-Pyridincarbonsäure-5-cyano-1,4-dihydro--2-[[(3-methoxy-3-oxopropyl)-sulfonyl]-methyl]-6-methyl-4-[2-(trifluormethyl)-phenyl]-ethylester;

3-Chinolincarbonsäure-1,4,5,6,7,8-hexahydro-1-methyl-5-oxo-2-[(phenylsulfonyl)-methyl]-4-[2-(trifluormethyl)-phenyl]-ethylester;

3-Chinolincarbonsäure-1,4,5,6,7,8-hexahydro-5-oxo-2-[(phenylsulfonyl)-methyl]-4-[2-(trifluormethyl)-phenyl]-ethylester und

3-Chinolincarbonsäure-1,4,5,6,7,8-hexahydro-1-methyl-5-oxo-2-[(phenylthio)methyl]-4-[2-(trifluormethyl)-phenyl]-ethylester.

9. Pharmazeutische Zusammensetzung, welche eine wirksame Menge einer wie in irgendeinem vorhergehenden Anspruch beanspruchten Verbindung in Mischung mit einem pharmazeutisch akzeptablen Träger oder Verdünnungsmittel umfaßt.

10. Verbindung, wie in irgendeinem der Ansprüche 1 bis 8 beansprucht, oder eine pharmazeutische Zusammensetzung, wie im Anspruch 9 beansprucht, zur Verwendung in einer Methode zur Behandlung eines an einer kardiovaskulären Erkrankung leidenden Säugers.

11. Verfahren zur Herstellung einer Verbindung der Formel (I) oder (II), wie jeweils in den Ansprüchen 1 und 2 beansprucht, welches Verfahren umfaßt: Kontaktieren der Verbindungen

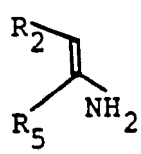

(III),

$R_6CHO$ (IV) und

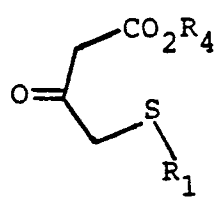

(V),

worin $R_1$, $R_2$, $R_4$, $R_5$ und $R_6$ wie im Anspruch 1 oder 2 definiert sind, in einem organischen Lösungsmittel zur Bildung einer Verbindung der Formel ($I_o$) oder ($II_o$)

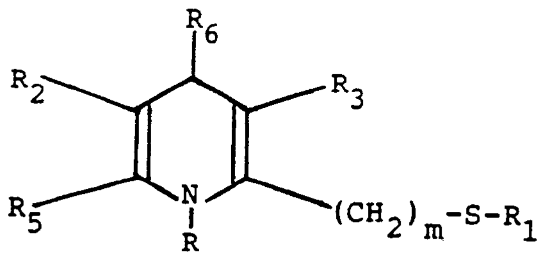

($I_o$)

oder

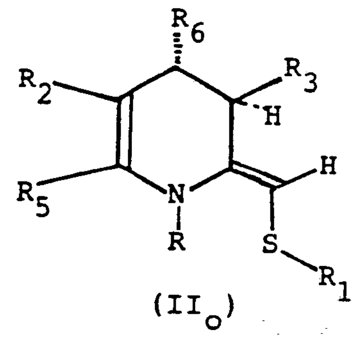

($II_o$)

und gewünschtenfalls weiteres Umsetzen des Produkts in einem oder mehreren der folgenden Schritte:

(1) Sauerstoffanreicherung des Schwefelatoms

(2) Alkylieren des Stickstoffatoms und

(3) Umsetzen der $—CO_2R_4$ Gruppe zum Erhalt einer $R_3$ Gruppe, welche nicht $—CO_2R_4$ ist;

und gegebenenfalls die Bildung eines pharmazeutisch akzeptablen Salzes davon.

**Patentansprüche für den Vertragsstaat: AT**

1. Verfahren zur Herstellung einer Verbindung der folgenden allgemeinen Formel (I)

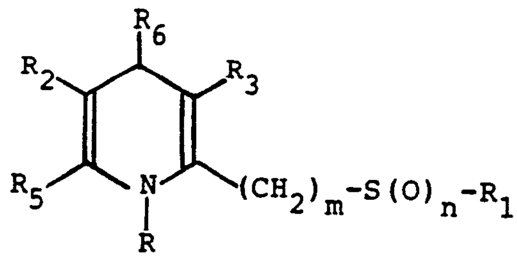

(I)

und eines pharmazeutisch akzeptablen Salzes davon; worin R ist:

(1) Wasserstoff;

(2) Niedrigalkyl mit einem bis vier Kohlenstoffatomen, gegebenenfalls substituiert mit einer Aminogruppe der Formel $NR_7R_8$, worin $R_7$ und $R_8$ unabhängig voneinander Wasserstoff oder Niedrigalkyl mit einem bis vier Kohlenstoffatomen sind, oder $R_7$ und $R_8$, wenn sie mit dem Stickstoffatom, an welches sie gebunden sind, zusammengenommen werden, einen fünf- oder sechsgliedrigen Ring mit vier oder fünf $CH_2$-Gruppen bilden; oder

(3) eine Arylalkylenradikal, worin der Arylteil Phenyl ist, gegebenenfalls substituiert mit einem oder mehreren von (i) Halogen, (ii) Trifluormethyl, (iii) Nitro, (iv) Amino, (v) Mono- oder Dialkylamino, worin das oder jedes Alkyl für sich ein Niedrigalkyl mit einem bis sechs Kohlenstoffatomen ist, oder im Falle von Dialkylamino, worin die zwei Alkyl, wenn sie zusammengenommen werden, ein Alkylen mit einem bis sechs Kohlenstoffatomen repräsentieren, (vi) Cyano, (vii) Niedrigalkoxy mit einem bis vier Kohlenstoffatomen, (viii) Niedrigthioalkyl mit einem bis vier Kohlenstoffatomen, und worin der Alkylenteil ein bis sechs Kohlenstoffatome hat;

$R_2$ für Cyano steht;

$R_3$ ist: Wasserstoff, Niedrigalkyl mit einem bis vier Kohlenstoffatomen, Cyano, Nitro, $—CO_2R_4$, worin $R_4$ Wasserstoff oder Niedrigalkyl mit einem bis vier Kohlenstoffatomen, gegebenenfalls substituiert mit Amino, Mono- oder Dialkylamino, worin das oder jedes Alkyl für sich ein Niedrigalkyl mit einem bis sechs Kohlenstoffatomen ist oder im Fall von Dialkylamino, worin die zwei Alkyl, wenn sie zusammengenommen werden, ein Alkylen mit einem bis sechs Kohlenstoffatomen repräsentieren;

$R_1$ ist:

(1) Cycloalkyl mit vier bis sieben Kohlenstoffatomen;

(2) Phenjb xyl gegebenenfalls substituiert mit einem oder mehreren von (i) Ajb clkyl mit einem bis vier Kohlenstoffatomen, (ii) Halogen, (iii) Trifluormethyl, (iv) Nitro, (v) Amino und (vi) Mono- oder Di-Alkylamino, worin das oder jedes Alkyl für sich ein Niedrigalkyl mit einem bis sechs Kohlenstoffatomen oder im Fall von Dialkylamino, worin die zwei Alkyl, wenn sie zusammengenommen werden, ein Alkylen mit einem bis sechs Kohlenstoffatomen repräsentieren; oder

(3) Thienyl, Furanyl, Pyryl, Pyridinyl, Chinolyl, Isochinolyl, Pyrimidyl, Pyridazinyl, Chinazolyl, Chinoxalyl, Benzothienyl, Pyrazolyl, Imidazolyl, Oxazolyl, Isoxazolyl, Thiazolyl, Triazolyl, Pyrazinyl,

Oxazinyl, Thiazinyl, Indolizinyl, Indolyl, Benzofuranyl, Indazolyl, Benzothienyl, Benzimidazolyl, Benzoxazolyl, Benzioxazolyl, Benzthiazolyl, Benztriazolyl, Benzoxadiazolyl, Cinnolinyl, Phthalaxinyl, Naphthyridinyl oder Benzothiazinyl;

$R_5$ und $R_6$, welche gleich oder verschieden sein können, jeweils sind:

(1) Niedrigalkyl mit einem bis vier Kohlenstoffatomen, gegebenenfalls substituiert mit (a) einer Aminogruppe der Formel $NR_7R_8$, worin $R_7$ und $R_8$ jedes für sich Wasserstoff oder Niedrigalkyl sind, oder $R_7$ und $R_8$, wenn sie mit dem Stickstoffatom, an welches sie gebunden sind, zusammengenommen werden, einen fünf- oder sechsgliedrigen Ring mit vier oder fünf Kohlenstoffatomen bilden, (b) —$OR_9$, worin $R_9$ Wasserstoff oder Niedrigalkyl mit einem bis vier Kohlenstoffatomen ist, oder (c) —$CO_2R_4$, worin $R_4$ wie oben definiert ist; (2) Cycloalkyl mit vier bis sieben Kohlenstoffatomen; (3) Phenyl gegebenenfalls substituiert mit einem oder mehreren von (i) Alkyl mit einem bis vier Kohlenstoffatomen, (ii) Halogen, (iii) Trifluormethyl, (iv) Nitro, (v) Amino, (vi) Mono- oder Dialkylamino, worin das oder jedes Alkyl für sich ein Niedrigalkyl mit einem bis sechs Kohlenstoffatomen ist, oder im Fall von Dialkylamino, worin die zwei Alkyl, wenn sie zusammengenommen werden, ein Alkylen mit einem bis sechs Kohlenstoffatomen repräsentieren, (vii) Cyano, (viii) Niedrigalkoxy mit einem bis vier Kohlenstoffatomen und (ix) Niedrigthioalkyl mit einem bis vier Kohlenstoffatomen;

(4) Arylalkylen, worin der Arylteil Phenyl ist, welches gegebenenfalls substituiert ist mit einem oder mehreren von (i) Alkyl mit einem bis vier Kohlenstoffatomen, (ii) Halogen, (iii) Trifluormethyl, (iv) Nitro, (v) Amino, (vi) Mono- oder Dialkylamino, worin das oder jedes Alkyl für sich ein Niedrigalkyl mit einem bis sechs Kohlenstoffatomen ist, oder im Fall eines Dialkylamino, worin die zwei Alkyl, wenn sie zusammengenommen werden, ein Alkylen mit einem bis sechs Kohlenstoffatomen repräsentieren, (vii) Cyano, (viii) Niedrigalkoxy mit einem bis vier Kohlenstoffatomen und (ix) Niedrigthioalkyl mit einem bis vier Kohlenstoffatomen; und worin der Alkylenteil ein bis vier Kohlenstoffatome aufweist; oder

(5) Thienyl, Furanyl, Pyryl, Pyridinyl, Chinolyl, Isochinolyl, Pyrimidyl, Pyridazinyl, Chinazolyl, Chinoxalyl, Benzothienyl, Pyrazolyl, Imidazolyl, Oxazolyl, Isoxazolyl, Thiazolyl, Triazolyl, Pyrazinyl, Oxazinyl, Thiazinyl, Indolizinyl, Indolyl, Benzofuranyl, Indazolyl, Benzothienyl, Benzimidazolyl, Benzoxazolyl, Benzioxazolyl, Benzthiazolyl, Benztriazolyl, Benzoxadiazolyl, Cinnolinyl, Phthalaxinyl, Naphthyridinyl oder Benzothiazinyl;

und worin $R_2$ und $R_5$ auch zusammengenommen werden können, um einen fünf- oder sechgliedrigen Ring zu bilden, welcher als ein Ringglied eine

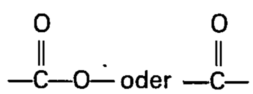
oder Gruppe

aufweist;

n für Eins oder Zwei steht; m eine ganze Zahl von Eins bis Sechs darstellt;

welches Verfahren umfaßt: Kontaktieren der Verbindungen

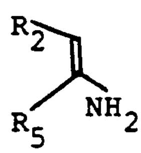
(III),

$R_6CHO$ (IV) und

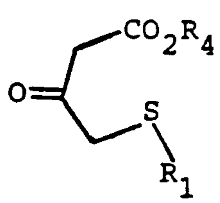
(V),

worin $R_1$, $R_2$, $R_4$, $R_5$ und $R_6$ wie oben definiert sind, in einem organischen Lösungsmittel zur Bildung einer verbindung der Formel ($I_o$)

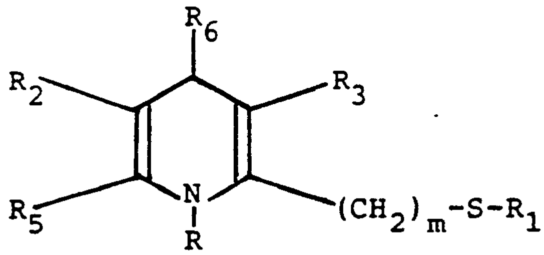

($I_o$)

und gewünschtenfalls weiteres Umsetzen des Produkts in einem oder mehreren der folgenden Schritte:

(1) Sauerstoffanreicherung des Schwefelatoms

(2) Alkylieren des Stickstoffatoms und

(3) Umsetzen der $—CO_2R_4$-Gruppe zum Erhalt einer $R_3$-Gruppe, welche nicht $—CO_2R_4$ ist;

und gegebenenfalls die Bildung eines pharmazeutisch akzeptablen Salzes davon.

2. Verfahren zur Herstellung einer Verbindung der Formel (II)

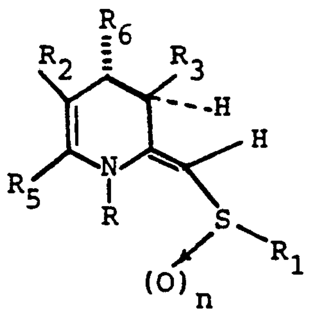

(II)

und eines pharmazeutisch akzeptablen Salzes davon; worin R ist:

(1) Wasserstoff;

(2) Niedrigalkyl mit einem bis vier Kohlenstoffatomen, gegebenenfalls substituiert mit einer Aminogruppe der Formel $NR_7R_8$, worin $R_7$ und $R_8$ unabhängig voneinander Wasserstoff oder Niedrigalkyl mit einem bis vier Kohlenstoffatomen sind, oder $R_7$ und $R_8$, wenn sie mit dem Stickstoffatom, an welches sie gebunden sind, zusammengenommen werden, einen fünf- oder sechsgliedrigen Ring bilden, welcher vier oder fünf $CH_2$-Gruppen bilden; oder

(3) eine Arylalkylenradikal, worin der Arylteil Phenyl ist, welches gegebenenfalls substituiert ist mit einem oder mehreren von (i) Halogen, (ii) Trifluormethyl, (iii) Nitro, (iv) Amino, (v) Mono- oder Dialkylamino, worin das oder jedes Alkyl für sich ein Niedrigalkyl mit einem bis sechs Kohlenstoffatomen ist oder im Fall von Dialkylamino, worin die zwei Alkyl, wenn sie zusammengenommen werden, ein Alkylen mit einem bis sechs Kohlenstoffatomen repräsentieren, (vi) Cyano, (vii) Niedrigalkoxy mit einem bis vier Kohlenstoffatomen, (viii) Niedrigalkyl mit einem bis vier Kohlenstoffatomen, (ix) Niedrigthioalkyl mit einem bis vier Kohlenstoffatomen; und worin der Alkylenteil ein bis vier Kohlenstoffatome aufweist;

$R_2$ und $R_3$ ist, welche gleich oder verschieden sein können, jeweils sind: Wasserstoff, Niedrigalkyl mit einem bis vier Kohlenstoffatomen, Cyano, Nitro, $—CO_2R_4$, worin $R_4$ Wasserstoff oder Niedrigalkyl mit einem bis vier Kohlenstoffatomen ist, gegebenenfalls substituiert mit Amino, Mono- oder Dialkylamino, worin das oder jedes Alkyl für sich ein Niedrigalkyl mit einem bis sechs Kohlenstoffatomen ist, oder im Fall eines Dialkylamino, worin die zwei Alkyl, wenn sie zusammengenommen werden, ein Alkylen mit einem bis sechs Kohlenstoffatomen repräsentieren;

$R_1$, $R_5$ und $R_6$, welche gleich oder verschieden sein können, jeweils sind:

(1) Niedrigalkyl mit 1 bis 4 Kohlenstoffatomen, gegebenenfalls substituiert mit (a) einer Aminogruppe der Formel $NR_7R_8$, worin $R_7$ und $R_8$ unabhängig voneinander Wasserstoff oder Niedrigalkyl sind, oder $R_7$ und $R_8$, wenn sie mit dem Stickstoffatom, an welches sie gebunden sind, zusammengenommen werden, einen fünf- oder sechsgliedrigen Ring mit vier bis fünf Kohlenstoffatomen bilden, (b) $—OR_9$, worin $R_9$ Wasserstoff oder Niedrigalkyl mit einem bis vier Kohlenstoffatomen ist, oder (c) $—CO_2R_4$, worin $R_4$ wie oben definiert ist;

(2) Cycloalkyl mit vier bis sieben Kohlenstoffatomen;

(3) Phenyl gegebenenfalls substituiert mit einem oder mehreren von (i) Alkyl mit einem bis vier Kohlenstoffatomen, (ii) Halogen, (iii) Trifluormethyl, (iv) Nitro, (v) Amino, (vi) Mono- oder Di-alkylamino, worin das oder jedes Alkyl für sich ein Niedrigalkyl mit einem bis sechs Kohlenstoffatomen oder im Fall von

Dialkylamino, worin die zwei Alkyl, wenn sie zusammengenommen werden, ein Alkylen mit einem bis sechs Kohlenstoffatomen repräsentieren; (vii) Cyano, (viii) Niedrigalkoxy mit einem bis vier Kohlenstoffatomen und (ix) Niedrigthioalkyl mit einem bis vier Kohlenstoffatomen;

(4) Arylalkylen, worin der Arylteil Phenyl ist, gegebenenfalls substituiert mit einem oder mehreren von (i) Alkyl mit einem bis vier Kohlenstoffatomen, (ii) Halogen, (iii) Trifluormethyl, (iv) Nitro, (v) Amino, (vi) Mono- oder Dialkylamino, worin das oder jedes Alkyl für sich ein Niedrigalkyl mit einem bis sechs Kohlenstoffatomen ist, oder im Fall von Dialkylamino, worin die zwei Alkyl, wenn sie zusammengenommen werden, ein Alkylen mit einem bis sechs Kohlenstoffatomen repräsentieren, (vii) Cyano, (viii) Niedrigalkoxy mit einem bis vier Kohlenstoffatomen, (ix) Niedrigthioalkyl mit einem bis vier Kohlenstoffatomen; und worin der Alkylenteil von ein bis vier Kohlenstoffatome hat; oder

(5) Thienyl, Furanyl, Pyryl, Pyridinyl, Chinolyl, Isochinolyl, Pyrimidyl, Pyridazinyl, Chinazolyl, Chinoxalyl, Benzothienyl, Pyrazolyl, Imidazolyl, Oxazolyl, Isoxazolyl, Thiazolyl, Triazolyl, Pyrazinyl, Oxazinyl, Thiazinyl, Indolizinyl, Indolyl, Benzofuranyl, Indazolyl, Benzothienyl, Benzimidazolyl, Benzoxazolyl, Benzioxazolyl, Benzthiazolyl, Benztriazolyl, Benzoxadiazolyl, Cinnolinyl, Phthalaxinyl, Naphthyridinyl oder Benzothiazinyl;

und worin $R_2$ und $R_5$ ebenso zusammengenommen werden können, um einen fünf- oder sechgliedrigen Ring zu bilden, welcher als ein Ringglied eine

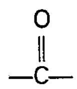 oder 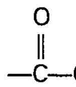O-Gruppe

aufweist;

n für Null oder eine ganze Zahl von Eins oder Zwei steht, welches Verfahren umfaßt: Kontaktieren der Verbindungen

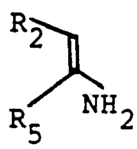

(III),

$R_6CHO$ (IV) und

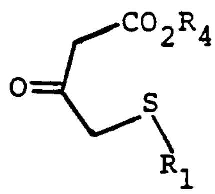

(V),

worin $R_1$, $R_2$, $R_4$, $R_5$ und $R_6$ wie oben definiert sind, in einem organischen Lösungsmittel zur Bildung einer Verbindung der Formel ($I_o$)

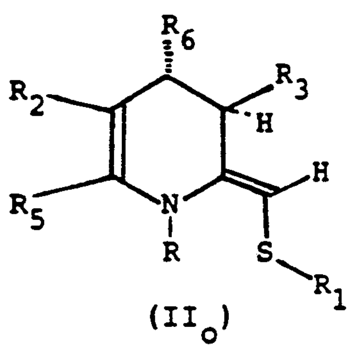

($II_o$)

und gewünschtenfalls weiteres Umsetzen des Produkts in einem oder mehreren der folgenden Schritte:

(1) Sauerstoffanreicherung des Schwefelatoms

(2) Alkylieren des Stickstoffatoms und

(3) Umsetzen der $—CO_2R_4$-Gruppe zum Erhalt einer $R_3$-Gruppe, welche nicht $—CO_2R_4$ ist;

und gegebenenfalls die Bildung eines pharmazeutisch akzeptablen Salzes davon.

3. Verfahren nach Anspruch 2, worin $R_2$ Cyano ist, und n für Eins oder Zwei steht.

4. Verfahren nach Anspruch 1 oder 2 zur Herstellung einer Verbindung der Formel

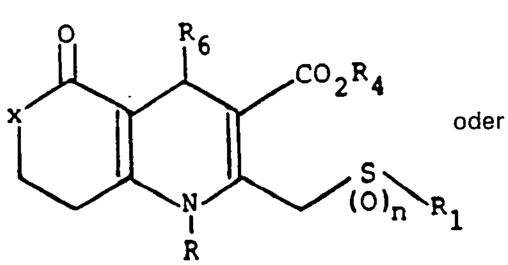

oder

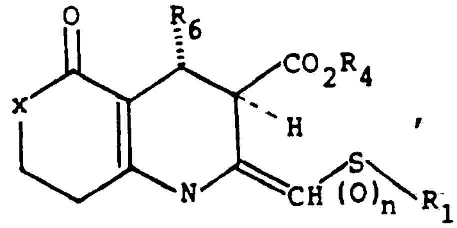

,

worin X für >$CH_2$ oder ein Sauerstoffatom steht.

5. Verfahren nach Anspruch 1 oder 2, worin R für Wasserstoff steht, $R_2$ Cyano ist, $R_3$ für —$CO_2R_4$ steht, $R_5$ Methyl ist, und m für Eins steht.

6. Verfahren nach Anspruch 2, worin R Wasserstoff steht, $R_2$ und $R_3$ unabhängig voneinander —$CO_2R_4$ sind, $R_5$ Methyl ist, und m Eins ist.

7. Verfahren nach Anspruch 4, 5 oder 6, wenn auf Anspruch 2 rückbezogen, worin n für Null steht.

8. Verfahren nach Anspruch 1 oder 2 zur Herstellung einer Verbindung mit einem der folgenden Namen:

3-Pyridincarboxysäure-5-cyano-1,2,3,4-tetrahydro-2-[(phenylthio)methylen]-6-methyl-4-[(2-trifluormethyl)-phenyl]-ethylester;

3-Chinolincarbonsäure-1,2,3,4,5,6,7,8-octahydro-5-oxo-2-[(phenylthio)-methylen]-4-[(2-trifluormethyl)-phenyl]-ethylester;

Ethyl-5-cyano-1,4-dihydro-6-methyl-2-[(phenylsulfinyl)-methyl)-4-[2-(trifluormethyl)-phényl]-3-pyridincarboxylat;

Ethyl-5-cyano-1,4-dihydro-6-methyl-2-[[(2-methylphenyl)sulfinyl]-methyl]-4-[2-(trifluormethyl)-phenyl]-3-pyridincarboxylat;

Ethyl-5-cyano-1,4-dihydro-6-methyl-4-(3-nitrophenyl)-2-[(phenylsulfinyl)-methyl]-3-pyridincarboxylat;

Ethyl-5-cyano-1,4-dihydro-6-methyl-2-[[(4-methoxyphenyl)sulfinyl]-methyl]-4-[2-(trifluormethyl)-phenyl]-3-pyridincarboxylat;

Ethyl-5-cyano-1,4-dihydro-6-methyl-2-[(phenylsulfinyl)-methyl]-4-[2-(chlorphenyl)-3-pyridincarboxylat;

Ethyl-5-cyano-1,4-dihydro-6-methyl-2-[(4-pyridinylsulfinyl)-methyl]-4-[2-(trifluormethyl)-phenyl]-3-pyridincarboxylat;

Isethyl-2-[[(2-chlorphenyl)-sulfinyl]-methyl]-5-cyano-1,4-dihydro-6-methyl-4-[(2-trifluormethyl)-phenyl]-3-pyridincarboxylat;

5-cyano-2-[[[2-(dimethylamino)-ethyl]-sulfinyl]-methyl]-1,4-dihydro-6-methyl-4-[2-(trifluormethyl)-phenyl]-3-pyridincarbonsäure-ethylester;

Ethyl-4-(2-chlorphenyl)-5-cyano-1,4-dihydro-6-methyl-2-[(4-pyridinylsulfinyl)-methyl]-3-pyridincarboxylat;

Ethyl-5-cyano-4-(2,3-dichlorphenyl)-1,4-dihydro-6-methyl-2-(4-pyridinylsulfinyl)-methyl-3-pyridincarboxylat;

5-Cyano-1,4-dihydro-6-methyl-2-[(2-pyrimidinylsulfinyl)-methyl]-4-[(2-(trifluormethyl)-phenyl]-3-pyridincarbonsäure-ethylester;

5-Cyano-1,4-dihydro-6-methyl-2-[[(1-methyl-1*H*-imidazol-2-yl)sulfinyl]-methyl]-4-[2-(trifluormethyl)-phenyl]-3-pyridincarbonsäure-ethylester;

Ethyl-5-cyano-1,4-dihydro-6-methyl-2-[(phenylsulfinyl)-methyl]-4-phenyl-3-pyridincarboxylat;

3,5-Pyridin-dicarbonsäure-2-[[(2-chlorphenyl)-sulfinyl]-methylen]-6-methyl-1,2,3,4-tetrahydro-4-[(2-trifluormethyl)-phenyl]-3-ethyl-5-methylester;

3-Pyridincarbonsäure-5-cyano-4-cyclohexyl-1,4-dihydro-6-methyl-2-[(phenylsulfinyl)-methyl]-ethylester (±)-;

3-Pyridincarbonsäure-5-cyano-4-[2-(difluormethoxy)-phenyl]-1,4-dihydro-6-methyl-2-[(phenylsulfinyl)-methyl]-ethylester;

3-Pyridincarbonsäure-5-cyano-1,4-dihydro-6-methyl-2-[(phenylsulfinyl)-methyl]-4-[2-(trifluormethyl)-phenyl]-methylester; (53,47 Mischung von Isomeren);

3-Pyridin-carbonsäure-5-cyano-1,4-dihydro-6-methyl-2-[(phenylsulfinyl)-methyl]-4-[2-(trifluormethyl)-phenyl]-1-methylethylester;

3-Pyridin-carbonsäure-5-cyano-1,4-dihydro-6-methyl-2-[(phenylsulfinyl)-methyl]-4-[2-(trifluormethyl)-phenyl]-2-hydroxyethylester;

3-Pyridincarbonsäure-5-cyano-1,4-dihydro-6-methyl-4-(2-phenyl-1*H*-imidazol-4-yl)-2-[(phenylsulfinyl)-methyl]-ethylester;

3-Pyridincarbonsäure-5-cyano-1,4-dihydro-6-methyl-2-[(2-pyridinylsulfinyl)-methyl]-4-[2-(trifluormethyl)-phenyl]-ethylester (±)-;

3-Pyridincarbonsäure-5-cyano-2-[[(2-fluorphenyl)-sulfinyl]-methyl]-1,4-dihydro-6-methyl-4-[2-(trifluormethyl)-phenyl]-ethylester;

3-Pyridincarbonsäure-2-[[[4-(acetylamino)-phenyl]-sulfinyl]-methyl]-5-cyano-1,4-dihydro-6-methyl-4-[2-(trifluormethyl)-phenyl]-ethylester;

3-Pyridincarbonsäure-2-[[(4-chlorphenyl)-sulfinyl]-methyl]-5-cyano-1,4-dihydro-6-methyl-4-[2-(trifluormethyl)-phényl]-ethylester (±)-;

3-Pyridincarbonsäure-5-cyano-2-[[(3,4-dichlorphenyl)-sulfinyl]-methyl]-1,4-dihydro-6-methyl-4-[2-(trifluormethyl)-phenyl]-ethylester;

3-Pyridincarbonsäure-5-cyano-1,4-dihydro-6-methyl-2-[(phenylsulfinyl)-methyl]-4-[2-(trifluormethyl)-phenyl]-;

3-Chinolincarbonsäure-1,4,5,6,7,8-hexahydro-1-methyl-5-oxo-2-[(phenylsulfinyl)-methyl]-4-[2-(trifluormethyl)-phenyl]-ethylester;

3-Chinolincarbonsäure-1,4,5,6,7,8-hexahydro-5-oxo-2-[(phenylsulfinyl)-methyl]-4-[2-(trifluormethyl)-phenyl]-ethylester (±)-;

Ethyl-3-cyano-1,4-dihydro-6-methyl-2-[(phenylsulfinyl)-methyl]-4-[2-(trifluormethyl)-phenyl]-5-pyridincarboxylat;

3-Pyridincarbonsäure-5-cyano-1-[2-(diethylamino)-ethyl]-1,4-dihydro-6-methyl-2-[(phenylsulfinyl)-methyl]-4-[2-(trifluormethyl)-phenyl]-ethylester;

3-Pyridincarbonsäure-5-cyano-1,4-dihydro-[[(3-methoxy-3-oxopropyl]-sulfinyl]-methyl]-6-methyl-4-[2-(trifluormethyl)-phenyl]-ethylester;

3-Pyridincarbonsäure-5-cyano-2-[[(2-ethoxy-2-oxo-ethyl)-sulfinyl]-methyl]-1,4-dihydro-6-methyl-4-[2-(trifluormethyl)-phenyl]-ethylester;

[2,4'-Bipyridin]-3'-carbonsäure-5'-cyano-1',4'-dihydro-6'-methyl-2'-[(phenylsulfinyl)-methyl]-ethylester;

Ethyl-5-cyano-1,4-dihydro-6-methyl-2-[(phenylsulfonyl)-methyl]-4-[2-(trifluormethyl)-phenyl]-3-pyridincarboxylat;

Ethyl-5-cyano-1,4-dihydro-6-methyl-2-[[(4-methoxyphenyl)-sulfonyl]-methyl]-4-[2-(trifluormethyl)-phenyl]-3-pyridincarboxylat;

Ethyl-5-cyano-1,4-dihydro-6-methyl-2-[[(2-methylphenyl)-sulfonyl]-methyl]-4-[2-(trifluormethyl)-phenyl]-3-pyridincarboxylat;

Ethyl-2-[[(2-chlorphenyl)-sulfonyl)-methyl]-5-cyano-1,4-dihydro-6-methyl-4-[2-(trifluormethyl) -phenyl]-3-pyridincarboxylat;

Ethyl-5-cyano-1,4-dihydro-6-methyl-2-[(4-pyridinylsulfonyl)-methyl]-4-[2-(trifluormethyl)-phényl]-3-pyridincarboxylat;

5-Cyano-1,4-dihydro-6-methyl-2-[(2-pyridinylsulfonyl)-methyl]-4-[2-(trifluormethyl)-phenyl]-3-pyridincarbonsäure-ethylester-N-oxid;

Ethyl-5-cyano-1,4-dihydro-6-methyl-2-[(phenylsulfonyl)-methyl]-4-phényl-3-pyridinecarboxylat;

[2,4'-Bipyridin]-3'-carbonsäure-5'-cyano-1',4'-dihydro-6'-methyl-2'-[(phenylsulfonyl)-methyl]-ethylester-1-oxid;

[3,4'-Bipyridin]-3'-carbonsäure-5'-cyano-1',4'-dihydro-6'-methyl-2'-[(phenylsulfonyl)-methyl]-ethylester;

3-Pyridin-carbonsäure-2-[[(4-aminophenyl)-sulfonyl]-methyl]-5-cyano-1,4'-dihydro-6-methyl-4-[2-(trifluormethyl)-phenyl]-ethylester;

[2,4'-Bipyridin]-3'-carbonsäure-5'-cyano-1',4'-dihydro-6'-methyl-2'-[(phenylsulfonyl)-methyl]-ethylester;

3-Pyridincarbonsäure-5-cyano-4-cyclohexyl-6-methyl-1,4-dihydro-2-[(phenylsulfonyl)-methyl]-ethylester;

3-Pyridincarbonsäure-5-cyano-1,4-dihydro-6-methyl-2-[(phenylsulfonyl)-methyl]-4-[2-(trifluormethyl)-phenyl]-1-methylethylester;

3-Pyridin-carbonsäure-5-cyano-1,4-dihydro-6-methyl-2-[(phenylsulfonyl)-methyl]-4-[2-(trifluormethyl)-phenyl]-2-propenylester;

3-Pyridincarbonsäure-5-cyano-2[[[4-(1,1-dimethylethyl)-2-methylphenyl]-sulfonyl]-methyl]-1,4-dihydro-6-methyl-4-[2-(trifluormethyl)-phenyl]-ethylester;

3-Pyridincarbonsäure-5-cyano-2-[[(2-fluorphenyl)-sulfonyl]-methyl]-1,4-dihydro-6-methyl-4-[2-(trifluormethyl)-phenyl]-ethylester;

3-Pyridincarbonsäure-2-[[(4-chlorphenyl)-sulfonyl]-methyl]-5-cyano-1,4-dihydro-6-methyl-4-[2-(trifluormethyl)-phenyl]-ethylester;

3-Pyridincarbonsäure-5-cyano-1,4-dihydro-6-methyl-4-(3-nitrophenyl)-2-[(phenylsulfonyl)-methyl]-ethylester;

3-Pyridincarbonsäure-5-cyano-1,4-dihydro-6-methyl-2-[(phenylsulfonyl)-methyl]-4-[2-(trifluormethyl)-phenyl]-2,3-dihydroxypropylester;

3-Pyridincarbonsäure-5-cyano-1,4-dihydro--2-[[(3-methoxy-3-oxopropyl)-sulfonyl]-methyl]-6-methyl-4-[2-(trifluormethyl)-phenyl]-ethylester;

3-Chinolincarbonsäure-1,4,5,6,7,8-hexahydro-1-methyl-5-oxo-2-[(phenylsulfonyl)-methyl]-4-[2-(trifluormethyl)-phenyl]-ethylester;

3-Chinolincarbonsäure-1,4,5,6,7,8-hexahydro-5-oxo-2-[(phenylsulfonyl)-methyl]-4-[2-(trifluormethyl)-phenyl]-ethylester und

3-Chinolincarbonsäure-1,4,5,6,7,8-hexahydro-1-methyl-5-oxo-2-[(phenylthio)methyl]-4-[2-(trifluormethyl)-phenyl]-ethylester.

9. Verfahren zur Herstellung einer pharmazeutischen Zusammensetzung, welches Verfahren das Zusammenfügen einer wirksamen Menge einer mittels eines wie in irgendeinem vorhergehenden Anspruch beanspruchten Verfahrens hergestellten Verbindung mit einem pharmazeutisch akzeptablen Träger oder Verdünnungsmittel umfaßt.

10. Verbindung, welche mittels eines in irgendeinem der Ansprüche 1 bis 8 beanspruchten Verfahrens hergestellt wird, oder eine pharmazeutische Zusammensetzung, welche mittels eines wie im Anspruch 9 beanspruchten Verfahrens hergestellt wird, zur Verwendung in einer Methode zur Behandlung eines an einer kardiovaskulären Erkrankung leidenden Säugers.

**Revendications pour les Etats contractants: BE CH DE FR GB IT LI LU NL SE**

1. Un dérivé répondant à la formule générale suivante (I)

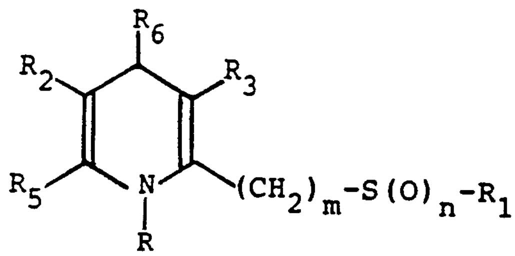

(I)

et un de sels pharmaceutiquement acceptables; dans laquelle

R représente

(1) un atome d'hydrogène;

(2) un radical alkyle inférieur renfermant du un à quatre atomes de carbone éventuellement substitué par un groupe amino répondant à la formule $NR_7R_8$ dans laquelle $R_7$ et $R_8$ représentent indépendamment un atome d'hydrogène ou un radical alkyle inférieur renfermant de un à quatre atomes de carbone ou $R_7$ et $R_8$, lorsqu'ils sont pris ensemble en même temps que l'atome d'azote auquel ils sont attachés, forment un cycle à cinq ou six éléments renfermant quatre on cinq groupes $CH_2$; ou

(3) un radical arylalkylène dans lequel la partie aryle est le radical phényle éventuellement substitué par un ou plusieurs d'entre (i) un groupe halo, (ii) le radical trifluorométhyle, (iii) un groupe nitro, (iv) un groupe amino, (v) mono- ou di-alkylamino dans lequel l'alkyle ou chacun des alkyles représente indépendamment un radical alkyle inférieur renfermant de un à six atomes de carbone ou, dans le cas d'un dialkylamino, dans lequel les deux radicaux alkyle, lorsqu'on les prend ensemble, représentent un alkylène renfermant de un à six atomes de carbone, (vi) un groupe cyano, (vii) un radical alcoxy inférieur renfermant de un à quatre atomes de carbone, (viii) un radical thioalkyle inférieur renferment de un à quatre atomes de carbone; et dans lequel la partie alkylène renferme de un à quatre atomes de carbone;

$R_2$ représente un groupe cyano;

$R_3$ représente un atome d'hydrogène, un radical alkyle inférieur renfermant de un à quatre atomes de carbone, un groupe cyano, nitro, $CO_2R_4$ dans lequel $R_4$ représente un atome d'hydrogène ou un radical alkyle inférieur renfermant de un à quatre atomes de carbone, éventuellement substitué par un groupe amino, mono- ou di-alkylamino dans lequel le radical alkyle ou chacun des radicaux alkyle représentent indépendamment un radical alkyle inférieur renfermant de un à six atomes de carbone ou, dans le cas d'un dialkylamino, dans lequel les deux alkyles, lorsqu'ils sont pris ensemble, représentent un radical alkylène renfermant de un à six atomes de carbone;

$R_1$ représente

(1) un radical cycloalkyle renfermant de quatre à sept atomes de carbone;

(2) le radical phényle éventuellement substitué par un ou plusieurs d'entre (i) radical alkyle renfermant de un à quatre atomes de carbone, (ii) un groupe halo, (iii) le radical trifluorométhyle, (iv) un groupe nitro, (v) un groupe amino, et (vi) un groupe mono- ou di-alkylamino dans lequel l'alkyle ou chacun des alkyles représente indépendamment un radical alkyle inférieur renfermant de un à six atomes de carbone ou, dans le cas d'un dialkylamino, dans lequel les deux alkyles, lorsqu'on les prend ensemble, représentent un radical alkylène renfermant de un à six atomes de carbone; ou

(3) un radical thiényle, furanyle, pyryle, pyridinyle, quinolyle, isoquinolyle, pyrimidyle, pyridazinyle, quinazolyle, quinoxalyle, benzothiényle, pyrazolyle, imidazolyle, oxazolyle, isoxazolyle, thiazolyle, triazolyle, pyrazinyle, oxazinyle, thiazinyle, indolizinyle, indolyle, benzofuranyle, indazolyle, benzothiényle, benzimidazolyle, benzoxazolyle, benzioxazolyle, benzthiazolyle, benztriazolyle, benzoxadiazolyle, cinnolinyle, phtalaxinyle, naphtyridinyle ou benzothiazinyle;

$R_5$ et $R_6$ qui pevent être identiques ou différents représentent chacun

(1) un radical alkyle inférieur renfermant de un à quatre atomes de carbone, éventuellement substitué par (a) un groupe amino répondant à la formule $NR_7R_8$ dans laquelle $R_7$ et $R_8$ représentent indépendamment un atome d'hydrogène ou un radical alkyle inférieur ou $R_7$ et $R_8$, lorsqu'ils sont pris ensemble en même temps que l'atome d'azote auquel ils sont attachés, forment un cycle à cinq ou six éléments renfermant quatre ou cinq atomes de carbone, (b) $—OR_9$ dans lequel $R_9$ représente un atome d'hydrogène ou un radical alkyle inférieur renfermant de un à quatre atomes de carbone, ou (c) $—CO_2R_4$ dans lequel $R_4$ et tel que défini ci-dessus;

(2) un radical cycloalkyle renfermant de quatre à sept atomes de carbone;

(3) un radical phényle éventuellement substitué par un ou plusieurs d'entre (i) radical alkyle renfermant de un à quatre atomes de carbone, (ii) un groupe halo, (iii) le radical trifluorométhyle, (iv) un groupe nitro, (v) amino, (vi) mono- ou di-alkylamino dans lequel le radical alkyle ou chacun des radicaux alkyle représente indépendamment un radical alkyle inférieur renfermant de un à six atomes de carbone ou, dans le cas d'un groupe dialkylamino, dans lequel les deux groupes alkyle, lorsqu'ils sont pris ensemble, représentent un groupe alkylène renfermant de un à six atomes de carbone; (vii) un groupe cyano, (viii) un radical alcoxy inférieur renfermant de un à quartre atomes de carbone, et (ix) un radical thioalkyle inférieur renfermant de un à quatre atomes de carbone;

(4) un radical alrylalkylène dans lequel la partie aryle est un radical phényle éventuellement substituté par un ou plusieurs d'entre (i) radical alkyle renferment de un à quatre atomes de carbone, (ii) un groupe halo, (iii) le radical trifluorométhyle, (iv) un groupe nitro, (v) amino, (vi) mono- ou di-alkylamino dans lequel le radical alkyle ou chacun des radicaux alkyle représente indépendamment un radical alkyle inférieur renfermant de un à six atomes de carbone ou, dans le cas d'un groupe dialkylamino, dans lequel les deux groupes alkyle, lorsqu'ils sont pris ensemble, représentent un groupe alkylène renfermant de un à six atomes de carbone; (vii) un groupe cyano, (viii) un radical alcoxy inférieur renfermant de un à quatre atomes de carbone, et (ix) un radical thioalkyle inférieur renfermant de un à quatre atomes de carbone; et la partie alkylène renferme de un à quatre atomes de carbone; ou

(5) un radical thiényle, furanyle, pyryle, pyridinyle, quinolyle, isoquinolyle, pyrimidyle, pyridazinyle, quinazolyle, quinoxalyle, benzothiényle, pyrazolyle, imidazolyle, oxozolyle, isoxazolyle, thiazolyle, triazolyle, pyrazinyle, oxazinyle, thiazinyle, indolizinyle, indolyle, benzofuranyle, indazolyle, benzothiényle, benzimidazolyle, benzoxazolyle, benzioxazolyle, benzthiazolyle, benztriazolyle, benzoxadiazolyle, cinnolinyle, phtalaxinyle, naphtyridinyle ou benzothiazinyle;

et dans laquelle $R_2$ et $R_5$ peuvent également être pris ensemble pour former un cycle à cinq ou six éléments renferment comme élément du cycle ou groupe

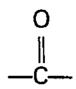 ou 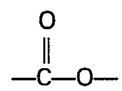

n est égal à un ou deux; m est un nombre entier compris entre un et six.

2. Un dérivé de formule (II)

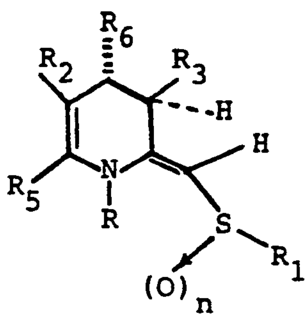

(II)

et un de sels pharmaceutiquement acceptables; dans laquelle

R représente

(1) un atome d'hydrogène;

(2) un radical alkyle inférieur renfermant du un à quatre atomes de carbone éventuellement substitué par un groupe amino répondant à la formule $NR_7R_8$ dans laquelle $R_7$ et $R_8$ représentent indépendamment un atome d'hydrogène ou un radical alkyle inférieur renfermant de un à quatre atomes de carbone ou $R_7$ et $R_8$, lorsqu'ils sont pris ensemble en même temps que l'atome d'azote auquel ils sont attachés, forment un cycle à cinq ou six éléments renfermant quatre ou cinq groupes $CH_2$; ou

(3) un radical arylalkylène dans lequel la partie aryle est le radical phényle éventuellement substitué par un ou plusieurs d'entre (i) un groupe halo, (ii) le radical trifluorométhyle, (iii) un groupe nitro, (iv) un groupe amino, (v) mono- ou di-alkylamino dans lequel l'alkyle ou chacun des alkyles représente indépendamment un radical alkyle inférieur renfermant de un à six atomes de carbone ou, dans le cas d'un dialkylamino, dans lequel les deux radicaux alkyle, lorsqu'on les prend ensemble, représentent un alkylène renfermant de un à six atomes de carbone, (vi) un groupe cyano, (vii) un radical alcoxy inférieur renfermant

de un à quatre atomes de carbone, (viii) un radical alkyle inférieur renferment de un à quatre atomes de carbone; (ix) un radical thioalkyle inférieur renfermant de un à quatre atomes de carbone; et dans lequel la partie alkylène renferme de un à quatre atomes de carbone;

$R_2$ et $R_3$, qui peuvent être identiques ou différents, représentent chacun un atome d'hydrogène, un radical alkyle inférieur renfermant de un à quatre atomes de carbone, un groupe cyano, nitro, $CO_2R_4$ dans lequel $R_4$ représente un atome d'hydrogène ou un radical alkyle inférieur renfermant de un à quatre atomes de carbone, éventuellement substitué par un groupe amino, mono- ou di-alkylamino dans lequel le radical alkyle ou chacun des radicaux alkyle représente indépendamment un radical alkyle inférieur renfermant de un à six atomes de carbone ou, dans le cas d'un dialkylamino, dans lequel les deux radicaux alkyle, lorsqu'ils sont pris ensemble, représentent un radical alkylène renfermant de un à six atomes de carbone;

$R_1$, $R_5$ et $R_6$, qui peuvent être indentiques ou différents, représentent chacun

(1) un radical alkyle inférieur renfermant de un à quatre atomes de carbone, éventuellement substitué par (a) un groupe amino répondant à la formule $NR_7R_8$ dans laquelle $R_7$ et $R_8$ représentent indépendamment un atome d'hydrogène ou un radical alkyle inférieur ou bien $R_7$ et $R_8$, lorsqu'ils sont pris ensemble en même temps que l'atome d'azote auquel ils sont attachés, forment un cycle à cinq ou six éléments renfermant quatre ou cinq atomes de carbone, (b) $—OR_9$ dans lequel $R_9$ représente un atome d'hydrogène ou un radical alkyle inférieur renfermant de un à quatre atomes de carbone, ou (c) $—CO_2R_4$ dans lequel $R_4$ et tel que défini ci-dessus;

(2) un radical cycloalkyle renfermant de quatre à sept atomes de carbone;

(3) un radical phényle éventuellement substitué par un ou plusieurs d'entre (i) radical alkyle renfermant de un à quatre atomes de carbone, (ii) un groupe halo, (iii) le radical trifluorométhyle, (iv) un groupe nitro, (v) amino, (vi) mono- ou di-alkylamino dans lequel le radical alkyle ou chacun des radicaux alkyle représente indépendamment un radical alkyle inférieur renfermant de un à six atomes de carbone ou, dans le cas d'un groupe dialkylamino, dans lequel les deux groupes alkyle, lorsqu'ils sont pris ensemble, représentent un groupe alkylène renfermant de un à six atomes de carbone; (vii) un groupe cyano, (viii) un radical alcoxy inférieur renfermant de un à quartre atomes de carbone, et (ix) un radical thioalkyle inférieur renfermant de un à quatre atomes de carbone;

(4) un radical alrylalkylène dans lequel la partie aryle est un radical phényle éventuellement substitué par un ou plusieurs d'entre (i) radical alkyle renfermant de un à quatre atomes de carbone, (ii) un groupe halo, (iii) le radical trifluorométhyle, (iv) un groupe nitro, (v) amino, (vi) mono- ou di-alkylamino dans lequel le radical alkyle ou chacun des radicaux alkyle représente indépendamment un radical alkyle inférieur renfermant de un à six atomes de carbone ou, dans le cas d'un groupe dialkylamino, dans lequel les deux groupes alkyle, lorsqu'ils sont pris ensemble, représentent un groupe alkylène renfermant de un à six atomes de carbone; (vii) un groupe cyano, (viii) un radical alcoxy inférieur renfermant de un à quatre atomes de carbone, et (ix) un radical thioalkyle inférieur renfermant de un à quatre atomes de carbone; et la partie alkylène renferme de un à quatre atomes de carbone; ou

(5) un radical thiényle, furanyle, pyryle, pyridinyle, quinolyle, isoquinolyle, pyrimidyle, pyridazinyle, quinazolyle, quinoxalyle, benzothiényle, pyrazolyle, imidazolyle, oxazolyle, isoxazolyle, thiazolyle, triazolyle, pyrazinyle, oxazinyle, thiazinyle, indolizinyle, indolyle, benzofuranyle, indazolyle, benzothiényle, benzimidazolyle, benzoxazolyle, benzioxazolyle, benzthiazolyle, benztriazolyle, benzoxadiazolyle, cinnolinyle, phtalaxinyle, naphtyridinyle ou benzothiazinyle;

et dans laquelle $R_2$ et $R_5$ peuvent également être pris ensemble pour former un cycle à cinq ou six éléments renfermant comme élément du cycle, un groupe

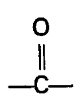 ou 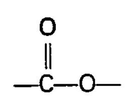

n est égal à zéro ou répresenté un nombre entier égal à un ou deux.

3. Un dérivé selon la revendication 2, dans lequel $R_2$ représente un groupe cyano, et n est égal à un ou deux.

4. Un dérivé selon la revendication 1 ou 2, et répondant à la formule

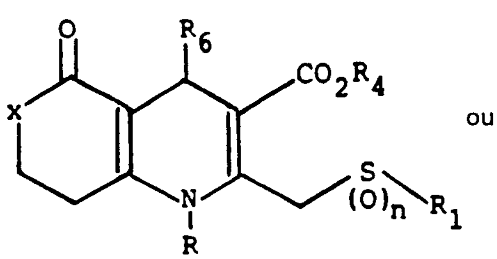

ou

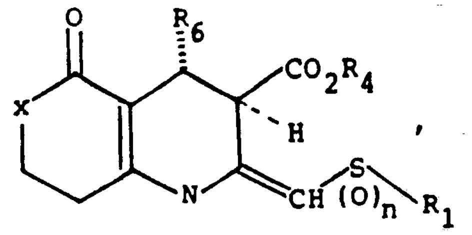

,

dans laquelle X représente $>CH_2$ ou un atome d'oxygène.

5. Un dérivé selon la revendication 1 ou 2, dans lequel R représente un atome d'hydrogène, $R_2$ représente un groupe cyano, $R_3$ représente $—CO_2R_4$, $R_5$ représente le radical méthyle et me est égal à un.

6. Un dérivé selon la revendication 2, dans lequel R représente un atome d'hydrogène, $R_2$ et $R_3$

représentent indépendamment —$CO_2R_4$, $R_5$ représente le radical méthyle et m est égal à un.

7. Un dérivé selon la revendication 4, 5 ou 6 prises en relation avec la revendication 2, dans lequel n est égal à zéro.

8. Un dérivé selon la revendication 1 ou 2, désigné sous l'un des noms suivants:

ester éthylique de l'acide 3-pyridinecarboxylique 5-cyano-1,2,3,4-tétrahydro-2-[(phénylthio)-méthylène]-6-méthyl-4-[(2-trifluorométhyl)phényl]-;

ester éthylique d'acide 3-quinoléinecarboxylique, 1,2,3,4,5,6,7,8-octahydo-5-oxo-2-[(phénylthio)-méthylène]-4-[(2-trifluorométhyl)phényl]-;

5-cyano-1,4-dihydro-6-méthyl-2-[(phénylsulfinyl)méthyl)-4-[2-(trifluorométhyl)phényl]-3-pyrindine-carboxylate d'éthyle;

5-cyano-1,4-dihydro-6-méthyl-2-[[(2-méthylphényl)sulfinyl)-méthyl]-4-[2-(trifluorométhyl)phényl]-3-pyridinecarboxylate d'éthyle;

5-cyano-1,4-dihydro-6-méthyl-4-(3-nitrophényl)-2-[(phénylsulfinyl)méthyl]-3-pyridinecarboxylate d'éthyle;

5-cyano-1,4-dihydro-6-méthyl-2-[[(4-méthoxyphényl)sulfinyl]méthyl]-4-[2-(trifluorométhyl)phényl]-3-pyridinecarboxylate d'éthyle;

5-cyano-1,4-dihydro-6-méthyl-2-[(phénylsulfinyl)méthyl]-4-[2-(chlorophényl)-3-pyridinecarboxylate d'éthyle;

5-cyano-1,4-dihydro-6-méthyl-2-[(4-pyridinylsulfinyl)-méthyl]-4-[2-(trifluorométhyl)phényl]-3-pyridinecarboxylate d'éthyle;

2-[[(2-chlorophényl)sulfinyl]méthyl]-5-cyano-1,4-dihydro-6-méthyl-4-[(2-trifluorométhyl)phényl]-3-pyridine-carboxylate d'éthyle;

Ester éthylique d'acide 5-cyano-2-[[[2-(diméthylamino)éthyl]sulfinyl]méthyl]-1,4-dihydro-6-méthyl-4-[2-(trifluorométhyl)phényl]-3-pyridinecarboxylique;

4-(2-chlorophényl)-5-cyano-1,4-dihydro-6-méthyl-2-[(4-pyridinylsulfinyl)méthyl]-3-pyridinecarboxylate d'éthyle;

5-cyano-4-(2,3-dichlorophényl)-1,4-dihydro-6-méthyl-2-[(4-pyridinylsulfinyl)méthyl]-3-pyridine-carboxylate d'éthyle;

Ester éthylique de l'acide 5-cyano-1,4-dihydro-6-méthyl-2-[(2-pyrimidinylsulfinyl)méthyl]-4-[2-(tri-fluorométhyl)phényl]-3-pyridinecarboxylique;

Ester éthylique de l'acide 5-cyano-1,4-dihydro-6-méthyl-2-[[(1-méthyl-1*H*-imidazol-2-yl)sulfinyl]-méthyl]-4-[2-(trifluorométhyl)phényl]-3-pyridinecarboxylique;

5-cyano-1,4-dihydro-6-méthyl-2-[(phénylsulfinyl)méthyl]-4-phényl-3-pyridinecarboxylate d'éthyle;

2-[[(2-chlorophenyl)sulfinyl]méthylène)-6-méthyl-1,2,3,4-tétrahydro-4-[(2-trifluorométhyl)phényl)-3-éthyl-5-méthyl ester d'acide 3,5-pyridinedicarboxylique;

5-cyano-4-cyclohexyl-1,4-dihydro-6-méthyl-2-[(phénylsulfinyl)méthyl)-ester éthylique d'acide 3-pyridinecarboxylique (±)-;

5-cyano-4-[2-(difluorométhoxy)phényl]-1,4-dihydro-6-méthyl-2-[(phénylsulfinyl)méthyl]-ester éthylique d'acide 3-pyridinecarboxylique;

5-cyano-1,4-dihydro-6-méthyl-2-[(phénylsulfinyl)méthyl]-4-[2-(trifluorométhyl)phényl]-méthyl ester d'acide 3-pyridinecarboxylique; (mélange d'isomères à 53/47%);

5-cyano-1,4-dihydro-6-méthyl-2-[(phénylsulfinyl)méthyl]-4-[2-(trifluorométhyl)phényl]-1-méthyléthyl ester d'acide 3-pyridinecarboxylique;

2-hydroxyéthyl ester d'acide 3-pyridinecarboxylique, 5-cyano-1,4-dihydro-6-méthyl-2-[(phénylsulfinyl)méthyl]-4-[2-(trifluorométhyl)phényl]-;

Ester éthylique d'acide 3-pyridinecarboxylique, 5-cyano-1,4-dihydro-6-méthyl-4-(2-phényl-1*H*-imidazol-4-yl)-2-[(phénylsulfinyl)méthyl]-(diastéréoisomères);

Ester éthylique d'acide 3-pyridinecarboxylique, 5-cyano-1,4-dihydro-6-méthyl-2-[(2-pyridinylsulfinyl)-méthyl]-4-[2-(trifluorométhyl)phényl]- (±)-;

Ester éthylique d'acide 3-pyridinecarboxylique, 5-cyano-2-[[(2-fluorophényl)sulfinyl]méthyl]-1,4-dihydro-6-méthyl-4-[2-(trifluorométhyl)phényl]-;

Ester éthylique d'acide 3-pyridinecarboxylique, 2-[[[4-(acétylamino)phényl]sulfinyl]methyl]-5-cyano-1,4-dihydro-6-méthyl-4-[2-(trifluorométhyl)phényl]-;

Ester éthylique d'acide 3-pyridinecarboxylique, 2-[[(4-chlorophényl)sulfinyl]méthyl]-5-cyano-1,4-dihydro-6-méthyl-4-[2-(trifluorométhyl)phényl]- (±)-;

Ester éthylique d'acide 3-pyridinecarboxylique, 5-cyano-2-[[(3,4-dichlorophényl)sulfinyl]méthyl]-1,4-dihydro-6-méthyl-4-[2-(trifluorométhyl)phényl]-;

Acide 3-pyridinecarboxylique, 5-cyano-1,4-dihydro-6-méthyl-2-[(phénylsulfinyl)méthyl]-4-[2-(trifluoro-méthyl)phényl]-;

Ester éthylique d'acide 3-quinoléinecarboxylique, 1,4,5,6,7,8-hexahydro-1-méthyl-5-oxo-2-[(phénylsulfinyl)méthyl]-4-[2-(trifluorométhyl)phényl]-;

Ester éthylique d'acide 3-quinoléinecarboxylique, 1,4,5,6,7,8-hexahydro-5-oxo-2-[phénylsulfinyl)méthyl]-4-[2-(trifluorométhyl)phényl]-; (±)-;

3-cyano-1,4-dihydro-6-méthyl-2-[(phénylsulfinyl)méthyl]-4-[2-(trifluorométhyl)phényl]-5-pyridine-carboxylate d'éthyle;

Ester éthylique d'acide 3-pyridinecarboxylique, 5-cyano-1-[2-(diéthylamino)éthyl]-1,4-dihydro-6-méthyl-2-[(phénylsulfinyl)méthyl]-4-[2-(trifluorométhyl)phényl]-;

Ester éthylique d'acide 3-pyridinecarboxylique, 5-cyano-1,4-dihydro-[[(3-méthoxy-3-oxopropyl]-sulfinyl]méthyl]-6-méthyl-4-[2-(trifluorométhyl)phényl]-;

Ester éthylique d'acide 3-pyridinecarboxylique, 5-cyano-2-[[(2-éthoxy-2-oxoéthyl)sulfinyl]méthyl]-1,4-dihydro-6-méthyl-4-[2-(trifluorométhyl)phényl]-;

Ester éthylique d'acide [2,4'-bipyridine]-3'-carboxylique, 5'-cyano-1',4'-dihydro-6'-méthyl-2'-[(phénylsulfinyl)méthyl]-;

5-cyano-1,4-dihydro-6-méthyl-2-[(phénylsulfonyl)méthyl]-4-[2-(trifluorométhyl)phényl]-3-pyridine-carboxylate d'éthyle;

5-cyano-1,4-dihydro-6-méthyl-2-[[(4-méthoxyphényl)sulfonyl]méthyl]-4-[2-(trifluorométhyl)phényl]-3-pyridinecarboxylate d'éthyle;

5-cyano-1,4-dihydro-6-méthyl-2-[[(2-méthylphényl)sulfonyl]méthyl]-4-[2-(trifluorométhyl)phényl]-3-pyridinecarboxylate d'éthyle;

2-[[(2-chlorophényl)sulfonyl)méthyl]-5-cyano-1,4-dihydro-6-méthyl-4-[2-(trifluorométhyl)phényl]-3-pyridinecarboxylate d'éthyle;

5-cyano-1,4-dihydro-6-méthyl-2-[(4-pyridinylsulfonyl)méthyl]-4-[2-(trifluorométhyl)phényl]-3-pyridine-carboxylate d'éthyle;

N-oxyde ester éthylique d'acide 5-cyano-1,4-dihydro-6-méthyl-2-[(2-pyridinylsulfonyl)méthyl]-4-[2-(tri-fluorométhyl)phényl]-3-pyridinecarboxylique;

5-cyano-1,4-dihydro-6-méthyl-2-[(phénylsulfonyl)méthyl-4-phényl-3-pyridinecarboxylate d'éthyle;

1-oxyde de 5'-cyano-1',4'-dihydro-6'-méthyl-2'-[(phénylsulfonyl)méthyl]ester éthylique d'acide [2,4'-bipyridine]-3'-carboxylique;

5'-cyano-1',4'-dihydro-6'-méthyl-2'-[(phénylsulfonyl)méthyl]-ester éthylique d'acide [3,4'-bipyridinel-3'-carboxylique;

2-[[(4-aminophényl)sulfonyl]méthyl]-5-cyano-1,4-dihydro-6-méthyl-4-[2-(trifluorométhyl)phényl]ester éthylique d'acide 3-pyridinecarboxylique;

5'-cyano-1',4'-dihydro-6'-méthyl-2'-[(phénylsulfonyl)méthyl]ester éthylique d'acide [2,4'-bipyridine)-3'-carboxylique;

5'-cyano-4-cyclohexyl-6-méthyl-1,4-dihydro-2-[(phénylsulfonyl)méthyl]-, ester éthylique d'acide 3-pyridinecarboxylique;

5-cyano-1,4-dihydro-6-méthyl-2-[(phénylsulfonyl)méthyl]-4-[2-(trifluorométhyl)phényl]-, 1-méthyléthyl ester d'acide 3-pyridinecarboxylique;

5-cyano-1,4-dihydro-6-méthyl-2-[(phénylsulfonyl)méthyl]-4-[2-(trifluorométhyl)phényl]-, 2-propényl ester d'acide 3-pyridinecarboxylique;

5-cyano-2[[[4-(1,1-diméthyléthyl)-2-méthylphényl]sulfonyl]méthyl]-1,4-dihydro-6-méthyl-4-[2-(tri-fluorométhyl)phényl]-, ester éthylique d'acide 3-pyridinecarboxylique;

5-cyano-2[[(2-fluorophényl)sulfonyl]méthyl]-1,4-dihydro-6-méthyl-4-[2-(trifluorométhyl)phényl]-, ester éthylique d'acide 3-pyridinecarboxylique;

2-[[(4-chlorophényl)sulfonyl]méthyl]-5-cyano-1,4-dihydro-6-méthyl-4-[2-(trifluorométhyl)phényl]-, ester éthylique d'acide 3-pyridinecarboxylique;

5-cyano-1,4-dihydro-6-méthyl-4-(3-nitrophényl)-2-[(phénylsulfonyl)méthyl]-, ester éthylique d'acide 3-pyridinecarboxylique;

5-cyano-1,4-dihydro-6-méthyl-2-[(phénylsulfonyl)méthyl]-4-[2-(trifluorométhyl)phényl]-, 2,3-dihydroxypropyl ester d'acide 3-pyridinecarboxylique;

5-cyano-1,4-dihydro-2-[[(3-méthoxy-3-oxopropyl)sulfonyl]métyl]-6-méthyl-4-[2-(trifluorométhyl)-phényl]-, ester éthylique d'acide 3-pyridinecarboxylique;

1,4,5,6,7,8-hexahydro-1-méthyl-5-oxo-2-[(phénylsulfonyl)méthyl]-4-[2-(trifluorométhyl)phényl]-, ester éthylique d'acide 3-quinoléinecarboxylique;

1,4,5,6,7,8-hexahydro-5-oxo-2-[(phénylsulfonyl)-méthyl]-4-[2-(trifluorométhyl)phényl]-, ester éthylique d'acide 3-quinoléinecarboxylique; et

1,4,5,6,7,8-hexahydro-1-méthyl-5-oxo-2[(phénylthio)méthyl]-4-[2-(trifluorométhyl)phényl]-, ester éthylique d'acide 3-quinoléinecarboxylique.

9. Une composition pharmaceutique comprenant une quantité efficace d'un dérivé selon l'une quelconque des revendications précédentes en mélange avec un support ou diluant pharmaceutiquement acceptable.

10. Pour l'utilisation dans un procédé de traitement d'un mammifère souffrant de troubles cardio-vasculaires, un dérivé selon l'une quelconque des revendications 1 à 8 ou une composition pharmaceutique selon la revendication 9.

11. Un procédé de préparation d'un dérivé répondant à la formule (I) ou (II) telle que définie dans les revendications 1 et 2 respectivement, ce procédé comprenant les étapes suivantes:

on met en contact les dérivés

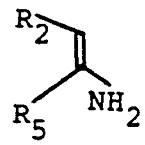

(III),

$R_6CHO$ (IV) et

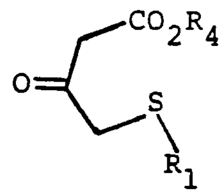

(V),

dans lesquels $R_1$, $R_2$, $R_4$, $R_5$ et $R_6$ sont tels que définis dans les revendications 1 ou 2 dans un solvant organique pour obtenir un dérivé de formule ($I_o$) ou ($II_o$)

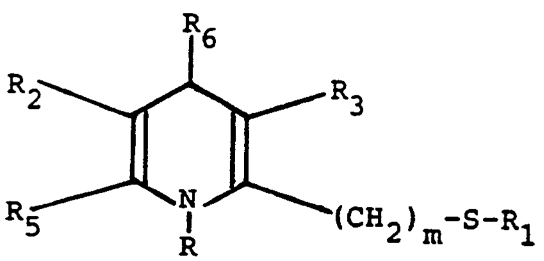

($I_o$)

ou

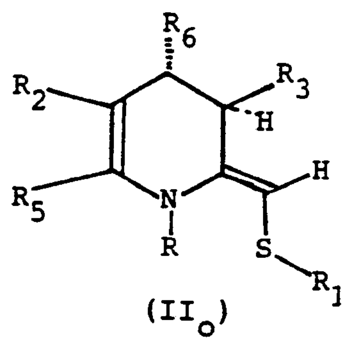

($II_o$)

et, si on le souhaite, on poursuit la réaction du produit par une ou plusieurs des étapes suivantes:

(1) on oxygène l'atome de soufre

(2) on procède à une alkylation de l'atome d'azote; et

(3) on fait réagir le groupe $—CO_2R_4$ pour obtenir un groupe $R_3$ différent de $—CO_2R_4$;

et éventuellement on en forme un sel pharmaceutiquement acceptable.

**Revendications pour l'Etat contractant: AT**

1. Un procédé de préparation d'un dérivé répondant à la formule générale suivante (I)

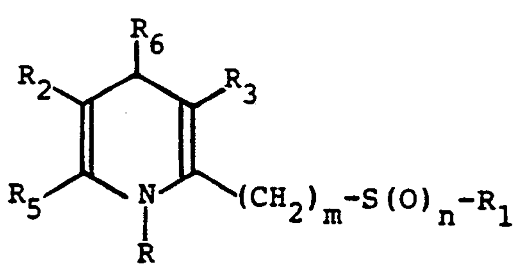

(I)

et un de sels pharmaceutiquement acceptables; dans laquelle

R représente

(1) un atome d'hydrogène;

(2) un radical alkyle inférieur renfermant du un à quatre atomes de carbone éventuellement substitué

par un groupe amino répondant à la formule $NR_7R_8$ dans laquelle $R_7$ et $R_8$ représentent indépendamment un atome d'hydrogène ou un radical alkyle inférieur renfermant de un à quatre atomes de carbone ou $R_7$ et $R_8$, lorsqu'ils sont pris ensemble en même temps que l'atome d'azote auquel ils sont attachés, forment un cycle à cinq ou six éléments renfermant quatre on cinq groupes $CH_2$; ou

(3) un radical arylalkylène dans lequel la partie aryle est le radical phényle éventuellement substitué par un ou plusieurs d'entre (i) un groupe halo, (ii) le radical trifluorométhyle, (iii) un groupe nitro, (iv) un groupe amino, (v) mono- ou di-alkylamino dans lequel l'alkyle ou chacun des alkyles représente indépendamment un radical alkyle inférieur renfermant de un à six atomes de carbone ou, dans le cas d'un dialkylamino, dans lequel les deux radicaux alkyle, lorsqu'on les prend ensemble, représentent un alkylène renfermant de un à six atomes de carbone, (vi) un groupe cyano, (vii) un radical alcoxy inférieur renfermant de un à quatre atomes de carbone, (viii) un radical thioalkyle inférieur renferment de un à quatre atomes de carbone; et dans lequel la partie alkylène renferme de un à quatre atomes de carbone;

$R_2$ représente un groupe cyano;

$R_3$ représente un atome d'hydrogène, un radical alkyle inférieur renfermant de un à quatre atomes de carbone, une groupe cyano, nitro, $CO_2R_4$ dans lequel $R_4$ représente un atome d'hydrogène ou un radical alkyle inférieur renfermant de un à quatre atomes de carbone, éventuellement substitué par un groupe amino, mono- ou di-alkylamino dans lequel le radical alkyle ou chacun des radicaux alkyle représentent indépendamment un radical alkyle inférieur renfermant de un à six atomes de carbone ou, dans le cas d'un dialkylamino, dans lequel les deux alkyles, lorsqu'ils sont pris ensemble, représentent un radical alkylène renfermant de un à six atomes de carbone;

$R_1$ représente

(1) un radical cycloalkyle renfermant de quatre à sept atomes de carbone;

(2) le radical phényle éventuellement substitué par un ou plusieurs d'entre (i) radical alkyle renfermant de un à quatre atomes de carbone, (ii) un groupe halo, (iii) le radical trifluorométhyle, (iv) un groupe nitro, (v) un groupe amino, et (vi) un groupe mono- ou di-alkylamino dans lequel l'alkyle ou chacun des alkyles représente indépendamment un radical alkyle inférieur renfermant de un à six atomes de carbone ou, dans le cas d'un dialkylamino, dans lequel les deux alkyles, lorsqu'on les prend ensemble, représentent un radical alkylène renfermant de un à six atomes de carbone; ou

(3) un radical thiényle, furanyle, pyryle, pyridinyle, quinolyle, isoquinolyle, pyrimidyle, pyridazinyle, quinazolyle, quinoxalyle, benzothiényle, pyrazolyle, imidazolyle, oxazolyle, isoxazolyle, thiazolyle, triazolyle, pyrazinyle, oxazinyle, thiazinyle, indolizinyle, indolyle, benzofuranyle, indazolyle, benzothiényle, benzimidazolyle, benzoxazolyle, benzioxazolyle, benzthiazolyle, benztriazolyle, benzoxadiazolyle, cinnolinyle, phtalaxinyle, naphtyridinyle ou benzothiazinyle;

$R_5$ et $R_6$ qui pevent être identiques ou différents représentent chacun

(1) un radical alkyle inférieur renfermant de un à quatre atomes de carbone, éventuellement substitué par (a) un groupe amino répondant à la formule $NR_7R_8$ dans laquelle $R_7$ et $R_8$ représentent indépendamment un atome d'hydrogène ou un radical alkyle inférieur ou $R_7$ et $R_8$, lorsqu'ils sont pris ensemble en même temps que l'atome d'azote auquel ils sont attachés, forment un cycle à cinq ou six éléments renfermant quatre ou cinq atomes de carbone, (b) $—OR_9$ dans lequel $R_9$ représente un atome d'hydrogène ou un radical alkyle inférieur renfermant de un à quatre atomes de carbone, ou (c) $—CO_2R_4$ dans lequel $R_4$ et tel que défini ci-dessus;

(2) un radical cycloalkyle renfermant de quatre à sept atomes de carbone;

(3) un radical phényle éventuellement substitué par un ou plusieurs d'entre (i) radical alkyle renfermant de un à quatre atomes de carbone, (ii) un groupe halo, (iii) le radical trifluorométhyle, (iv) un groupe nitro, (v) amino, (vi) mono- ou di-alkylamino dans lequel le radical alkyle on chacun des radicaux alkyle représente indépendamment un radical alkyle inférieur renfermant de un à six atomes de carbone ou, dans le cas d'un groupe dialkylamino, dans lequel les deux groupes alkyle, lorsqu'ils sont pris ensemble, représentent un groupe alkylène renfermant de un à six atomes de carbone; (vii) un groupe cyano, (viii) un radical alcoxy inférieur renfermant de un à quartre atomes de carbone, et (ix) un radical thioalkyle inférieur renfermant de un à quatre atomes de carbone;

(4) un radical alrylalkylène dans lequel la partie aryle est un radical phényle éventuellement substituté par un ou plusieurs d'entre (i) radical alkyle renferment de un à quatre atomes de carbone, (ii) un groupe halo, (iii) le radical trifluorométhyle, (iv) un groupe nitro, (v) amino, (vi) mono- ou di-alkylamino dans lequel le radical alkyle ou chacun des radicaux alkyle représente indépendamment un radical alkyle inférieur renfermant de un à six atomes de carbone ou, dans le cas d'un groupe dialkylamino, dans lequel les deux groupes alkyle, lorsqu'ils sont pris ensemble, représentent un groupe alkylène renfermant de un à six atomes de carbone; (vii) un groupe cyano, (viii) un radical alcoxy inférieur renfermant de un à quatre atomes de carbone, et (ix) un radical thioalkyle inférieur renfermant de un à quatre atomes de carbone; et la partie alkylène renferme de un à quatre atomes de carbone; ou

(5) un radical thiényle, furanyle, pyryle, pyridinyle, quinolyle, isoquinolyle, pyrimidyle, pyridazinyle, quinazolyle, quinoxalyle, benzothiényle, pyrazolyle, imidazolyle, oxozolyle, isoxazolyle, thiazolyle, triazolyle, pyrazinyle, oxazinyle, thiazinyle, indolizinyle, indolyle, benzofuranyle, indazolyle, benzothiényle, benzimidazolyle, benzoxazolyle, benzioxazolyle, benzthiazolyle, benztriazlyle, benzoxadiazolyle, cinnolinyle, phtalaxinyle, naphtyridinyle ou benzothiazinyle;

et dans laquelle $R_2$ et $R_5$ peuvent également être pris ensemble pour former un cycle à cinq ou six éléments renferment comme élément du cycle ou groupe

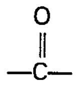 ou 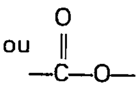

n est égal à un ou deux; m est un nombre entier compris entre un et six;
ce procédé comprenant les étapes suivantes:
on met en contact les dérivés

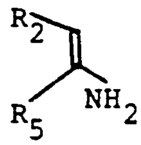

(III),

$R_6CHO$ (IV) et

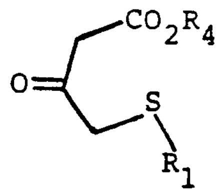

(V),

dans lesquels $R_1$, $R_2$, $R_4$, $R_5$ et $R_6$ sont tels que définis ci-dessus dans un solvant organique pour obtenir un dérivé de formule ($I_o$)

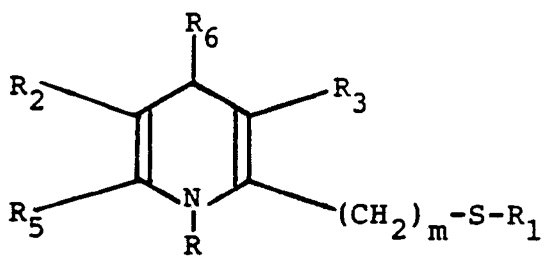

($I_o$)

et, si on le souhaite, on poursuit la réaction du produit par une ou plusieurs des étapes suivantes:
(1) on oxygène l'atome de soufre
(2) on procédé à une alkylation de l'atome d'azote; et
(3) on fait réagir le groupe $—CO_2R_4$ pour obtenir un groupe $R_3$ différent de $—CO_2R_4$;
et éventuellement on en forme un sel pharmaceutiquement acceptable.
2. Un procédé de préparation d'un dérivé de formule (II)

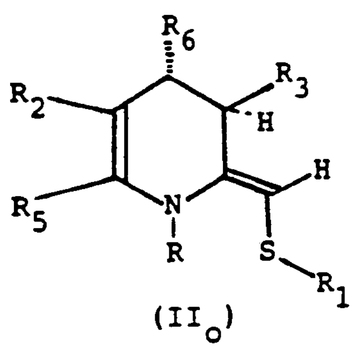

($II_o$)

et un de sels pharmaceutiquement acceptables; dans laquelle
R représente
(1) un atome d'hydrogène;

(2) un radical alkyle inférieur renfermant du un à quatre atomes de carbone éventuellement substitué par un groupe amino répondant à la formule $NR_7R_8$ dans laquelle $R_7$ et $R_8$ représentent indépendamment un atome d'hydrogène ou un radical alkyle inférieur renfermant de un à quatre atomes de carbone ou $R_7$ et $R_8$, lorsqu'ils sont pris ensemble en même temps que l'atome d'azote auquel ils sont attachés, forment un cycle à cinq ou six éléments renfermant quatre ou cinq groupes $CH_2$; ou

(3) un radical arylalkylène dans lequel la partie aryle est le radical phényle éventuellement substitué par un ou plusieurs d'entre (i) un groupe halo, (ii) le radical trifluorométhyle, (iii) un groupe nitro, (iv) un groupe amino, (v) mono- ou di-alkylamino dans lequel l'alkyle ou chacun des alkyles représente indépendamment un radical alkyle inférieur renfermant de un à six atomes de carbone ou, dans le cas d'un dialkylamino, dans lequel les deux radicaux alkyle, lorsqu'on les prend ensemble, représentent un alkylène renfermant de un à six atomes de carbone, (vi) un groupe cyano, (vii) un radical alcoxy inférieur renfermant de un à quatre atomes de carbone, (viii) un radical alkyle inférieur renferment de un à quatre atomes de carbone; (ix) un radical thioalkyle inférieur renfermant de un à quatre atomes de carbone; et dans lequel la partie alkylène renferme de un à quatre atomes de carbone;

$R_2$ et $R_3$, qui peuvent être identiques ou différents, représentent chacun un atome d'hydrogène, un radical alkyle inférieur renfermant de un à quatre atomes de carbone, un groupe cyano, nitro, $CO_2R_4$ dans lequel $R_4$ représente un atome d'hydrogène ou un radical alkyle inférieur renfermant de un à quatre atomes de carbone, éventuellement substitué par un groupe amino, mono- ou di-alkylamino dans lequel le radical alkyle ou chacun des radicaux alkyle représente indépendamment un radical alkyle inférieur renfermant de un à six atomes de carbone ou, dans le cas d'un dialkylamino, dans lequel les deux radicaux alkyle, lorsqu'ils sont pris ensemble, représentent un radical alkylène renfermant de un à six atomes de carbone;

$R_1$, $R_5$ et $R_6$, qui peuvent être indentiques ou différents, représentent chacun

(1) un radical alkyle inférieur renfermant de un à quatre atomes de carbone, éventuellement substitué par (a) un groupe amino répondant à la formule $NR_7R_8$ dans laquelle $R_7$ et $R_8$ représentent indépendamment un atome d'hydrogène ou un radical alkyle inférieur ou bien $R_7$ et $R_8$, lorsqu'ils sont pris ensemble en même temps que l'atome d'azote auquel ils sont attachés, forment un cycle à cinq ou six éléments renfermant quatre ou cinq atomes de carbone, (b) —$OR_9$ dans lequel $R_9$ représente un atome d'hydrogène ou un radical alkyle inférieur renfermant de un à quatre atomes de carbone, ou (c) —$CO_2R_4$ dans lequel $R_4$ et tel que défini ci-dessus;

(2) un radical cycloalkyle renfermant de quatre à sept atomes de carbone;

(3) un radical phényle éventuellement substitué par un ou plusieurs d'entre (i) radical alkyle renfermant de un à quatre atomes de carbone, (ii) un groupe halo, (iii) le radical trifluorométhyle, (iv) un groupe nitro, (v) amino, (vi) mono- ou di-alkylamino dans lequel le radical alkyle ou chacun des radicaux alkyle représente indépendamment un radical alkyle inférieur renfermant de un à six atomes de carbone ou, dans le cas d'un groupe dialkylamino, dans lequel les deux groupes alkyle, lorsqu'ils sont pris ensemble, représentent un groupe alkylène renfermant de un à six atomes de carbone; (vii) un groupe cyano, (viii) un radical alcoxy inférieur renfermant de un à quartre atomes de carbone, et (ix) un radical thioalkyle inférieur renfermant de un à quatre atomes de carbone;

(4) un radical alrylalkylène dans lequel la partie aryle est un radical phényle éventuellement substitué par un ou plusieurs d'entre (i) radical alkyle renfermant de un à quatre atomes de carbone, (ii) un groupe halo, (iii) le radical trifluorométhyle, (iv) un groupe nitro, (v) amino, (vi) mono- ou di-alkylamino dans lequel le radical alkyle ou chacun des radicaux alkyle représente indépendamment un radical alkyle inférieur renfermant de un à six atomes de carbone ou, dans le cas d'un groupe dialkylamino, dans lequel les deux groupes alkyle, lorsqu'ils sont pris ensemble, représentent un groupe alkylène renfermant de un à six atomes de carbone; (vii) un groupe cyano, (viii) un radical alcoxy inférieur renfermant de un à quatre atomes de carbone, et (ix) un radical thioalkyle inférieur renfermant de un à quatre atomes de carbone; et la partie alkylène renferme de un à quatre atomes de carbone; ou

(5) un radical thiényle, furanyle, pyryle, pyridinyle, quinolyle, isoquinolyle, pyrimidyle, pyridazinyle, quinazolyle, quinoxalyle, benzothiényle, pyrazolyle, imidazolyle, oxazolyle, isoxazolyle, thiazolyle, triazolyle, pyrazinyle, oxazinyle, thiazinyle, indolizinyle, indolyle, benzofuranyle, indazolyle, benzothiényle, benzimidazolyle, benzoxazolyle, benzioxazolyle, benzthiazolyle, benztriazolyle, benzoxadiazolyle, cinnolinyle, phtalaxinyle, naphtyridinyle ou benzothiazinyle;

et dans laquelle $R_2$ et $R_5$ peuvent également être pris ensemble pour former un cycle à cinq ou six éléments renfermant comme élément du cycle, un groupe

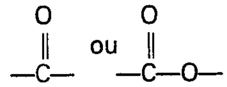

n est égal à zéro ou répresenté un nombre entier égal à un ou deux;

ce procédé comprenant les étapes suivantes:
on met en contact les dérivés

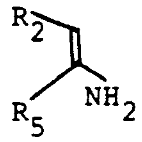

(III),

$R_6CHO$ (IV) et

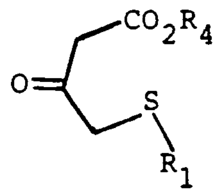

(V),

dans lesquels $R_1$, $R_2$, $R_4$, $R_5$ et $R_6$ sont tels que définis ci-dessus dans un solvant organique pour obtenir un dérivé de formule ($II_o$)

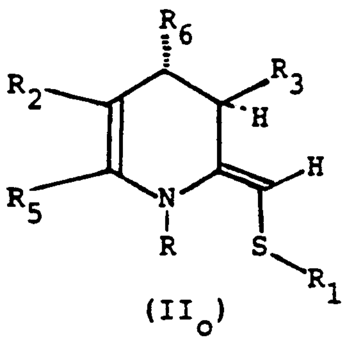

($II_o$)

et, si on le souhaite, on poursuit la réaction du produit par une ou plusieurs des étapes suivantes:
(1) on oxygène l'atome de soufre
(2) on procédé à une alkylation de l'atome d'azote; et
(3) on fait réagir le groupe $—CO_2R_4$ pour obtenir un groupe $R_3$ différent de $—CO_2R_4$;
et éventuellement on en forme un sel pharmaceutiquement acceptable.

3. Un procédé selon la revendication 2, dans lequel $R_2$ représente le groupe cyano et n est égal à un ou deux.

4. Un procédé selon la revendication 1 ou 2, de préparation d'un dérivé de formule

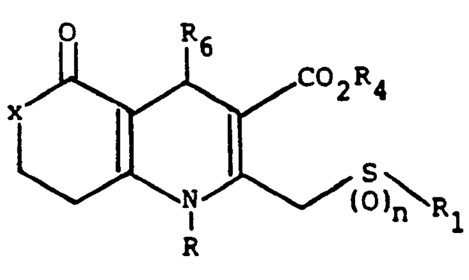

ou

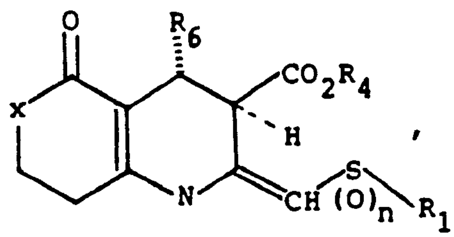

,

dans laquelle X représente $>CH_2$ ou un atome d'oxygène.

5. Un procédé selon la revendication 1 ou 2, dans lequel R représente un atome d'hydrogène, $R_2$ représente un groupe cyano, $R_3$ représente $—CO_2R_4$, $R_5$ représente le radical méthyle et m est égal à un.

6. Un procédé selon la revendication 2, dans lequel R représente un atome d'hydrogène, $R_2$ et $R_3$ représentent indépendamment $—CO_2R_4$, $R_5$ représente le radical méthyle et m est égal à un.

7. Un procédé selon la revendication 4, 5 ou 6 prises en relation avec la revendication 2, dans lequel n est égal à zéro.

8. Un procédé selon la revendication 1 ou 2 de préparation d'un dérivé désigné sous l'un des noms suivants:

ester éthylique de l'acide 3-pyridinecarboxylique 5-cyano-1,2,3,4-tétrahydro-2-[(phénylthio)-méthylène]-6-méthyl-4-[(2-trifluorométhyl)phényl]-;

ester éthylique d'acide 3-quinoléinecarboxylique, 1,2,3,4,5,6,7,8-octahydro-5-oxo-2-[(phénylthio)-méthylène]-4-[(2-trifluorométhyl)phényl]-;

5-cyano-1,4-dihydro-6-méthyl-2-[(phénylsulfinyl)méthyl)-4-[2-(trifluorométhyl)phényl]-3-pyrindine-carboxylate d'éthyle;

5-cyano-1,4-dihydro-6-méthyl-2-[[(2-méthylphényl)sulfinyl)méthyl]-4-[2-(trifluorométhyl)phényl]-3-pyridinecarboxylate d'éthyle;

5-cyano-1,4-dihydro-6-méthyl-4-(3-nitrophényl)-2-[(phénylsulfinyl)méthyl]-3-pyridinecarboxylate d'éthyle;

5-cyano-1,4-dihydro-6-méthyl-2-[[(4-méthoxyphényl)sulfinyl]méthyl]-4-[2-(trifluorométhyl)phényl]-3-pyridinecarboxylate d'éthyle;

5-cyano-1,4-dihydro-6-méthyl-2-[(phénylsulfinyl)méthyl]-4-[2-(chlorophényl)-3-pyridinecarboxylate d'éthyle;

5-cyano-1,4-dihydro-6-méthyl-2-[(4-pyridinylsulfinyl)-méthyl]-4-[2-(trifluorométhyl)phényl]-3-pyridinecarboxylate d'éthyle;

2-[[(2-chlorophényl)sulfinyl]méthyl]-5-cyano-1,4-dihydro-6-méthyl-4-[(2-trifluorométhyl)phényl]-3-pyridine-carboxylate d'éthyle;

Ester éthylique d'acide 5-cyano-2-[[[2-(diméthylamino)éthyl]sulfinyl]méthyl]-1,4-dihydro-6-méthyl-4-[2-(trifluorométhyl)phényl]-3-pyridinecarboxylique;

4-(2-chlorophényl)-5-cyano-1,4-dihydro-6-méthyl-2-[(4-pyridinylsulfinyl)méthyl]-3-pyridinecarboxylate d'éthyle;

5-cyano-4-(2,3-dichlorophényl)-1,4-dihydro-6-méthyl-2-[(4-pyridinylsulfinyl)méthyl]-3-pyridine-carboxylate d'éthyle;

Ester éthylique de l'acide 5-cyano-1,4-dihydro-6-méthyl-2-[(2-pyrimidinylsulfinyl)méthyl]-4-[2-(tri-fluorométhyl)phényl]-3-pyridinecarboxylique;

Ester éthylique de l'acide 5-cyano-1,4-dihydro-6-méthyl-2-[[(1-méthyl-1*H*-imidazol-2-yl)sulfinyl]-méthyl]-4-[2-(trifluorométhyl)phényl]-3-pyridinecarboxylique;

5-cyano-1,4-dihydro-6-méthyl-2-[(phénylsulfinyl)méthyl]-4-phényl-3-pyridinecarboxylate d'éthyle;

2-[[(2-chlorophenyl)sulfinyl]méthylène)-6-méthyl-1,2,3,4-tétrahydro-4-[(2-trifluorométhyl)phényl)-3-éthyl-5-méthyl ester d'acide 3,5-pyridinecarboxylique;

5-cyano-4-cyclohexyl-1,4-dihydro-6-méthyl-2-[(phénylsulfinyl)méthyl)-ester éthylique d'acide 3-pyridinecarboxylique (±)-;

5-cyano-4-[2-(difluorométhoxy)phényl]-1,4-dihydro-6-méthyl-2-[(phénylsulfinyl)méthyl]-ester éthylique d'acide 3-pyridinecarboxylique;

5-cyano-1,4-dihydro-6-méthyl-2-[(phénylsulfinyl)méthyl]-4-[2-(trifluorométhyl)phényl]-méthyl ester d'acide 3-pyridinecarboxylique; (mélange d'isomères à 53/47%);

5-cyano-1,4-dihydro-6-méthyl-2-[(phénylsulfinyl)méthyl]-4-[2-(trifluorométhyl)phényl]-1-méthyléthyl ester d'acide 3-pyridinecarboxylique;

2-hydroxyéthyl ester d'acide 3-pyridinecarboxylique, 5-cyano-1,4-dihydro-6-méthyl-2-[(phénylsulfinyl)méthyl]-4-[2-(trifluorométhyl)phényl]-;

Ester éthylique d'acide 3-pyridinecarboxylique, 5-cyano-1,4-dihydro-6-méthyl-4-(2-phényl-1*H*-imidazol-4-yl)-2-[(phénylsulfinyl)méthyl]-(diastéréoisomères);

Ester éthlique d'acide 3-pyridinecarboxylique, 5-cyano-1,4-dihydro-6-méthyl-2-[(2-pyridinylsulfinyl)-méthyl]-4-[2-(trifluorométhyl)phényl]- (±)-;

Ester éthylique d'acide 3-pyridinecarboxylique, 5-cyano-2-[[(2-fluorophényl)sulfinyl]méthyl]-1,4-dihydro-6-méthyl-4-[2-(trifluorométhyl)phényl]-;

Ester éthylique d'acide 3-pyridinecarboxylique, 2-[[[4-(acétylamino)phényl]sulfinyl]methyl]-5-cyano-1,4-dihydro-6-méthyl-4-[2-(trifluorométhyl)phényl]-;

Ester éthylique d'acide 3-pyridinecarboxylique, 2-[[(4-chlorophényl)sulfinyl]méthyl]-5-cyano-1,4-dihydro-6-méthyl-4-[2-(trifluorométhyl)phényl]- (±)-;

Ester éthylique d'acide 3-pyridinecarboxylique, 5-cyano-2-[[(3,4-dichlorophényl)sulfinyl]méthyl]-1,4-dihydro-6-méthyl-4-[2-(trifluorométhyl)phényl]-;

Acide 3-pyridinecarboxylique, 5-cyano-1,4-dihydro-6-méthyl-2-[(phénylsulfinyl)méthyl]-4-[2-(trifluoro-méthyl)phényl]-;

Ester éthylique d'acide 3-quinoléinecarboxylique, 1,4,5,6,7,8-hexahydro-1-méthyl-5-oxo-2-[(phényl-sulfinyl)méthyl]-4-[2-(trifluorométhyl)phényl]-;

Ester éthylique d'acide 3-quinolécarboxylique, 1,4,5,6,7,8-hexahydro-5-oxo-2-[phénylsulfinyl)méthyl]-4-[2-(trifluorométhyl)phényl]-; (±)-;

3-cyano-1,4-dihydro-6-méthyl-2-[(phénylsulfinyl)méthyl]-4-[2-(trifluorométhyl)phényl]-5-pyridine-carboxylate d'éthyle;

Ester éthylique d'acide 3-pyridinecarboxylique, 5-cyano-1-[2-(diéthylamino)éthyl]-1,4-dihydro-6-méthyl-2-[(phénylsulfinyl)méthyl]-4-[2-(trifluorométhyl)phényl]-;

Ester éthylique d'acide 3-pyridinecarboxylique, 5-cyano-1,4-dihydro-[[(3-méthoxy-3-oxopropyl]-sulfinyl]méthyl]-6-méthyl-4-[2-(trifluorométhyl)phényl]-;

Ester éthylique d'acide 3-pyridinecarboxylique, 5-cyano-2-[[(2-éthoxy-2-oxoéthyl)sulfinyl]méthyl]-1,4-dihydro-6-méthyl-4-[2-(trifluorométhyl)phényl]-;

Ester éthylique d'acide [2,4'-bipyridine]-3'-carboxylique, 5'-cyano-1',4'-dihydro-6'-méthyl-2'-[(phénylsulfinyl)méthyl]-;

5-cyano-1,4-dihydro-6-méthyl-2-[(phénylsulfonyl)méthyl]-4-[2-(trifluorométhyl)phényl]-3-pyridine-carboxylate d'éthyle;

5-cyano-1,4-dihydro-6-méthyl-2-[[(4-méthoxyphényl)sulfonyl]méthyl]-4-[2-(trifluorométhyl)phényl]-3-pyridinecarboxylate d'éthyle;

5-cyano-1,4-dihydro-6-méthyl-2-[[(2-méthylphényl)sulfonyl]méthyl]-4-[2-(trifluorométhyl)phényl]-3-pyridinecarboxylate d'éthyle;

2-[[(2-chlorophényl)sulfonyl]méthyl]-5-cyano-1,4-dihydro-6-méthyl-4-[2-(trifluorométhyl)phényl]-3-pyridinecarboxylate d'éthyle;

5-cyano-1,4-dihydro-6-méthyl-2-[(4-pyridinylsulfonyl)méthyl]-4-[2-(trifluorométhyl)phényl]-3-pyridine-carboxylate d'éthyle;

N-oxyde ester éthylique d'acide 5-cyano-1,4-dihydro-6-méthyl-2-[(2-pyridinylsulfonyl)méthyl]-4-[2-(tri-fluorométhyl)phényl]-3-pyridinecarboxylique;

5-cyano-1,4-dihydro-6-méthyl-2-[(phénylsulfonyl)méthyl-4-phényl-3-pyridinecarboxylate d'éthyle;

1-oxyde de 5'-cyano-1',4'-dihydro-6'-méthyl-2'-[(phénylsulfonyl)méthyl]ester éthylique d'acide [2,4'-bipyridine]-3'-carboxylique;

5'-cyano-1',4'-dihydro-6'-méthyl-2'-[(phénylsulfonyl)méthyl]-ester éthylique d'acide [3,4'-bipyridinel-3'-carboxylique;

2-[[(4-aminophényl)sulfonyl]méthyl]-5-cyano-1,4-dihydro-6-méthyl-4-[2-(trifluorométhyl)phényl]ester éthylique d'acide 3-pyridinecarboxylique;

5'-cyano-1',4'-dihydro-6'-méthyl-2'-[(phénylsulfonyl)méthyl]ester éthylique d'acide [2,4'-bipyridine)-3'-carboxylique;

5'-cyano-4-cyclohexyl-6-méthyl-1,4-dihydro-2-[(phénylsulfonyl)méthyl]-, ester éthylique d'acide 3-pyridinecarboxylique;

5-cyano-1,4-dihydro-6-méthyl-2-[(phénylsulfonyl)méthyl]-4-[2-(trifluorométhyl)phényl]-, 1-méthyléthyl ester d'acide 3-pyridinecarboxylique;

5-cyano-1,4-dihydro-6-méthyl-2-[(phénylsulfonyl)méthyl]-4-[2-(trifluorométhyl)phényl]-, 2-propényl ester d'acide 3-pyridinecarboxylique;

5-cyano-2[[[4-(1,1-diméthyléthyl)-2-méthylphényl]sulfonyl]méthyl]-1,4-dihydro-6-méthyl-4-[2-(tri-fluorométhyl)phényl]-, ester éthylique d'acide 3-pyridinecarboxylique;

5-cyano-2[[(2-fluorophényl)sulfonyl]méthyl]-1,4-dihydro-6-méthyl-4-[2-(trifluorométhyl)phényl]-, ester éthylique d'acide 3-pyridinecarboxylique;

2-[[(4-chlorophényl)sulfonyl]méthyl]-5-cyano-1,4-dihydro-6-méthyl-4-[2-(trifluorométhyl)phényl]-, ester éthylique d'acide 3-pyridinecarboxylique;

5-cyano-1,4-dihydro-6-méthyl-4-(3-nitrophényl)-2-[(phénylsulfonyl)méthyl]-, ester éthylique d'acide 3-pyridinecarboxylique;

5-cyano-1,4-dihydro-6-méthyl-2-[(phénylsulfonyl)méthyl]-4-[2-(trifluorométhyl)phényl]-, 2,3-dihydroxypropyl ester d'acide 3-pyridinecarboxylique;

5-cyano-1,4-dihydro-2-[[(3-méthoxy-3-oxopropyl)sulfonyl]métyl]-6-méthyl-4-[2-(trifluorométhyl)-phényl]-, ester éthylique d'acide 3-pyridinecarboxylique;

1,4,5,6,7,8-hexahydro-1-méthyl-5-oxo-2-[(phénylsulfonyl)méthyl]-4-[2-(trifluorométhyl)phényl]-, ester éthylique d'acide 3-quinoléinecarboxylique;

1,4,5,6,7,8-hexahydro-5-oxo-2-[(phénylsulfonyl)-méthyl]-4-[2-(trifluorométhyl)phényl]-, ester éthylique d'acide 3-quinoléinecarboxylique; et

1,4,5,6,7,8-hexahydro-1-méthyl-5-oxo-2[(phénylthio)méthyl]-4-[2-(trifluorométhyl)phényl]-, ester éthylique d'acide 3-quinoléinecarboxylique.

9. Un procédé de préparation d'une composition pharmaceutique, ce procédé consistant à associer une quantité efficace d'un dérive préparé par un procédé selon l'une quelconque des revendications précédentes à un support ou à un diluant pharmaceutiquement acceptable.

10. Pour l'utilisation dans une méthode de traitement d'un mammifère souffrant de troubles cardio-vasculaires, un dérivé préparé par un procédé selon l'une quelconque des revendications 1 à 8 ou une composition pharmaceutique préparée par un procédé selon la revendication 9.